# EUROPEAN PATENT SPECIFICATION

(11) **EP 2 355 849 B1**
(45) Date of publication and mention of the grant of the patent: **02.05.2018**
(21) Application number: 09824314.0
(22) Date of filing: 06.11.2009
(51) Int. Cl.: A61K 39/395, A61P 37/04, C07K 16/12, C07K 19/00

(54) **METHODS OF IMPROVING THE THERAPEUTIC EFFICACY AND UTILITY OF ANTIBODY FRAGMENTS**
VERFAHREN ZUR VERBESSERUNG DER THERAPEUTISCHEN WIRKSAMKEIT UND DES THERAPEUTISCHEN NUTZENS VON ANTIKÖRPERFRAGMENTEN
PROCÉDÉS D'AMÉLIORATION DE L'EFFICACITÉ THÉRAPEUTIQUE ET UTILISATION DE FRAGMENTS D'ANTICORPS

(30) Priority: 06.11.2008 US 111915 P
(43) Date of publication of application: 17.08.2011
(73) Proprietor: University Of Guelph, Guelph, ON N1G 2W1 (CA)
(72) Inventor: HALL, Christopher, J., Guelph, Ontario N1G 2R4 (CA); McLEAN, Michael, D., Guelph, Ontario N1H 0A9 (CA); XIE, Xuemei, Houston, Texas, 77096 (US); WEISSER, Nina, Delta, British Columbia V4K 2E7 (CA); ALMQUIST, Kurt, C., Guelph, Ontario N1K 1P9 (CA)
(74) Representative: Owen, Deborah Jane
(86) International application number: PCT/CA2009/001606
(87) International publication number: WO 2010/051635

(56) References cited:
- US-A1- 2010 278 830
- REHLAENDER B N ET AL: "Anti-drug antibodies as drug carriers. I. For small molecules", PHARMACEUTICAL RESEARCH, KLUWER ACADEMIC PUBLISHERS, NEW YORK, NY, US, vol. 18, no. 6, 1 June 2001 (2001-06-01), pages 745-752, XP002499903, ISSN: 0724-8741, DOI: 10.1023/A:1011072009408
- BRUCE N. REHLAENDER ET AL: PHARMACEUTICAL RESEARCH, vol. 18, no. 6, 1 January 2001 (2001-01-01), pages 753-760, XP55034415, ISSN: 0724-8741, DOI: 10.1023/A:1011024126247
- BILL BRIZZARD: "Epitope tagging", BIOTECHNIQUES, vol. 44 Supplement, no. 4, 1 April 2008 (2008-04-01), pages 693-695, XP55034344, ISSN: 0736-6205, DOI: 10.2144/000112841
- SHAKI-LOEWENSTEIN, S. ET AL.: 'A universal strategy for stable intracellular antibodies' J. IMMUNOL. METHODS vol. 303, June 2005, pages 19 - 39, XP027659288
- BACH, H. ET AL.: 'Escherichia coli maltose-binding protein as a molecular chaperone for recombinant intracellular cytoplasmic single-chain antibodies' J. MOL. BIOL. vol. 312, 2001, pages 79 - 93, XP004466120
- WANG, X. ET AL.: 'Enhancement of scFV fragment reactivity with target antigens in binding assays following mixing with anti-tag monoclonal antibodies' J. IMMUNOL. METHODS vol. 294, September 2004, pages 23 - 35, XP004679752
- WEISSNER, N. E. ET AL.: 'Applications of single-chain variable fragment antibodies in therapeutics and diagnostics' BIOTECH. ADVANCES vol. 27, April 2009, pages 502 - 520, XP026127899

## Description

The present disclosure relates to the field of therapeutic antibody fragments.

### BACKGROUND OF THE DISCLOSURE

A number of small recombinant antibody (Ab) fragments (rAbs) including monovalent fragments, such as Fab, scFv, V_{H}H and multivalent fragments, such as diabodies, triabodies and minibodies have been engineered for various applications (reviewed in [1]). These rAb fragments retain the target specificity of the full length monoclonal Abs (mAbs), can be produced more economically than mAbs, and possess unique properties that are suitable for specific diagnostic and therapeutic applications. Such applications include those where Fc-mediated effector functions are not required or are undesirable, for example, for use in *in vivo* imaging. For imaging, radiolabeled rAb fragments exhibit rapid tumor localization and diffusion and better imaging contrast due to their shorter *in vivo* half-life, and thus result in shorter exposure of non-specific tissues in comparison to their mAb counterparts (reviewed in [2]). Consequently, rAb fragments are being used as alternatives to mAbs for various applications such as *in vivo* tumor- and-clot imaging applications and *in vitro* immunoassays, and are expected to capture a significant share of the approximately $6 billion (US) per year diagnostic market[1].

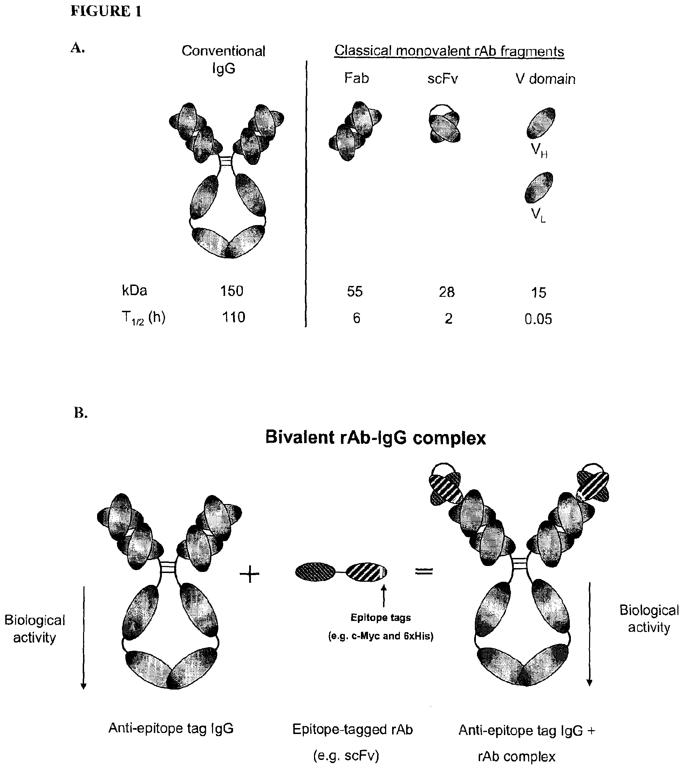

However, compared to full length Abs and in situations where Fc-mediated effects are desired, the classic monovalent rAb fragments have three major therapeutic limitations: 1) a shorter *in vivo* half-life due to rapid elimination by first pass renal clearance because their MW is below the filtration barrier (approximately 65 kDa) of the kidney glomeruli, and because there is no interaction with the neonatal receptors (FcRns) that bind to Fc regions to regulate IgG catabolism, 2) reduced apparent affinity due the lack of avidity, and 3) the inability to recruit Fc-mediated effector functions such as phagocytosis, complement dependent cell cytotoxicity (CDC) and Ab dependent cellular cytotoxicity (ADCC) (Figure 1a) [3]. Thus, in situations where longer *in vivo* half-lives, increased apparent affinity and Fc-mediated effector functions are desired, small rAb fragments have limited therapeutic applications.

Epitope tagging is a technique in which a short antigenic amino acid sequence is added to a protein of interest, often at the amino or carboxy-terminus, by recombinant DNA methods. The antigenic tag is used in a variety of *in vitro* applications for easy detection, characterization and purification of the tagged protein with a mAb against the peptide tag [reviewed in [4]]. Combining epitope-tagged rAb fragments with an anti-epitope tag IgG in the proper ratios should result in the non-covalent formation of bivalent rAb-anti-epitope tag IgG complex. The utility of combining epitope-tagged rAb fragments with an anti-epitope tag IgG to increase the rAb reactivity in an ELISA has been previously described [5]. However, the potential *in vivo* benefits of using this technology therapeutically has not yet been presented in the literature.
Weisser & Hall (Biotechnol. Adv., 2009, 27, pp 502-20) includes an overview of immunotoxins. Bach et al. (J. Mol. Biol., 2001, 312, pp 79-93) relates to *Escherichia coli* maltose-binding protein as a molecular chaperone for recombinant intracellular cytoplasmic single-chain antibodies. Rehlaender & Cho (Pharm. Research 18: 745-752 and 753-760, 2001) describe anti-drug antibodies as drug carriers for small molecules and proteins.

Accordingly, there is a need for: (1) an efficient and inexpensive means of producing antibody fragments that are specific for antigenic targets in mammals, and particularly in humans; and (2) methods of improving the therapeutic utility and efficacy of the antibody fragments produced in (1) in situations where activation of downstream immune system functions is desired.

### SUMMARY OF THE DISCLOSURE

Embodiments of the present invention are as defined in the claims. In particular, the present invention relates to an antibody fragment linked to an epitope and an antibody that binds to the epitope for use in treating an infection from bacteria or viruses in an animal in need thereof, wherein:
(i) the antibody fragment linked to the epitope is to be administered to the animal; and
(ii) the antibody that binds to the epitope is to be administered to the animal prior to, at the same time or after said antibody fragment linked to the epitope;
wherein upon use a complex forms between the antibody fragment linked to the epitope and the antibody that binds to the epitope, thereby enhancing the efficacy of the antibody fragment compared to an equivalent uncomplexed antibody fragment, and wherein the antibody fragment binds to a target antigen selected from bacterial antigens or viral antigens.

The present inventors have discovered a method of improving the therapeutic efficacy and utility of antibody fragments by employing anti-epitope-tagging technologies. The epitope-tagged antibody fragments of the uses described herein exhibited an increased *in vivo* persistence and the ability to recruit downstream immune system functions to the target antigen specified by the antibody fragment. The present inventors demonstrated that the therapeutic efficacy and utility was achieved by the non-covalent binding between epitope-tagged rAb fragments (e.g. 6xHis-tagged scFv and Fab) and an anti-epitope tag IgG (e.g. anti-Penta-His) that resulted in the formation of a bivalent rAb-IgG complex.

Disclosed herein is a method of enhancing efficacy of an antibody fragment comprising administering an effective amount of the antibody fragment linked to an epitope to an animal in need thereof, wherein a complex forms between the antibody fragment linked to the epitope and an antibody that binds to the epitope. The present invention includes the use of an antibody fragment linked to an epitope to enhance the efficacy of the antibody fragment in an animal in need thereof, wherein upon use a complex forms between the antibody fragment linked to the epitope and an antibody that binds to the epitope

The antibody that binds to the epitope linked to the antibody fragment is co-administered or used with the antibody fragment linked to the epitope. Disclosed herein, the anti-epitope antibody may already present in the animal via previous immunizations with the epitope or through standard vaccine protocols.

The uses of the disclosure described herein result in an enhanced efficacy of the antibody fragment including an increased therapeutic effect of the antibody fragment; an increased persistence or half-life and/or stability of the antibody fragment; an increased immune response; activation of downstream immune system functions; increased recruitment of FcR-mediated effector functions; recruitment of the complement system and/or increasing phagocytosis; enhanced avidity of the antibody fragment; and enhanced protective efficacy of the antibody fragment against its target antigen including enhanced protection against infection from bacteria or viruses, or the toxins thereof.

Disclosed herein is the generation of a number of epitope-tagged antibody fragments that recognize different target antigens, and the corresponding generation of one or a few anti-epitope antibodies that recognize the epitope tag of the antibody fragments.

Disclosed herein is a pharmaceutical composition comprising an effective amount of the antibody fragment linked to an epitope with a pharmaceutically acceptable carrier or diluent, adjuvant or mixtures thereof. Disclosed herein, the composition may also comprise an antibody that binds to the epitope.

Other features and advantages of the present disclosure will become apparent from the following detailed description. It should be understood, however, that the detailed description and the specific examples while indicating preferred embodiments of the disclosure are given by way of illustration only.

### BRIEF DESCRIPTION OF THE DRAWINGS

The disclosure will now be described in relation to the drawings in which:
Figure 1 (a) is a diagram showing IgG and three major types of monovalent Abs used in research together with their respective molecular weights (kDa) and serum half life (β phase), (modified from Holliger and Hudson, 2005 [1]). Figure 1 (b) is a diagram of a bivalent rAb-anti-epitope tag IgG complex as illustrated with a rAb scFv. Monovalent rAb and anti-epitope tag IgG molecules when mixed (2:1) result in the formation of a bivalent rAb-anti-epitope tag IgG complex.

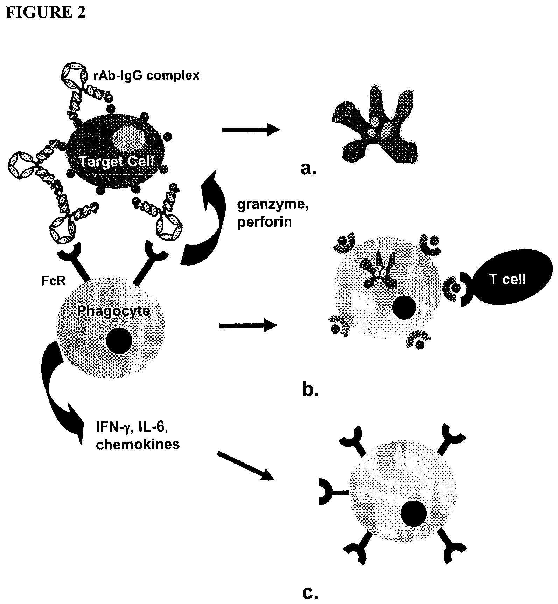

Figure 2 is a diagram showing the proposed FcR-mediated effector function associated with administration of bivalent rAb-IgG complexes. (a) Antibody dependent cell cytotoxicity (ADCC; cell lysis), (b) Phagocytosis, antigen presentation and T cell activation and, (c) Immune activation via cytokine release e.g. increased FcR and MHC expression (modified in part from Desjarlais et al. 2007 [6]).

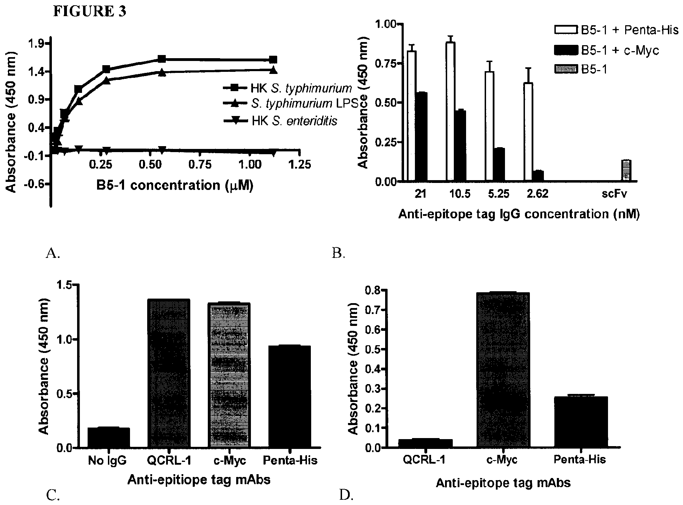

Figure 3 summarizes data from ELISA experiments. (a) ELISA data of the binding specificity of the B5-1 scFv clone to immobilized heat-killed S. *typhimurium,* heat-killed S. *enteridititis* (10⁷ cfu/well) and S. *typhimurium* LPS (10 ug/well) in monovalent format. (b) ELISA data demonstrating the increased binding affinity of the scFv-anti-epitope tag IgG complex to heat-killed S. *typhimurium* in comparison to binding of scFv alone. The anti-Penta-His and anti-c-myc IgGs were compared at various concentrations in complex with a constant scFv concentration (10 nM). Tukey's HSD analysis of Penta-His™ vs c-Myc resulted in significant differences at P-values < 0.01 for each concentration of the anti-tag IgG, respectively. (c) ELISA data demonstrating the C1q binding ability of the c-Myc, Penta-His™ and QCRL-1 anti-tag IgG1 Abs. Anti-tag Abs were immobilized on the ELISA plate (10 µg/mL), purified mouse C1q was incubated at 2 µg/mL and detected with goat-anti-C1q (1:2000) and then with anti-goat-HRP. A Dunnett's test was used to compare each anti-tag IgG with the control (i.e. no Ab) and resulted in significant differences at P < 0.001 for each anti-tag IgG. (d) ELISA data demonstrating C1q recruitment of the anti-tag IgG-scFv complex when bound to wells coated with heat-killed S. *typhimurium* (10⁷/well). C1q binding was detected with goat-anti-C1q and anti-goat-HRP, as above. A Dunnett's test to compare each specific anti-tag IgG with the control (i.e. no Ab) resulted in significant differences at P < 0.001. All ELISAs were performed in triplicate with background values to non-coated wells subtracted.

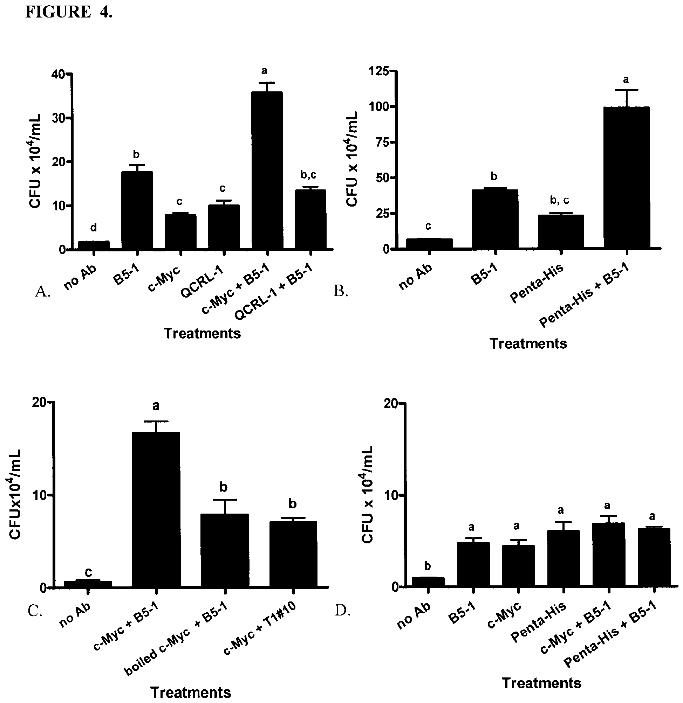

Figure 4 summarizes *in vitro* data comparing Ab-dependent phagocytosis of S. *typhimurium* (a-c) and S. *enteriditis* (d) by J774 MΦ cells. (a) Treatment with the B5-1-anti-c-Myc IgG complex and B5-1-anti-QCRL-1 non-complex. (b) Treatment with the B5-1-anti-Penta-His complex (c) Treatment with boiled anti-tag mAb (anti-c-Myc) in association with B5-1 and in intact anti-c-Myc in complex with a non-specific T1#10 (d) Phagocytosis of the non-specific bacterium *S. enteriditis.* In (a-d) treated means were separated using a Tukey's test. Significant differences between treatment groups, P < 0.05, are indicated by the letter designations a-d.

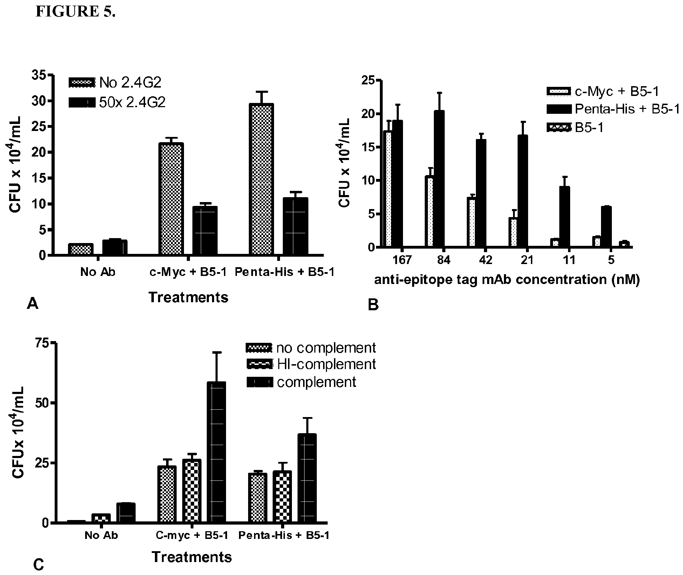

Figure 5 summarizes *in vitro* data examining the effects of FcR blocking, anti-epitope tag IgG affinity and the presence of complement on phagocytosis. (a) Blocking of J774 MΦ cell-mediated phagocytosis of the B5-1-anti-Penta-His and B5-1-anti-c-Myc complex treated cells with the anti-FcR mAb 2.4G2. A Dunnett's test was used to compare each treatment with the control and resulted in P < 0.001 and < 0.001 for the B5-1 anti-c-Myc and B5-1-anti-Penta-His treatments, respectively. (b) Treatment with the B5-1-anti-c-Myc and B5-1-anti-Penta-His complexes was compared at various anti-tag IgG1 concentrations. A Dunnett's test was used to compare of the B5-1-anti-Penta-His vs. the B5-1-anti-c-Myc treatments and resulted in P values of > 0.05, < 0.05, < 0.01, < 0.01, < 0.01, < 0.001 for the 167, 83, 41.7, 21, 10.5 and 5.25 nM anti-tag IgG concentrations, respectively. (c) Phagocytosis of S. *typhimurium* in the presence of whole murine complement serum, HI-complement (heat inactivated complement) or no complement. A Dunnett's test of no complement vs. HI-complement and no complement vs. complement treatments, and resulted in P values of > 0.05 and < 0.05 for the B5-1-anti-c-Myc treatment and > 0.05 and ≤ 0.06 for the B5-1-anti-Penta-His treatment.

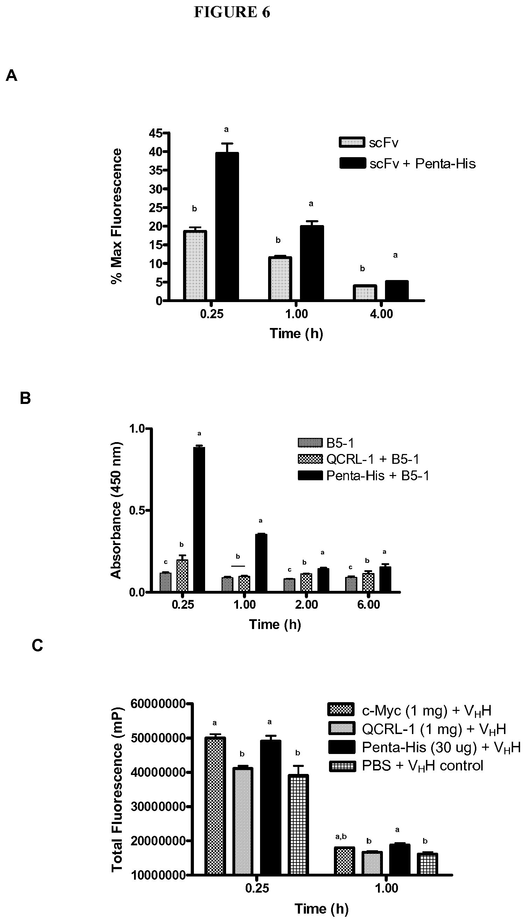

Figure 6 summarizes in vivo rAb clearance data. RAb-IgG complexes improved rAb in vivo serum persistence. (a) In vivo experiment #1. RAbs were labeled with FITC, then rAb-IgG complexes were allowed to preform in vitro before intravenous administration to CD-1 female mice. Serum persistence of T1#10 scFv through fluorescence analysis is shown. Mean % maximum fluorescence (y-axis) is given versus time (x-axis) from sera of treated mice, with standard error bars. Dark bars represent T1#10 scFv-anti-Penta-His IgG complexes; light bars, T1#10 alone. Each treatment and time point involved 5 mice. The rAb:IgG ratio was 20:1; 200 µg scFv and 0 or 50 µg IgG were used per mouse. (b) In vivo experiment #2. Administered rAb persistence and binding-specificity was maintained in vivo. The binding of B5-1 in pooled mouse serum from the 2:1 assay to immobilized S. Typhimurium LPS (10 µg/mL) was tested by ELISA. Data from pooled mouse serum diluted 1/3 is presented as an average of three replicates plus SEM. Background binding to non-coated wells was not subtracted. (c) In vivo experiment #3. Total fluorescence values (mP) of the mouse serum. Data from each of the five mice were pooled per time point. Maximum fluorescence values of the injected dose were not measured and thus these data are presented as total fluorescence (mP) values. In (a-c), treatments are given in the upper right of each graph. Tukey's analysis was performed for analysis of difference between treatments. Significant differences between treatment groups, P values < 0.05, are indicated by the letter designations a-c.

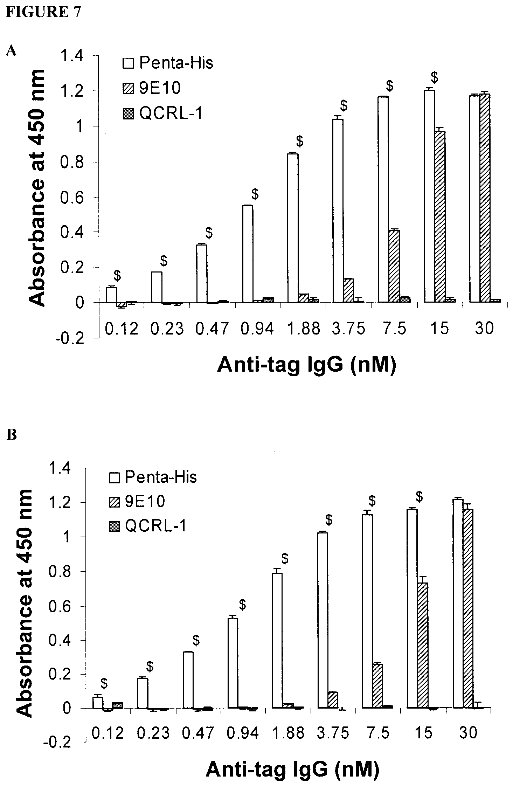

Figure 7 summarizes the binding properties of anti-tag IgGs to epitope-tagged rAbs. Binding of Penta-His, 9E10, and QCRL-1 to the epitope-tagged scFv (60 nM; Graph A) and Fab (60 nM; Graph B) was determined by ELISA; both scFv and Fab are specific for *P. aeruginosa* O6ad. Data represent the background-subtracted means of triplicates ± SD. Statistical differences (P<0.0001) among the three means at each concentration of anti-tag IgG were analyzed by One-Way ANOVA and are indicated by $.

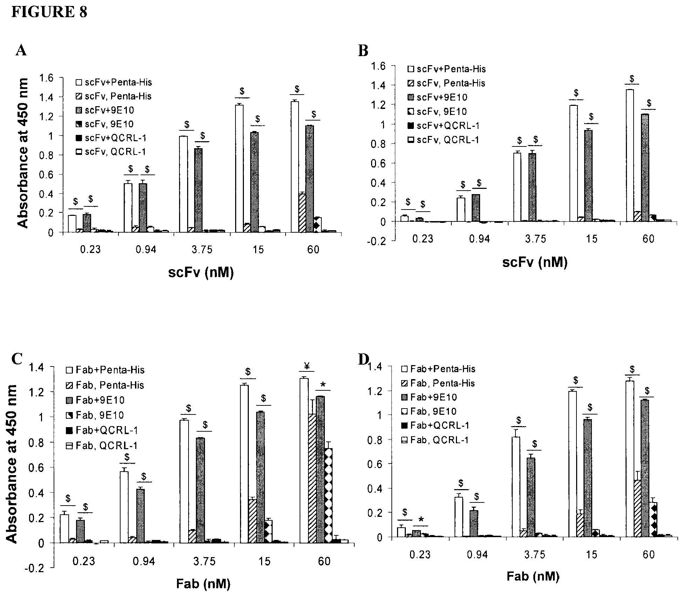

Figure 8 summarizes the antigen binding ability of epitope-tagged rAbs following complex formation with anti-tag IgGs. **A** and **B,** Binding of P. *aeruginosa* O6ad-specific scFv treatments to heat-killed O6ad (1 x 10⁸ CFU/ml) and LPS_{O6ad} (1 µg/ml), respectively. **C** and **D,** Binding of P. *aeruginosa* O6ad-specific Fab treatments to heat-killed O6ad (1 x 10⁸ CFU/ml) and LPS_{O6ad} (1 µg/ml), respectively. Treatment legend is given in the upper left of each graph. Each rAb-IgG complex treatment (e.g. scFv + Penta-His) was prepared by mixing scFv or Fab with Penta-His, 9E10, or QCRL-1 prior to conducting the ELISA. Each sequential treatment (e.g., scFv, Penta-His) was comprised of scFv or Fab added to the ELISA plate to interact with bound antigen following by washing of the plate before one of the three anti-tag IgGs was added. scFv or Fab applied together or sequentially with QCRL-1 are non-specific controls. Data represent the background-subtracted means of triplicates ± SD. Symbols above bars represent significant differences ($ = P<0.0001, * = P<0.001, and # = P<0.01) between pairs of treatment means (e.g. scFv + Penta-His vs.
   scFv, Penta-His) at each concentration of rAb, as analyzed by One-Way AN OVA.

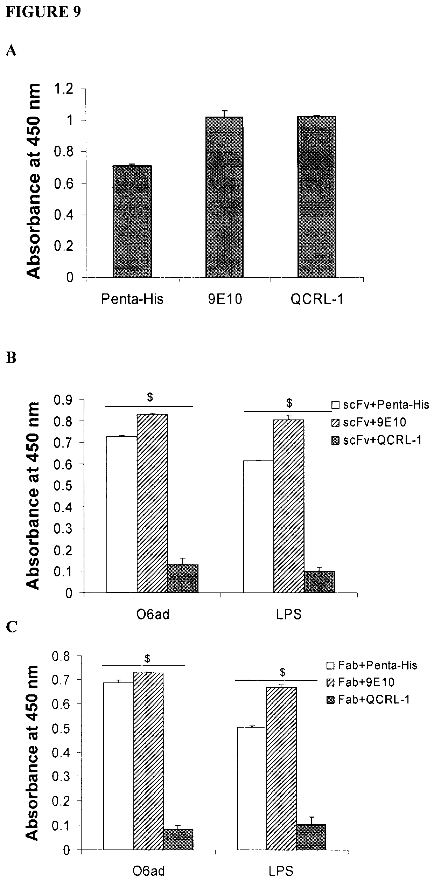

Figure 9 summarizes data of C1q deposition by anti-tag IgG when applied alone or complexed with epitope-tagged rAbs. **A,** C1q deposition by each anti-tag IgG (66.67 nM) when applied alone. **B,** C1q deposition by scFv-anti-tag IgG complexes using heat-killed *P. aeruginosa* O6ad (1 x 10⁸ CFU/ml) and LPS_{O6ad} (10 µg/ml) as coating antigens. **C,** C1q deposition by Fab-anti-tag IgG complexes using heat-killed *P. aeruginosa* O6ad (1 x 10⁸ CFU/ml) and LPS_{O6ad} (10 µg/ml) as coating antigens. Data represent the background-subtracted means of triplicates ± SD. Symbol ($) above bars represent significant differences at $ = P<0.0001 among all three treatment means within each antigen, as analyzed by One-Way ANOVA.

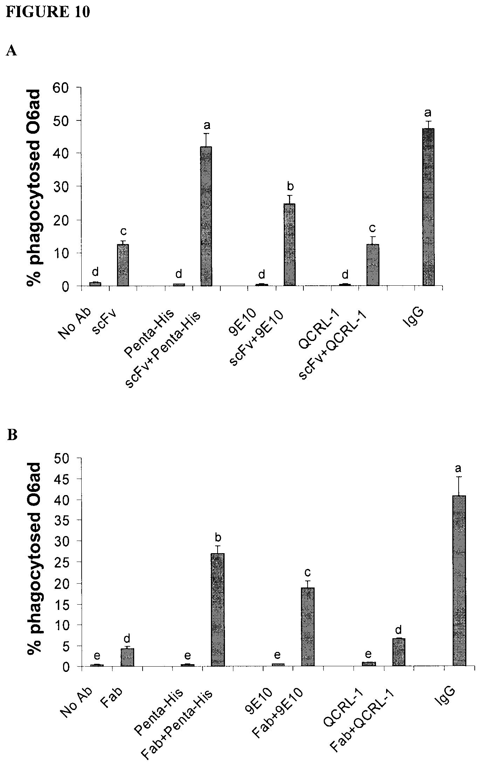

Figure 10 summarizes data showing rAb-anti-tag IgG complex-mediated phagocytosis of *P. aeruginosa* O6ad (1 x 10⁶ CFU) by macrophage J774.1A (1 x 10⁵ cells). **A,** scFv-IgG complex-mediated phagocytosis; **B,** Fab-IgG complex-mediated phagocytosis. Incubations of P. *aeruginosa* O6ad cells with J774.1A cells in the absence of antibodies or in the presence of rAb alone, anti-tag IgG alone, mixture of rAbs and QCRL-1, or anti-O6ad IgG were used as controls. The antibody concentrations used were 335 nM for rAbs and 167.5 nM for anti-tag IgGs. The phagocytosed population of bacteria was calculated according to the formula % phagocytosed bacteria = (phagocytosed bacterial number at the end of 30 min incubation/initial bacterial number at the beginning of 30 min incubation) x 100%. Data represent the means ± SE from a single experiment performed at least in triplicate. The different letters a-d indicate statistical differences (P<0.001) among the nine treatments, as analyzed by One-Way ANOVA.

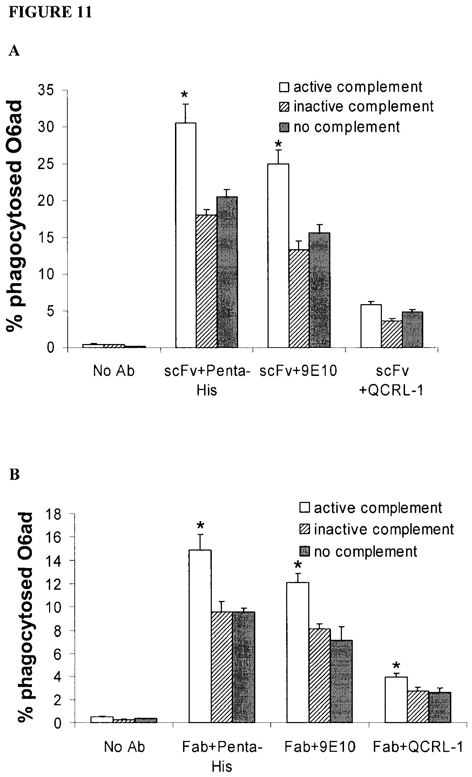

Figure 11 summarizes data showing rAb-anti-tag IgG complex-mediated phagocytosis of *P. aeruginosa* O6ad (1 x 10⁶ CFU) by macrophage J774.1A cells (1 x 10⁵ cells) in the presence of 1.25% murine complement. **A,** scFv-anti-tag IgG-mediated phagocytosis. **B,** Fab-anti-tag IgG-mediated phagocytosis. Incubations of the bacteria with J774.1A cells in the absence of antibodies or in the presence of rAbs plus QCRL-1 were used as controls. The antibody concentrations used were 335 nM for rAbs and 167.5 nM for anti-tag IgGs. The phagocytosed population of bacteria was calculated according to the formula % phagocytosed bacteria = (phagocytosed bacterial number at the end of 30 min incubation/initial bacterial number at the beginning of 30 min incubation) x 100%. Data represent the means ± SE from a single experiment performed at least in triplicate. Statistical differences within a treatment are analyzed by One-Way ANOVA and indicated by * = P<0.001.

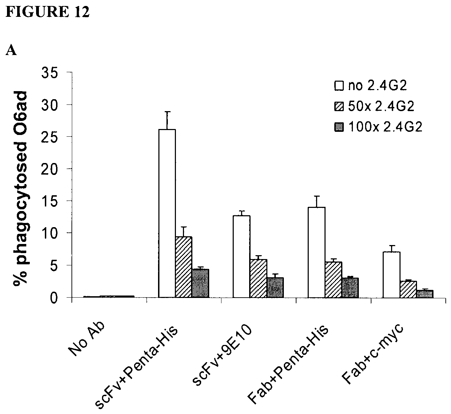

Figure 12 summarizes data showing inhibition of rAb-anti-tag IgG complex-mediated phagocytosis of *P. aeruginosa* O6ad by anti-FcγRIIB/III mAb 2.4G2. Phagocytosis inhibition was carried out by 1 h preincubation of J774.1A cells with mAb 2.4G2 at concentrations 50 and 100 times greater than that of anti-tag IgG (167.5 nM) prior to addition of antibody-opsonized bacteria. The blocked population of bacteria was calculated according to the formula % of blocked phagocytosis = [(phagocytosed bacterial number without 2.4G2 - phagocytosed bacterial number with 2.4G2)/phagocytosed bacterial number without 2.4G2] x 100%. Data represent the means ± SE from a single experiment performed at least in triplicate. Statistical differences within a treatment were analyzed by One-Way ANOVA and are indicated by $ = P<0.0001 and * = P<0.001.

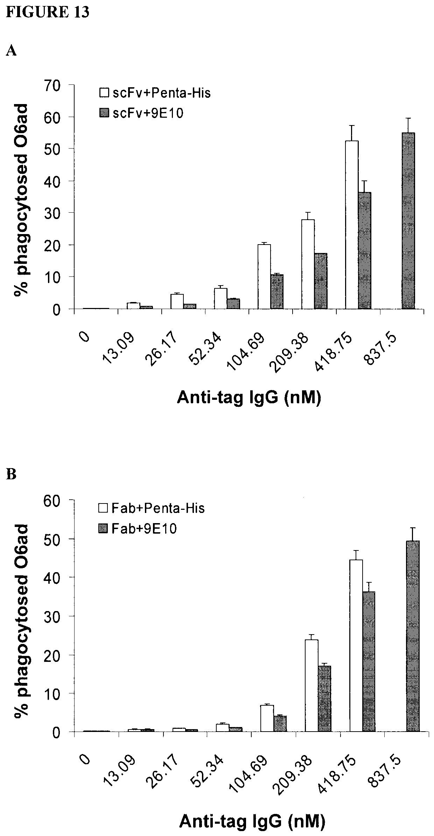

Figure 13 summarizes data showing the dose response of rAb-anti-tag IgG complex-mediated phagocytosis of P. *aeruginosa* O6ad (1 x 10⁶ CFU) by macrophage J774.1A cells (1 x 10⁵). **A,** scFv-anti-tag IgG complex-mediated phagocytosis; **B,** Fab-anti-tag IgG complex-mediated phagocytosis. The phagocytosed population of bacteria was calculated according to the formula % phagocytosed bacteria = (phagocytosed bacterial number at the end of 30 min incubation/initial bacterial number at the beginning of 30 min incubation) x 100%. Data represent the means ± SE from a single experiment. Each experiment was performed at least in triplicate.

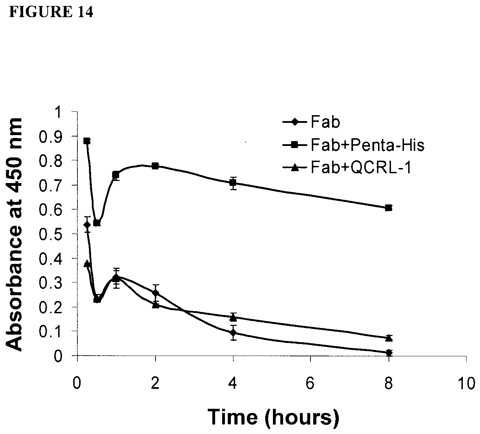

Figure 14 summarizes data showing *in vivo* serum persistence of Fab. Fab in mouse sera was analyzed by ELISA following *i.v.* administration at 30 µg/mouse alone or with an anti-tag IgG (50 µg/mouse). Fab in pooled mouse serum (n = 5/time point) was quantified by ELISA using heat-killed *P. aeruginosa* O6ad cells (10⁸ cells/ml) as a coating antigen; data represent the means ± SE of three replicates.

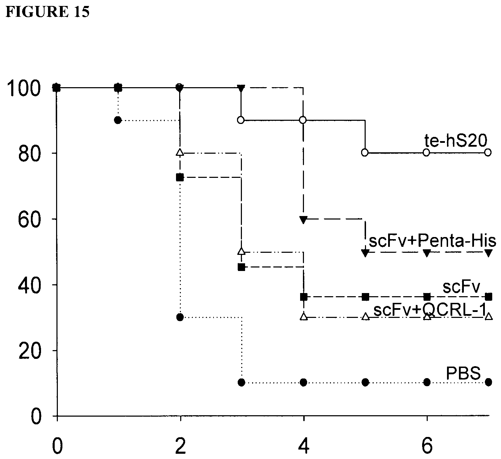

Figure 15 summarizes data showing survival of *P. aeruginosa* O6ad-infected leukopenic mice following treatment with anti-O6ad antibodies. Mice (n = 10-11/group) were treated *i.v.* with te-hS20 or scFv-Penta-His complexes (32 µg/mouse for scFv and 80 µg/mouse for mAb) at time zero; 15 min later mice were inoculated *i.v.* with a LD₈₀₋₉₀ of live P. *aeruginosa* O6ad (10³ CFU/mouse). Animals receiving the same volume of PBS, scFv alone, or scFv plus QCRL-1 were used as controls. Mortality was recorded daily for 7 days.

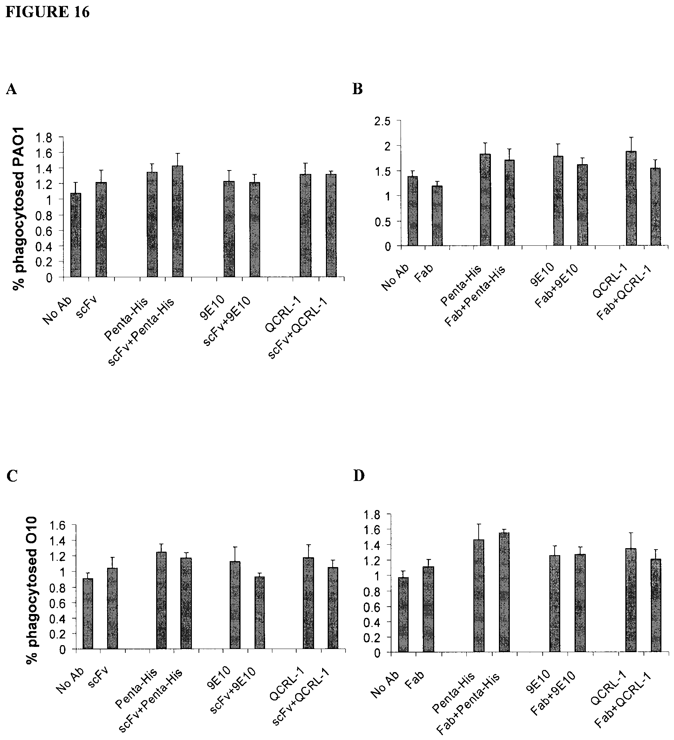

Figure 16 summarizes data showing rAb-anti-tag IgG complex-mediated phagocytosis of non-specific P. *aeruginosa* PAO1 and O10 (1 x 10⁶ CFU) by macrophage J774.1A (1 x 10⁵ cells). **A** and **C,** scFv-anti-tag IgG complex-mediated phagocytosis against PAO1 and O10, respectively; **B** and **D,** Fab-anti-tag IgG complex-mediated phagocytosis against PAO1 and O10, respectively. Incubations of the bacteria with J774.1A cells in the absence of antibodies or in the presence of rAb alone, anti-tag IgG alone, or mixture of rAbs plus QCRL-1 were used as controls. The antibody concentrations used were 335 nM for rAbs and 167.5 nM for anti-tag IgGs. The phagocytosed population of bacteria was calculated according to the formula % phagocytosed bacteria = (phagocytosed bacterial number at the end of 30 min incubation/initial bacterial number at the beginning of 30 min incubation) x 100%. Data represent the means ± SE from a single experiment performed at least in triplicate.

### DETAILED DESCRIPTION OF THE DISCLOSURE

### I. Uses For Improving Therapeutic Efficacy of Antibody Fragments

As described above, the present inventors discovered a method of improving the therapeutic efficacy and utility of antibody fragments by employing anti-epitope-tagging technologies, which resulted in antibody fragments that exhibited an increased *in vivo* persistence, enhanced antigen binding avidity, the ability to recruit downstream immune system functions to the target antigen specified by the antibody fragment, enhanced in vivo protective efficacy against infection from bacteria or viruses and/or the toxins thereof. The method of enhanced therapeutic efficacy, utility and potency involves non-covalent interactions between the epitope-tagged antibody fragments and anti-epitope tagged antibodies, which results in the formation of a complex between the epitope-tagged antibody fragment and the anti-epitope antibody. Specifically, the present inventors demonstrated that increased therapeutic utility was achieved by the non-covalent binding between epitope-tagged rAb fragments (e.g. 6xHis-tagged scFv) and an anti-epitope tag IgG (e.g. anti-Penta-His) that resulted in the formation of a bivalent rAb-IgG complex.

The inventors used two different murine anti-epitope tag IgG1 Abs (i.e. anti-c-Myc and anti-Penta-His) in combination with an epitope-tagged (i.e. c-Myc and 6xHis) murine *anti-Salmonella enterica* serovar *typhimurium* (*S. typhimurium*) scFv, to examine both *in vivo* persistence in mice and FcR-mediated complement recruitment and phagocytosis of S. *typhimurium* by the murine macrophage (MΦ)-like cell line J774. When compared to the monovalent scFv controls, the data showed that bivalent rAb-IgG complexes recruited Fc-mediated effector functions as demonstrated by the binding of human complement C1q by ELISA and by greater phagocytosis of *S. typhimurium* by J774 MΦ cells following treatment with the B5-1-anti-tag IgG complexes (Figures 4 and 5). Increased *in vivo* serum persistence of rAb fragments was demonstrated by data showing greater quantities of epitope-tagged scFvs (i.e. B5-1 and T1#10) and V_{H}H at various time points following i.v. administration to CD1 mice (Figure 6).

The inventors also used murine anti-epitope tag IgG1 Abs (i.e. anti-5xHis IgG (Penta-His) and anti-c-myc IgG (9E10)) in combination with c-myc- and 6xHis-tagged Fab and scFv, which were directed against *Pseudomonas aeruginosa* O6ad lipopolysaccharide (LPS) to examine their *in vitro* antigen binding ability, *in vivo* serum persistence, ability to mediate effector functions including complement fixation, complement-dependent cytotoxicity (CDC) and bacterial opsonization for phagocytosis, and their protective efficacy against bacterial infection. The data showed that complexes with the anti-tag IgGs significantly improved the antigen binding avidity of both the Fab and scFv (Figures 7-8), extended the serum persistence of the Fab (Figure 14), effectively recruited Fc-dependent effector functions including complement deposition and opsonization of the target bacteria by macrophages *in vitro* (Figures 9-13), and enhanced *in vivo* protective efficacy of the anti-O6ad scFv against infection with P. *aeruginosa* as demonstrated by prolonged animal survival (Figure 15).

In summary, the present inventors demonstrated that 1) terminal epitope tags expressed on antibody fragments specifically recruited functional Fc regions, supplied by full-length anti-epitope tag IgGs, to antigens targeted by the epitope-tagged rAb fragments; 2) an epitope-tagged antibody fragment in complex with an anti-epitope tag IgG increased *in vivo* persistence of the antibody fragment; 3) an epitope-tagged antibody fragment in complex with an anti-epitope tag IgG enhanced target-binding avidity of the antibody fragment; and 4) complex formation of the epitope-tagged antibody fragment with anti-epitope tag IgG enhanced protective efficacy of the antibody fragment against bacterial infection and/or prolonged survival.

The present disclosure addresses the need for improving the therapeutic utility, efficacy and potency of antibody fragments, which are produced more efficiently and at a lower expense as compared to full length antibodies, but which are suitable for use in situations where activation of downstream immune system functions including Fc-mediated effector functions are desired. The antibody fragments linked to epitopes described herein may be developed and produced quickly in simple microbial bioreactor systems such as bacteria and yeast. The antibodies comprising sites that bind to the epitopes described herein (anti-epitope antibodies) are full-length or near full-length antibodies that may be produced in more complex dedicated bioreactor systems such as mammalian cells, and plants, when large-scale production is warranted. The uses described herein provides the biopharmaceutical industry substantial flexibility to adapt to antibody specificity and capacity needs by prioritizing full-length anti-epitope antibody production in more complex bioreactors, and epitope-tagged antibody fragments in simpler bioreactors.

Disclosed herein is a method of enhancing the efficacy of an antibody fragment comprising administering an effective amount of the antibody fragment linked to an epitope to an animal in need thereof wherein a complex forms between the antibody fragment linked to the epitope and an antibody that binds to the epitope. In one embodiment, the antibody fragment is covalently linked to an epitope. The present invention includes the use of an antibody fragment linked to an epitope to enhance the efficacy of the antibody fragment in an animal in need thereof wherein upon use a complex forms between the antibody fragment linked to the epitope and an antibody that binds to the epitope. Disclosed herein is the use of an antibody fragment linked to an epitope in the manufacture of a medicament to enhance the efficacy of the antibody fragment in an animal in need thereof wherein upon use a complex forms between the antibody fragment linked to the epitope and an antibody that binds to the epitope.

As used herein the term "enhancing efficacy" in reference to an antibody fragment linked to an epitope includes without limitation increasing the therapeutic effect of the antibody fragment, increasing the half-life of the antibody fragment, increasing persistence of the antibody fragment, including for example, increasing serum persistence of the antibody fragment, increasing potency of the antibody fragment and/or increasing the utility of the antibody fragment.

The term "antibody fragment" as used herein includes any fragment that is capable of binding to a target antigen and that is linked to an epitope. An antibody fragment may be a small fragment or derivative of a larger antibody, however derived, that recognizes a target antigen, including but not limited to, scFv antibodies, disulphide stabilized scFv fragments and V_{HH} single domain antibodies, such as those of the camelid family, V_{H}, V_{L}, F_{V}, ScAb, HcAb and Fab. A V_{HH} antibody is the single heavy chain variable domain of a heavy chain antibody of the type that can be found in Camelid mammals which are naturally devoid of light chains. A Fab antibody is the Fv (variable) domain of an antibody. The techniques for preparing and using various antibody-based constructs and fragments are well known by those of skill in the art.

In one embodiment, the antibody fragment is scFv. An scFv (single-chain Fv) antibody is a genetically engineered monospecific binding protein that has a specific affinity for an antigen target. An scFv is a derivative of the Fv portion of an antibody molecule, or other receptor molecule of the Ig superfamily. It comprises one heavy and one light chain variable region (V_{H} and V_{L,} respectively) of an antibody, joined by a flexible peptide linker. The scFv antibody fragment contains all of the information required to determine antigen specificity and none of the constant region domain that activates downstream effector functions. An scFv antibody fragment is generally in the size range of 25 to 30 kD, and therefore is small enough to be synthesized efficiently in a bacterial or yeast expression system. Because of their small size, scFv antibody fragments have a short circulating half-life as compared to full-size antibodies, or other antibody derivatives (i.e. diabodies, minibodies).

In another embodiment, the antibody fragment is Fab. Fab is the Fv (variable) domain of an antibody. Fab fragments are disulphide-linked papain-cleavage fragments derived from whole antibodies. A Fab antibody fragment comprises one constant and one variable domain of each of the heavy and the light chain of antibody and is a region on the antibody that binds to antigens. A Fab antibody fragment is generally in the size range of 50 kDa, and therefore is small enough to be synthesized efficiently in a bacterial [7] or yeast expression system. Fab antibody fragments may also be made recombinantly in mammalian cell bioreactors or plants. Fab antibody fragments are intended to be included herein as useful epitope-tagged antibody fragments, which may be so-produced and a suitable peptide epitope could be covalently linked.

The specificity of the antibody fragment will be selected based on the antigen that one wishes to target in the animal. In the uses of the present invention the target antigen is selected from viral antigens or bacterial antigens. The antibody fragment can be generated using techniques known in the art or can be a known antibody that is readily available. The CDR regions of many antibodies are readily available which facilitates the recombinant production of antibody fragments.

The term "epitope" as used herein means an antigenic determinant that may be bound by an antibody, and may be a peptide derived from a toxin, pathogen, virus, bacteria, tumour antigen or autoantigen, and includes an antigen for which an animal has been previously immunized. For example, infants and children are immunized and/or vaccinated with one or more of the following immunizations and/or vaccines: Diphtheria, tetanus, acellular pertussis and inactivated polio virus vaccine (DTaP-IPV); *Haemophilus influenzae* type b conjugate vaccine (Hib); Measles, mumps and rubella vaccine (MMR); Varicella vaccine (Var); Hepatitis B vaccine (HB); Pneumococcal conjugate vaccine - 7-valent (Pneu-C-7); Pneumococcal polysaccharide - 23-valent (Pneu-P-23); Meningococcal C conjugate vaccine (Men-C); Diphtheria, tetanus, acellular pertussis vaccine - adult/adolescent formulation (Tdap); Diphtheria, tetanus vaccine (Td); Influenza vaccine (Inf); IPV Inactivated polio virus. In addition, adults with specific risk indications may also be immunized and/or vaccinated with one or more of the following immunizations and/or vaccines: Influenza; Pneumococcal polysaccharide; Hepatitis A and B; Bacille Calmette- Guerin (BCG); Cholera; Japanese encephalitis; Poliomyelitis; Meningococcal conjugate; Meningococcal polysaccharide; Rabies, pre-exposure use; Typhoid; Yellow fever; Smallpox.

In another embodiment, the epitope may be a peptide epitope contrived and made completely unique from any amino acid sequences found in nature. In another embodiment, the epitope may be a small molecule hapten such as fluorescein. This embodiment would require the corresponding anti-hapten mAb to be coadministered.

The epitope is of no specific length, and can be any size upwards of 3 amino acid residues in length, including the size of a full-length protein such as glutathione S transferase (GST) and maltose binding protein (MBP). Smaller epitopes are preferred, for example epitopes between 8 to 50 amino acids in length, as these can result in smaller epitope-tagged antibody fragments that are easier to produce, purify and use as compared to larger recombinant proteins. Additionally, smaller epitopes are less likely to interfere with the target antigen binding function of the antibody fragment. Commonly used epitope tags include glutathione-S-transferase (GST), c-Myc, polyhistidine (6X-His), penta-histidine (Penta-His), FLAG®, green fluorescent protein (GFP), maltose binding protein (MBP), influenza A virus haemaglutinin (HA tag; YPYDVPDYA (SEQ ID NO: 1)), β-gaiactosidase (β-gal), GAL4, human MRP, V5 epitope from the simian virus, polyoma virus T antigen epitopes, and the KT3 viral epitope or portions thereof of these proteins. Some epitopes including c-Myc, QCRL-1 and poly-His epitopes are listed in Table 1, and provide examples of various epitopes and their known antibodies which bind to the epitopes, which can be used in the present disclosure. In another embodiment, the epitope may be any epitope that is experimentally determined to raise a monoclonal response in an animal that results in a high-affinity antibody for a defined peptide epitope. "A portion" of the above named proteins is any sequence of 3 amino acids or longer.

The phrase "antibody fragment linked to an epitope" as used herein may be used interchangeably with "epitope-tagged antibody fragment" in the present disclosure. As used herein the term "linked" includes an epitope attached to the antibody fragment using techniques known in the art, including for example recombinant DNA techniques such as fusion protein technology or by chemical means such as cross-linking. The method used to link the antibody fragment to an epitope must be capable of linking the antibody fragment and epitope without interfering with the ability of the antibody fragment to bind to its target antigen. In one embodiment, the antibody fragment is covalently linked to an epitope.

In one embodiment, the antibody fragment is linked to an epitope using recombinant DNA techniques. In such a case a DNA sequence encoding the antibody fragment is fused to a DNA sequence encoding the epitope, resulting in a chimeric DNA molecule. The chimeric DNA sequence is transfected into a host cell that expresses the fusion protein. The fusion protein can be recovered from the cell culture and purified using techniques known in the art. In another embodiment, the nucleotide sequence encoding the epitope could be fused to the DNA sequence encoding the antibody fragment at carboxy-terminus, or distant from the antigen-binding site of the epitope-tagged antibody. In a further embodiment, epitope tagged antibody fragments may be screened out from recombinant antibody libraries, which typically have epitope tags genetically engineered so as to be present on any rAb.

In another embodiment, the antibody may be linked to an epitope via chemical cross-linking using techniques well known in the art. There are several hundred crosslinkers available that can conjugate two proteins. (See for example "Chemistry of Protein Conjugation and Crosslinking". 1991, Shans Wong, CRC Press, Ann Arbor). The crosslinker is generally chosen based on the reactive functional groups available or inserted on the antibody fragment, and/or the epitope. In addition, if there are no reactive groups, a photoactivatible crosslinker can be used. In certain instances, it may be desirable to include a spacer between the antibody fragment, and epitope. Crosslinking agents known to the art include the homobifunctional agents: glutaraldehyde, dimethyladipimidate and bis(diazobenzidine) and the heterobifunctional agents: m-maleimidobenzoyl-N-hydroxysuccinimide and sulfo-m-maleimidobenzoyl-N-hydroxysuccinimide.

As used herein, the phrase "effective amount" means an amount effective, at dosages and for periods of time necessary to achieve the desired result. Effective amounts of the antibody fragment linked to an epitope may vary according to factors such as the disease state, age, sex, weight of the animal. Dosage regime may be adjusted to provide the optimum therapeutic response. For example, several divided doses may be administered daily or the dose may be proportionally reduced as indicated by the exigencies of the therapeutic situation.

The term "animal" as used herein refers to any member of the animal kingdom, preferably a mammal, more preferably a human being.

The term "administered" as used herein means that the antibody fragment linked to an epitope may be administered or used either as a protein conjugate or as a chimeric nucleic acid construct. In the latter instance the antibody fragment linked to the epitope will be expressed *in vivo* in a DNA-based therapy. The form of administration or use will depend on the nature and location of the target antigen. Suitable forms of administration include systemic (subcutaneous, intravenous, intramuscular), oral administration, inhalation, transdermal administration, topical application (such as topical cream or ointment, etc.) or by other methods known in the art. Other modes of administration are described in Section II under "Pharmaceutical Compositions".

The administration or use of the antibody fragment linked to the epitope results in the formation of a complex between the antibody fragment linked to the epitope (epitope-tagged antibody fragment) and an antibody that binds to the epitope (anti-epitope antibody). As used herein the term "complex" refers to the non-covalent interaction between the epitope-tagged antibody fragment, and the anti-epitope antibody. In one embodiment, a bivalent rAb-IgG complex forms between an anti-epitope IgG1 antibody (IgG) and an epitope-tagged antibody fragment (rAb). This is shown schematically in Figure 1B. In another embodiment, the antibody fragment forms a complex with the antibody in a 20:1 ratio. In a further embodiment, the antibody fragment forms a complex with the antibody in a 2:1 ratio.

The term "antibody that binds to the epitope" as used herein may be used interchangeably with "anti-epitope antibody" and includes full-length or near full-length antibodies that can enhance the efficacy of the antibody fragment. In one embodiment, the anti-epitope antibody will comprise an Fc region. The antibodies may be wild-type antibodies and natural variants thereof, and molecularly-engineered antibodies, polyclonal and monoclonal antibodies, IgG, IgM, IgA, IgE or IgD antibodies, humanized antibodies, crosslinked antibodies, heterospecific antibodies, bispecific antibodies, crosslinked heterobispecific antibodies, chimeric antibodies, minibodies, diabodies, triabodies, HCAb, Dab, Scab, V_{H}, V_{L}, F_{V} and Fab. In another embodiment, the antibody includes IgG1, IgG2a/c, IgG2b, and/or IgG3. In a further embodiment, the anti-epitope antibody may be a full-length polyclonal or monoclonal antibody selected from the group consisting of IgG, IgM, IgA, IgE and IgD.

In one embodiment, the anti-epitope antibody includes all isotypes of IgG and IgM antibodies, including for example monoclonal antibodies, as these can be produced industrially. In another embodiment, the anti-epitope antibody includes a full-length polyclonal or monoclonal antibody capable of activating all downstream effector functions. A polyclonal or monoclonal antibody useful in the present disclosure can be obtained as a cell line from the American Type Culture Collection of monoclonal antibodies, from commercially available sources, from polyclonal sources (i.e. animal- or serum-derived), or produced through recombinant DNA technology in a bioreactor (hybridoma, plant, etc.). In one embodiment, for human therapy the anti-epitope antibody is human or humanized. In another embodiment, for animal therapy, the anti-epitope antibody is from the same animal.

Examples of anti-epitope antibodies with a binding domain that may be useful in the present disclosure include antibodies specific for c-Myc and QCRL-1 epitopes, which have an affinity (K_{d}) for these epitopes of less than 200 nM, and are listed in Table 1. Other anti-epitope antibodies useful in the present disclosure, and the epitopes that they recognize, are also listed in Table 1. It is understood that other anti-epitope antibodies may be known, or can be generated, which may have an affinity (K_{d}) for their epitope that is lower or higher than 200 nM, and these may useful in the methods of the disclosure disclosed herein.

Anti-epitope antibodies suitable for the methods and uses of the present disclosure may be readily selected by persons skilled in the art depending on the therapeutic effect sought (i.e. increased persistence or half-life, and/or activation of downstream immune system functions and/or improved or enhanced protective efficacy against infection from bacteria, viruses or the toxins thereof. For example, the antibody may be selected such that it comprises a functional Fc region or derivative thereof, and may therefore be able to activate downstream immune system functions. The Fc region of immunoglobulin antibodies are known to trigger ADCC, the complement pathway and opsonization. "Derived from" includes a natural variant of a wild-type Fc sequence, a genetically- or biochemically-engineered variant thereof, or an entirely artificial amino acid sequence.

In addition, if the anti-epitope antibody is intended to be used for neutralizing a toxin, such as botulinum toxin, an anti-epitope antibody that increases the half-life of epitope-tagged antibody fragment, is sufficient for use herein. This anti-epitope antibody could be a
full-length antibody, for example, a full-length antibody produced from a plant and scaled up in production. Other potential anti-epitope antibodies useful herein for binding to the epitope-tagged antibody fragment include scFv, V_{HH}, V_{H}, V_{L}, Fab, F_{V}, ScAb, HcAb, diabodies, triabodies and minibodies. These provide the advantage that they can be produced in simple bacterial or yeast bioreactors, as opposed to full-length antibodies. However, it is understood by those skilled in the art that the smaller the anti-epitope antibody, the lesser can likely be its ability to enhance the efficacy of the epitope-tagged antibody fragment. Accordingly, there is a trade-off between ease of manufacture of the anti-epitope antibody and ability to enhance the efficacy of the epitope-tagged antibody fragment.

Furthermore, if it is desired that the anti-epitope antibody also activates downstream effector functions, in addition to increasing the persistence or half-life and/or stability of the epitope-tagged antibody fragment (i.e., the antibody fragment is intended to be used to bind to a pathogen, such as a virus or a bacterium) the anti-epitope antibody may be produced by a mammalian system, such as a cell-line bioreactor, so as to allow for complete downstream immune system function. In this regard, full-length polyclonal or monoclonal antibodies are preferred. Furthermore, if the anti-epitope antibody is from a mammalian system, or if it has human specific or compatible glycosylations, it can activate downstream immune system function against the target antigen of the epitope-tagged antibody fragment, thus transforming the tagged antibody into a therapeutic antibody that can be used in situations where an FcR-mediated effector functions are desired.

The anti-epitope antibody is administered or used prior to, at the same time, or after the epitope-tagged antibody fragment. Disclosed herein, the anti-epitope antibody is already present in the animal.

As disclosed herein "already present in the animal" includes an antibody that is generated by immunizing the animal with an epitope and/or using the epitope in the animal. Alternatively, the phrase also includes an antibody that is generated by an immunization previously administered to the animal and/or previously used in the animal, wherein the immunization comprised the epitope. For example, previously administered immunizations may include: Diphtheria, tetanus, acellular pertussis and inactivated polio virus vaccine (DTaP-IPV); *Haemophilus influenzae* type b conjugate vaccine (Hib); Measles, mumps and rubella vaccine (MMR); Varicella vaccine (Var); Hepatitis B vaccine (HB); Pneumococcal conjugate vaccine - 7-valent (Pneu-C-7); Pneumococcal polysaccharide - 23-valent (Pneu-P-23); Meningococcal C conjugate vaccine (Men-C); Diphtheria, tetanus, acellular pertussis vaccine - adult/adolescent formulation (Tdap); Diphtheria, tetanus vaccine (Td); Influenza vaccine (Inf); IPV Inactivated polio virus; Influenza; Pneumococcal polysaccharide; Hepatitis A and B; Bacille Calmette- Guerin (BCG); Cholera; Japanese encephalitis; Poliomyelitis; Meningococcal conjugate; Meningococcal polysaccharide; Rabies, pre-exposure use; Typhoid; Yellow fever; and Smallpox. Disclosed herein, the antibody already present in the animal is a monoclonal antibody, IgG, or IgG1.

Disclosed herein is a method of enhancing the efficacy of an antibody fragment comprising: a) immunizing an animal with an epitope; and b) administering an effective amount of the antibody fragment linked to the epitope to the animal in need thereof; wherein the efficacy of the administered antibody fragment is enhanced. Also disclosed herein is a use to enhance efficacy of an antibody fragment in an animal in need thereof comprising: a) use of an epitope to immunize the animal; and b) use of the antibody fragment linked to the epitope in the animal to enhance efficacy of the antibody fragment. Also disclosed herein is a use to enhance efficacy of an antibody fragment in an animal in need thereof comprising: a) use of an epitope to immunize the animal; and b) use of the antibody fragment linked to the epitope in the animal in the manufacture of a medicament to enhance efficacy of the antibody fragment.

"Immunizing an animal" with an epitope and/or use of an epitope in an animal allows the animal to produce native or natural antibodies that are raised against the epitope (anti-epitope antibodies). Accordingly, immunizing the animal or using the epitope in the animal negates the need for coadministering the epitope-tagged antibody fragment with an anti-epitope antibody but rather provides an anti-epitope antibody that is made by the animal itself. Disclosed herein, an epitope may be used in an immunization protocol in the case of an animal requiring continual immunotherapy, thus reducing the need for administration of the full-length anti-epitope antibody in a long-term immune response therapy protocol.

In another embodiment, the formation of epitope-tagged antibody fragments:anti-epitope antibody complexes provides a basis from which oligoclonal or polyclonal antibody (pAb) therapeutics can be created, including for example, administration of pAb repertoires that mimic the natural Ab response aimed towards multiple target antigens. pAb production using the methods of present disclosure disclosed herein would require only one, or alternatively a few, humanized anti-epitope antibodies (i.e. IgG molecules), while multi-antigen specificities are supplied by antibody fragments linked to different epitope tags. A facility producing the anti-epitope antibodies, in combination with the rapid and inexpensive production of polyclonal epitope-tagged antibody fragments, could provide speed and flexibility in pAb development. In one embodiment, only one anti-epitope antibody may be required to deliver several therapeutic epitope-tagged antibody fragments. In another embodiment, a few anti-epitope antibody molecules could be tailored for specific and multiple types of therapeutic outcomes.

For example, the epitope-tagged antibody fragments described herein may differ from one another in regard to their affinity for various antigenic targets - i.e. toxins, pathogens, idiotypes and autoantigen targets. They are linked or tagged with an epitope recognized by an anti-epitope antibody. The anti-epitope antibody recognizes and binds to the epitope of an epitope-tagged antibody fragment. More than one anti-epitope antibody may be generated, which may differ in their ability to recognize different epitope tags, and/or in their ability to activate downstream immune functions, to thereby increase the flexibility of the disclosure described herein.

Accordingly, disclosed herein is the generation of a number of epitope-tagged antibody fragments that recognize different antigenic targets, and the corresponding generation of one or a few anti-epitope antibodies that recognize the epitope tag of the antibody fragments. By changing the tagged antibody fragment that is combined with an anti-epitope antibody, a large number of different therapeutic antibody fragments with different specific affinities towards antigens, or different abilities to activate downstream immune functions, can be generated. Because a relatively large number of epitope-tagged antibody fragments required can be produced quickly and inexpensively, and only one or a few complex anti-epitope antibodies are required, this approach provides the therapeutic antibody industry substantial flexibility to adapt to antibody specificity and capacity needs.

In one embodiment, the present disclosure includes methods and uses of a plurality of epitope-tagged antibody fragments, which differ in their capability of binding different antigens, but are all tagged with the same epitope. Accordingly, these antibody fragments may be used in conjunction with one anti-epitope antibody which binds to the epitope tag on the antibody fragments.

In another embodiment, more than one epitope tag may be linked to the antibody fragment. For example, two or three copies of the same epitope can be linked along antibody fragment to provide for increased binding of the anti-epitope antibody. Alternatively, two or more different epitopes can be linked to one antibody fragment. For example, two different epitopes could be spaced on the antibody fragment to provide for an epitope-tagged antibody fragment that is recognized by two different anti-epitope antibodies. For example, c-Myc and polyhistidine epitopes may be combined on one antibody fragment.

In a further embodiment, an anti-epitope antibody is monospecific for an epitope. However, in another embodiment, the anti-epitope antibody is bispecific for two different epitopes. This embodiment of the anti-epitope antibody could be used with epitope-tagged antibody fragments that comprise either, or both, of the two epitope tags recognized by the bispecific anti-epitope antibodies.

In another aspect of the method of this disclosure, a plurality of epitope-tagged antibodies are provided, from which one or more is selected for administration to the animal or use in the animal in combination with an anti-epitope antibody.

In some situations it may be beneficial to select, for administration, two or more epitope-tagged antibody fragments that each have a specific affinity for the same antigen- i.e. two tagged antibody fragments that each have a slightly different binding affinity for a particular antigen. In other situations it may be beneficial to administer two or more epitope-tagged antibody fragments that have a specific affinity for different but related antigens. For example, the different antigens may be two different proteins on the membrane surface of the same pathogen or, a toxin produced by a pathogen, and the pathogen itself. In some situations, the epitope on the two epitope-tagged antibody fragments may be the same (i.e., so they are recognized by the anti-epitope antibody), or they may be different (*i.e*., so that they are recognized by different anti-epitope antibodies).

Once the appropriate tagged antibody fragment, or combination of tagged antibody fragments, is selected, it can be combined with the appropriate anti-epitope antibody, to form a complex as described herein. The anti-epitope antibody can be an antibody that can only function to stabilize the tagged antibody fragment (*i.e*., used for incomplete immunotherapy), or it can be an antibody that also activates downstream immune system functions. More than one anti-epitope antibody can be used, and they can have different specific affinities for the epitope, or recognize different epitopes, altogether. The anti-epitope antibody may be a natural antibody that is made by the animal, as a result of immunization with the epitope.

In one embodiment, the epitope-tagged antibody fragment and anti-epitope antibody are combined before administration to the animal or use in the animal. It is preferred that, if a complex as described herein is to be generated by mixing an anti-epitope antibody and an epitope-tagged antibody fragment together before administration to an animal or used in an animal, that such administration or use occurs immediately or within 30 minutes of mixing. In another embodiment, anti-epitope antibody and epitope-tagged antibody fragment are coadministered or used separately in the animal. In yet another embodiment, anti-epitope antibody and epitope-tagged antibody fragment are administered or used sequentially. In the latter embodiment, anti-epitope antibody may be administered or used either before, or after, epitope-tagged antibody fragment. It is also preferred in this embodiment to administer or use anti-epitope antibody first. Disclosed herein, epitope-tagged antibody is administered or used and it combines with natural anti-epitope antibody produced by the animal.

Disclosed herein is a method of increasing the therapeutic effect of an antibody fragment comprising administering an effective amount of the antibody fragment linked to an epitope to an animal in need thereof, wherein a complex forms between the antibody fragment linked to the epitope and an antibody that binds to the epitope. The present invention includes the use of an antibody fragment linked to an epitope to increase the therapeutic effect of the antibody fragment in an animal in need thereof wherein upon use a complex forms between the antibody fragment linked to the epitope and an antibody that binds to the epitope. Also disclosed herein is the use of an antibody fragment linked to an epitope in the manufacture of a medicament to increase the therapeutic effect of the antibody fragment in an animal in need thereof wherein upon use a complex forms between the antibody fragment linked to the epitope and an antibody that binds to the epitope.

The term "increasing or increases the therapeutic effect" as used herein includes without limitation increasing the persistence or half-life of the antibody fragment, including, for example, increasing serum persistence or serum half-life of the antibody fragment and/or stability of the antibody fragment, increasing the immune response of the antibody fragment, for example, by activating downstream immune system functions, such as the ability to recruit FcR-mediated effector functions, and includes for example recruiting the complement system, enhanced avidity of the antibody fragment and/or increasing phagocytosis, and enhancing protective efficacy against infection from bacteria or viruses, or the toxins thereof.

Disclosed herein is a method of increasing persistence and/or stability of an antibody fragment comprising administering an effective amount of the antibody fragment linked to an epitope to an animal in need thereof wherein a complex forms between the antibody fragment linked to the epitope and an antibody that binds to the epitope. The present invention includes the use of an antibody fragment linked to an epitope to increase the persistence and/or stability of the antibody fragment in an animal in need thereof wherein upon use a complex forms between the antibody fragment linked to the epitope and an antibody that binds to the epitope. Also disclosed herein is the use of an antibody fragment linked to an epitope in the manufacture of a medicament to increase the persistence and/or stability of the antibody fragment in an animal in need thereof wherein upon use a complex forms between the antibody fragment linked to the epitope and an antibody that binds to the epitope.

As used herein "persistence and/or stability" includes increased in vivo persistence, increased serum persistence or increased serum half-life (including increased in vivo serum persistence or increased in vivo serum half-life), and/or a reduction in the rate of degradation or clearance of the antibody fragment described herein, so that it remains capable of, or available for, binding to its target antigen for a longer period of time.

The antibody fragments disclosed herein are small proteins and therefore may be unstable, for example in serum, meaning that the antibody fragments may be degraded or cleared from the serum more rapidly than is desired for therapeutic applications. Accordingly, by employing the methods of the present disclosure, the antibody fragments are not cleared as rapidly, exhibit an increased persistence or half-life and/or stability and are thus are useful for exerting therapeutic effects for longer periods of time. In other words, the persistence or half-life of the epitope-tagged antibody fragment may be improved, resulting in an increased persistence or half-life of the antibody fragment in the animal in need thereof. As is apparent, the longer that the antibody fragment is able to bind to its target antigen, the longer can be its therapeutic effectiveness.

Increased persistence, half-life and/or stability may be measured using techniques known in the art, for example, by labeling the antibody fragment with a fluorescein label, and then comparing the amount of label remaining in the serum. If after a certain period of time, for example one hour, or one day, there is a statistically significant higher amount of fluorescein in the serum as compared to controls, the antibody fragment exhibits an increased half-life and/or stability. As is apparent to one of skill in the art, a stable epitope-tagged antibody fragment can have a longer persistence or half-life in the serum, peritoneum or other tissue to which it is administered, as compared to controls. Whether there is a statistically significant difference higher amount of fluorescein tag may be determined by analyzing fluorescent readings with GraphPad Prism (GraphPad Software Inc.) using 1 way ANOVA analysis of variance, followed by Dunnett's multiple comparison test, in which each experimental group is compared to the control values at the corresponding time point. A difference is statistically significant if it has a P value of <0.05.

Disclosed herein is a method of increasing the immune response of an antibody fragment comprising administering an effective amount of the antibody fragment linked to an epitope to an animal in need thereof wherein a complex forms between the antibody fragment linked to the epitope and an antibody that binds to the epitope. The present invention includes the use of an antibody fragment linked to an epitope to increase the immune response of the antibody fragment in an animal in need thereof wherein upon use a complex forms between the antibody fragment linked to the epitope and an antibody that binds to the epitope. Also disclosed herein is the use of an antibody fragment linked to an epitope in the manufacture of a medicament to increase the immune response of the antibody fragment in an animal in need thereof wherein upon use a complex forms between the antibody fragment linked to the epitope and an antibody that binds to the epitope.

The terms "increasing or increase the immune response" "eliciting an immune response" or "inducing an immune response" as used herein includes increasing the immunotherapeutic potential of the antibody fragment including initiating, triggering, causing, enhancing, improving or augmenting any response of the immune system, for example, of either a humoral or cell-mediate nature. The initiation or enhancement of an immune response can be assessed using assays known to those skilled in the art including, but not limited to, antibody assays (for example ELISA assays), antigen specific cytotoxicity assays and the production of cytokines (for example ELISPOT assays). In one embodiment, the methods of the present disclosure trigger or enhance a cellular immune response, antibody dependent cell cytotoxicity (ADCC; cell lysis), phagocytosis, antigen presentation and T cell activation, and/or immune activation via cytokine release, for example, increased FcR and MHC expression.

Disclosed herein is a method of activating downstream immune system functions comprising administering an effective amount of the antibody fragment linked to an epitope to an animal in need thereof wherein a complex forms between the antibody fragment linked to the epitope and an antibody that binds to the epitope. The present invention includes the use of an antibody fragment linked to an epitope
to activate downstream immune system functions in an animal in need thereof wherein upon use a complex forms between the antibody fragment linked to the epitope and an antibody that binds to the epitope. Also disclosed herein is the use of an antibody fragment linked to an epitope in the manufacture of a medicament to activate downstream immune system functions in an animal in need thereof wherein upon use a complex forms between the antibody fragment linked to the epitope and an antibody that binds to the epitope.

The term "activating or activate downstream immune system functions" as used herein includes for example one or more of: (a) activating FcR-mediated effector functions; (a) activating the complement cascade for complement dependent cytotoxicity (CDC); (b) directing the immune system through Fc receptor function in antibody-dependent cell-mediated cytotoxicity (ADCC); (c) increasing avidity of the antibody fragment; and (d) opsonization.

Disclosed herein is a method of recruiting FcR-mediated effector functions comprising administering an effective amount of the antibody fragment linked to an epitope to an animal in need thereof wherein a complex forms between the antibody fragment linked to the epitope and an antibody that binds to the epitope. The present invention includes the use of the antibody fragment linked to an epitope to recruit FcR-mediated effector functions in an animal in need thereof wherein upon use a complex forms between the antibody fragment linked to the epitope and an antibody that binds to the epitope. Also disclosed herein is the use of the antibody fragment linked to an epitope in the manufacture of a medicament to recruit FcR-mediated effector functions in an animal in need thereof wherein upon use a complex forms between the antibody fragment linked to the epitope and an antibody that binds to the epitope.

The term "recruiting or recruit FcR-mediated effector functions" as used herein means the ability to recruit the complement system, and/or increase phagocytosis to the target antigens specified by the epitope-tagged antibody fragment, including, for example, opsonic phagocytosis.

Disclosed herein is a method of recruiting the complement system and/or increasing phagocytosis comprising administering an effective amount of the antibody fragment linked to an epitope to an animal in need thereof wherein a complex forms between the antibody fragment linked to the epitope and an antibody that binds to the epitope. The present invention includes the use of an antibody fragment linked to an epitope to recruit the complement system and/or increase phagocytosis in an animal in need thereof wherein upon use a complex forms between the antibody fragment linked to the epitope and an antibody that binds to the epitope. Also disclosed herein is the use of an antibody fragment linked to an epitope in the manufacture of a medicament to recruit the complement system and/or increase phagocytosis in an animal in need thereof wherein upon use a complex forms between the antibody fragment linked to the epitope and an antibody that binds to the epitope.

As used herein "complement system" refers to a complement cascade of proteins or protein fragments, including for example, serum proteins, serosal proteins and cell membrane receptors, which help clear pathogens from an organism. The complement system includes the classical complement pathway, the alternative complement pathway, and the mannose-binding lectin pathway, which would all be known to those skilled in the art. Recruitment of the complement system can be assessed using assays known to those skilled in the art, including, but not limited to, antibody assays (for example ELISA assays, immunofluorescence assays, radioimmunoassays and radioassays involving radiolabeled complement proteins, surface plasmon resonance assays). For example, recruitment of the classical complement system may be determined by assessing recruitment of the first complement protein C1q, for example by determining the ability of the complexes described herein to deposit complement protein C1q or C1q complex.

As used herein "increasing phagocytosis" means an increase in the ingesting, taking in and/or engulfing of particles including for example, pathogens, such as bacteria, viruses, parasites, protozoans and/or yeasts, and cellular and/or foreign debris by phagocytes, including for example, macrophages. Phagocytosis occurs after a particle has bound to receptors that are present on the surface of phagocytes. A number of receptors are present on phagocytes, including for example, opsonins. Phagocytosis can be assessed using assays known to those skilled in the art, including, but not limited to, antibody assays (for example ELISA assays, microscopy assays, macrophage lysis and bacterial colony forming unit enumeration assays, etc.).

Disclosed herein is a method of enhancing protective efficacy against infection comprising administering an effective amount of the antibody fragment linked to an epitope to an animal in need thereof wherein a complex forms between the antibody fragment linked to the epitope and an antibody that binds to the epitope. The present invention includes the use of an antibody fragment linked to an epitope to enhance protective efficacy against infection in an animal in need thereof wherein upon use a complex forms between the antibody fragment linked to the epitope and an antibody that binds to the epitope. Also disclosed herein is the use of an antibody fragment linked to an epitope in the manufacture of a medicament to enhance protective efficacy against infection in an animal in need thereof wherein upon use a complex forms between the antibody fragment linked to the epitope and an antibody that binds to the epitope.

As used herein "enhancing protective efficacy against infection" includes increased protection against infection and/or increased protection efficiency against infection and/or increased protection capacity against infection, which may be assessed by determining survival of infected animals and/or prolonged survival of infected animals after treatment with the complexes described herein. As used herein "infection" includes infection from pathogens, which includes for example infection from bacteria, viruses, protozoans and/or yeasts.

### II. Pharmaceutical Compositions

Disclosed herein is a pharmaceutical composition in a biologically compatible form suitable for use or administration *in vivo.* The term "biologically compatible form suitable for administration *in vivo*" means a form of the substance to be administered in which any toxic effects are outweighed by the therapeutic effects. Disclosed herein is a pharmaceutical composition comprising an effective amount of the epitope-tagged antibody fragments and/or anti-epitope antibodies disclosed herein with a pharmaceutically acceptable carrier or diluent, adjuvant or mixtures thereof to an animal in need thereof.

The compositions containing the epitope-antibody fragments and/or anti-epitope antibodies described herein can be prepared by known methods for the preparation of pharmaceutically acceptable compositions which can be administered to animals, such that an effective quantity of the active substance is combined in a mixture with a pharmaceutically acceptable vehicle, including for example, a carrier or diluent. Suitable vehicles are described, for example, in Remington's Pharmaceutical Sciences (2003 - 20th edition) and in The United States Pharmacopeia: The National Formulary (USP 24 NF19) published in 1999. On this basis, the compositions include, albeit not exclusively, solutions of the substances in association with one or more pharmaceutically acceptable vehicles, carriers or diluents, and contained in buffered solutions with a suitable pH and iso-osmotic with the physiological fluids.

Pharmaceutical compositions disclosed herein include, without limitation, lyophilized powders or aqueous or non-aqueous sterile injectable solutions or suspensions, which may further contain antioxidants, buffers, bacteriostats and solutes that render the compositions substantially compatible with the tissues or the blood of an intended recipient. Other components that may be present in such compositions include water, surfactants (such as Tween),
alcohols, polyols, glycerin and vegetable oils, for example. Extemporaneous injection solutions and suspensions may be prepared from sterile powders, granules, tablets, or concentrated solutions or suspensions. The pharmaceutical composition may be supplied, for example but not by way of limitation, as a lyophilized powder which is reconstituted with sterile water or saline prior to administration to the animal.

The compositions containing the epitope-tagged antibody fragments and/or anti-epitope antibodies disclosed herein may comprise a pharmaceutically acceptable carrier. Suitable pharmaceutically acceptable carriers include essentially chemically inert and nontoxic compositions that do not interfere with the effectiveness of the biological activity of the pharmaceutical composition. Examples of suitable pharmaceutical carriers include, but are not limited to, water, saline solutions, glycerol solutions, ethanol, N-(1(2,3-dioleyloxy)propyl)N,N,N-trimethylammonium chloride (DOTMA), diolesylphosphotidyl-ethanolamine (DOPE), and liposomes. Such compositions should contain a therapeutically effective amount of the compound, together with a suitable amount of carrier so as to provide the form for direct administration to the animal.

The composition disclosed herein may be in the form of a pharmaceutically acceptable salt which includes, without limitation, those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylarnino ethanol, histidine, procaine, etc.

Immunogenicity can be significantly improved if the immunizing agent is regardless of administration format, co-immunized with an immunostimulatory component, such as an adjuvant. Adjuvants enhance the immunogenicity of an immunogen but are not necessarily immunogenic in of themselves. Adjuvants may act by retaining the immunogen locally near the site of administration to produce a depot effect facilitating a slow, sustained release of immunogen to cells of the immune system. Adjuvants can also attract cells of the immune system to an immunogen depot and stimulate such cells to elicit immune response. As such, disclosed herein are compositions of epitope-tagged antibody fragments and/or anti-epitope antibodies further comprising adjuvants.

Adjuvants have been used for many years to improve the host immune responses to, for example, vaccines. Intrinsic adjuvants (such as lipopolysaccharides) normally are the components of killed or attenuated bacteria used as vaccines. Extrinsic adjuvants are immunomodulators which are typically non-covalently linked to antigens and are formulated to enhance the host immune responses. Thus, adjuvants have been identified that enhance the immune response to antigens delivered parenterally. Some of these adjuvants are toxic, however, and can cause undesirable side-effects making them unsuitable for use in humans and many animals. Indeed, only aluminum hydroxide and aluminum phosphate (collectively commonly referred to as alum) are routinely used as adjuvants in human and veterinary vaccines. The efficacy of alum in increasing antibody responses to Diphtheria and Tetanus toxoids is well established.

A wide range of extrinsic adjuvants can provoke potent immune responses to immunogens. These include saponins complexed to membrane protein antigens (immune stimulating complexes), pluronic polymers with mineral oil, killed mycobacteria and mineral oil, Freund's complete adjuvant, bacterial products such as muramyl dipeptide (MDP) and lipopolysaccharide (LPS), as well as lipid A, and liposomes.

In one aspect of the present disclosure, adjuvants useful in any of the embodiments described herein are as follows. Adjuvants for parenteral immunization include aluminum compounds (such as aluminum hydroxide, aluminum phosphate, and aluminum hydroxy phosphate). The antigen can be precipitated with, or adsorbed onto, the aluminum compound according to standard protocols. Other adjuvants such as RIBI (ImmunoChem, Hamilton, MT) can also be used in parenteral administration.

Adjuvants for mucosal immunization include bacterial toxins (e.g., the cholera toxin (CT), the E. coli heat-labile toxin (LT), the Clostridium difficile toxin A and the pertussis toxin (PT), or combinations, subunits, toxoids, or mutants thereof). For example, a purified preparation of native cholera toxin subunit B (CTB) can be of use. Fragments, homologs, derivatives, and fusion to any of these toxins are also suitable, provided that they retain adjuvant activity. Preferably, a mutant having reduced toxicity is used. Suitable mutants have been described (e.g., in WO 95/17211 (Arg-7-Lys CT mutant), WO 96/6627 (Arg-192-Gly LT mutant), and WO 95/34323 (Arg-9-Lys and Glu-129-Gly PT mutant)). Additional LT mutants that can be used in the methods and compositions disclosed herein include, for example Ser-63-Lys, Ala-69-Gly, Glu-110-Asp, and Glu-112-Asp mutants. Other adjuvants (such as a bacterial monophosphoryl lipid A (MPLA) of various sources (e.g., E. coli, Salmonella minnesota, Salmonella typhimurium, or Shigella flexneri, saponins, or polylactide glycolide (PLGA) microspheres) can also be used in mucosal administration.

Adjuvants useful for both mucosal and parenteral immunization include polyphosphazene (for example, WO 95/2415), DC-chol (3 b-(N-(N',N'-dimethyl aminomethane)-carbamoyl) cholesterol (for example, U.S. Patent No. 5,283,185 and WO 96/14831) and QS-21 (for example, WO 88/9336).

An animal may be immunized with an epitope disclosed in the present disclosure by any conventional route as is known to one skilled in the art. This may include, for example, immunization via a mucosal (e.g., ocular, intranasal, oral, gastric, pulmonary, intestinal, rectal, vaginal, or urinary tract) surface, via the parenteral (e.g., subcutaneous, intradermal, intramuscular, intravenous, or intraperitoneal) route or intranodally. Preferred routes depend upon the choice of the immunogen as will be apparent to one skilled in the art. The administration can be achieved in a single dose or repeated at intervals. The appropriate dosage depends on various parameters understood by skilled artisans such as the immunogen itself (i.e. peptide vs. nucleic acid (and more specifically type thereof), the route of administration and the condition of the animal to be vaccinated (weight, age and the like).

The epitope-tagged antibody fragments and/or anti-epitope antibodies of the present disclosure may be administered to an animal in a variety of forms depending on the selected route of administration, as will be understood by those skilled in the art. The epitope-tagged antibody fragments and/or anti-epitope antibodies disclosed in the present disclosure may be used or administered for example, by parenteral, intravenous, subcutaneous, intramuscular, intracranial, intraorbital, ophthalmic, intraventricular, intracapsular, intraspinal, intracisternal, intraperitoneal, intranasal, transepithelial, intrapulmonary, aerosol, topical, transdermal, buccal, nasal, rectal, intrathecal, sublingual, or oral administration, and the pharmaceutical compositions may be formulated accordingly. Parenteral administration may occur by continuous infusion over a selected period of time.

Parenteral liquid administration, *i.e*., I.V. or intramuscular injection, is the preferred means of administration of epitope-tagged antibody fragment and/or anti-epitope antibody. It is anticipated that a cocktail or separate doses, epitope-tagged antibody fragment and/or anti-epitope antibody can be administered by this route. Formulations for topical, oral, pulmonary and transnasal delivery are also envisioned, as these antibody drugs can also be amenable to administration by alternate means, such as when the pharmaceutical industry develops needle-free delivery systems. Those skilled in the art are aware of general methods for administration to animals of drugs of this type- *i.e*., antibodies, or smaller binding proteins.

For parenteral administration, a therapeutically effective amount of the epitope-tagged antibody fragment and/or anti-epitope antibody can be administered as injectable dosages of a solution or suspension of the substance in a physiologically acceptable diluent with a pharmaceutical carrier that can be a sterile liquid such as water oils, saline, glycerol, or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions. Antibodies can be administered in the form of a depot injection or implant preparation which can be formulated in such a manner as to permit a sustained release of the active ingredient.

Oral formulations include a therapeutically effective amount of the epitope-tagged antibody fragment and/or anti-epitope antibody and excipients, such as pharmaceutical grades of mannitol, lactose, starch, magnesium stearate, sodium saccharine, cellulose, and magnesium carbonate. These compositions take the form of solutions, suspensions, tablets, pills, capsules, delayed-, sustained- or extended- release formulations or powders.

A topical formulation typically contains a therapeutically effective amount of the epitope-tagged antibody fragment and/or anti-epitope antibody in a carrier such as a cream base. Various formulations for topical use include drops, tinctures, lotions, creams, solutions and ointments containing the antibodies and various supports and vehicles.

The dosage administered is dependent on the affinity of anti-epitope antibody for epitope-tagged antibody fragment. In one embodiment, the objective of administration is to provide a situation where the initial (T=0) blood concentration of anti-epitope antibody is equal to or greater than the dissociation constant (Kd) of the anti-epitope antibody's affinity for the epitope.

### III. Methods of Making the Antibody Fragments and Antibodies of the Present Disclosure

In one embodiment, the epitope-tagged antibody fragment is capable of being produced in a biologically active form (i.e., functionally) in simple bioreactors, such as either or both of, a prokaryotic expression system (e.g., bacterial cells) or a simply eukaryotic expression system (e.g., yeast cells). However, this may not always be the case. For example, Fab fragments, which are disulphide-linked papain-cleavage fragments derived from whole antibodies, can be made recombinantly in mammalian cell bioreactors or plants, and are intended to be included herein as useful epitope-tagged antibody fragments. These could be so-produced and a suitable peptide epitope could be covalently linked, or alternatively Fab can be also be made in bacteria [7]. Although an epitope-tagged antibody could be made in more complex systems such as mammalian cell cultures and plants, this is not necessary, as it is intended that the industry adapt this technology and thereby dedicate more complex bioreactors, such as mammalian cell production facilities and plants, for large-scale production of anti-epitope antibodies.

For example, tagged scFv antibody fragments useful in the present disclosure, can be made by means of recombinant antibody technology whereby native or naïve antibody libraries, built on phage-display, cell-display or ribosome-display platforms, are panned for antibody fragments that bind to target antigens [8-10]. These libraries can include the epitope tags engineered onto each antibody fragment that is selected from them, or the epitope tags can be added afterwards by means of recombinant DNA technology, using synthetic oligonucleotides and PCR, or by cloning directly into bacterial or yeast expression systems, using techniques known to those skilled in the art.

It is one advantage of the present disclosure that epitope-tagged antibody fragment does not have to be made by a mammalian cell bioreactor, although it can be. Bacterial and yeast expression systems (i.e., bacterial or yeast bioreactors) offer many advantages over mammalian expression systems (*i.e*., mammalian bioreactors) in terms of ease of manipulation, high yields and reduced cost. Many publications regarding the production of antibody fragments in *E coli* exist, as this system is quickly adaptable to producing large amounts of a recombinant protein (see, for example [11, 12]). In some instances, a preferred approach is to use periplasmic expression systems for the production of epitope-tagged antibody fragments. Other bacteria, such as *Lactobacillus zeae,* have also been used [13]. Yeasts and other lower eukaryotes such as *Candida boidinii, Saccharomyces cerevisiae* and *Pichia pastoris* are attractive and cost-effective bioproduction systems for industrial scale processes [12].

Bacterial and yeast cells are grown with agitation in fermenters. Typical sizes for production fermenters are 60,000 to 200,000 liters, although products based on genetic engineering tend to be produced in small amounts and are suited to much smaller bioreactors. *E. coli* is easily accessible for genetic modifications, requires simple inexpensive media for rapid growth and can easily be cultured in fermenters permitting large-scale production of proteins of interest. Several g/L can be obtained in fermentation processes [14]. Antibody fragment production in *E. coli* can be accomplished either by secretion of the fragments in to the culture medium and/or periplasmic space, or by preparation of inclusion bodies with subsequent *in vitro* folding. Recently, improved disulfide bond formation in the cytoplasm, using mutants and over-expression of disulfide-bond isomerase allows *E. coli* to carry out post-translational modifications involving disulfide bonds.

Bacteria and yeast can be grown in liquid media that range from simple to complex, and which include sources of C, N, P, and S, as well as micronutrients. Many types of culture media have been developed; and are known to those of skill in the art (see for example BBL or DIFCO catalogs and manuals for formulations). Complex media comprises constituents that are not completely defined, and are often made from inexpensive organic materials such as brewing and dairy industry wastes and the like. Synthetic media is comprised of completes that are all known and measured. A standard bacterial growth medium, such as Luria Broth, contains tryptone (tryptic digest of casein), yeast extract and salt. In bacterial systems producing an antibody or fragment, the use of a drug in the medium to ensure maintenance of a linked selectable marker is usually required.

Generally, yeast can grow on media such as yeast extract, peptone and dextrose. Antibody production may require a drug for selectable marker maintenance as well. Some yeast hosts producing a transgene-encoded protein are auxotrophic in nature, requiring growth on defined media called "dropout media." Dropout media lacks a known component required for growth of the original auxotrophic host, which is produced by the activity of a gene product encoded on a plasmid that also contains the desired transgene.

Most yeast and bacteria are generally grown at temperatures between 30°C and 37°C, with agitation to provide aeration (see [12] and [14]). Primary expression hosts are *E. coli* and the yeast *Pichia pastoris.* Growth in most bioreactors requires: (1) strain development; (2) media development and optimization; (3) overall process development, and, (4) scale-up. Downstream processing, for the recovery and purification of protein products, can require: (1) production of frozen cell pellets; (2) cell disruption; (3) inclusion body preparations: (4) product harvest and concentration by micro- and ultrafiltration; (5) chromatographic purification (ion-exchange, affinity, size exclusion); (6) protein refolding, and, (7) freeze drying. All of these steps and processes are known to those of skill in the art.

An anti-epitope antibody can usually be of a size and biochemical complexity that can require it to be produced in a complex bioreactor, such as a plant or mammalian cell bioreactor, or a transgenic animal such as a cow or goat. Mammalian cell lines were first used as producers of biopharmaceuticals for an inactivated polio virus vaccine [15]. Mammalian cell culture, and especially Chinese Hamster Ovary and murine myeloma SP2/0 and NS0 cell cultures, for the production of biopharmaceuticals, are now the industrial standards. Currently, large-scale production using mammalian cell culture uses a platform of suspension cell cultures in suspension-tank reactors [16, 17], from which full-length monoclonal antibodies are purified by evolving downstream processing technologies [18].

The above generally describes the present disclosure. A more complete understanding can be obtained by reference to the following specific examples. These examples are described solely for the purpose of illustration and are not intended to limit the scope of the disclosure.

The following non-limiting examples are illustrative of the present disclosure:

### EXAMPLES

### Example 1

### 1.0 Summary

The present inventors determined that an epitope-tagged antibody (rAb):anti-epitope antibody (mAb) complex increased the rAb circulating *in vivo* concentration or persistence of the rAb fragment and recruited Fc-mediated effector functions such as phagocytosis (as supplied by Fc-region on the anti-epitope tag mAb) of the antigenic target specified by the rAb. The rAb-anti-epitope tag IgG protein complex has a higher apparent MW, and increased valency and an association with a functional Fc-region when compared to the monovalent rAb fragments (Figure 2).

The present inventors used two different specific murine anti-tag IgG1 Abs (i.e. anti-c-Myc and anti-Penta-His) and one non-specific anti-epitope tag IgG1 (i.e. anti-QCRL-1) in combination with a c-Myc and 6xhis-tagged murine anti-S. *typhimurium* scFv (B5-1), to examine *in vivo* persistence in CD1 mice and FcR-mediated phagocytosis of S. *typhimurium* by the murine MΦ-like cell line J774. The data demonstrated that bivalent rAb-IgG complexes increased the *in vivo* persistence of rAbs in mice and recruited Fc-mediated effector functions such as C1q binding and phagocytosis by J774 MΦ cells. Bivalent rAb-IgG complexes may also be used to extend the potential of rAb technologies against any antigenic target for complete functional immunotherapies including the easy preparation of polyclonal Ab (pAb) cocktails [19] for increased therapeutic potency. The potential of this therapeutic application is discussed.

### 2.0 Materials and Methods

### 2.1. Materials and Reagents.

*E.coli* serotype 0111:B4 LPS, recombinant mouse INF-γ, and mouse complement sera were obtained from Sigma-Aldrich Canada Ltd. (Oakville, ON, Canada). Human complement C1q and goat anti-human C1q were obtained from Quidel Corporation (San Diego, CA, USA). Swine anti-goat Ab labeled with horseradish peroxidase (HRP), mouse and human absorbed, was obtained from Cedarlane Laboratories Ltd (Hornby, ON, Canada). Goat-anti-mouse-Immunoglobulin (Ig)-HRP (GAM-HRP) and goat-anti-mouse Ig-alkaline phosphatase (GAM-AP), as well as the NBT-BCIP and TMB-ELISA substrates and protein G resin were obtained from Pierce Biotechnology (Rockford, IL, USA). HisTrap™ columns, which were used for immobilized metal affinity chromatography (IMAC), were obtained from GE Healthcare Life Sciences (Uppsala, Sweden).

### 2.2. Bacterial strains and Abs.

*Salmonella enterica* serovar *Typhimurium* (strain pT 104 SA98-3200; S. *typhimurium*) and *Salmonella enterica* serovar *enteriditis* (strain pT3 SA00-4419 09+; S. *enteriditis*) were kindly provided by the Health Canada Salmonella typing laboratory (Research Park, University of Guelph, Guelph, ON). Both strains were grown in brain heart infusion media and grown on Salmonella-Shigella agar plates. *E. coli* HB2151 was used for soluble scFv expression (MRC, Cambridge, UK). Anti-Penta-His™ IgG1 murine mAb (anti-Penta-His) was obtained from QIAgen Inc. (Mississaugua, ON). The anti-QCRL-1 IgG1 murine mAb (anti-QCRL-1) [20] was used as a negative anti-tag IgG1 control and was kindly provided by Toxin Alert (Toronto, ON Canada). The T1#10 scFv is a peptide binding scFv (i.e. to FDTGAFDPDWPAC peptide) that contains both c-terminal c-Myc and 6xHis tags [21]. T1#10 scFv was used in the first *in vivo* experiment and as a non-specific scFv binder to S. *typhimurium* in the phagocytosis assays. The AFA1 V_{H}H binds to non-small cell lung carcinoma cell line A549 and contain c-terminal c-Myc and 6xHis tags [22] and was used in the third in vivo experiment. Both the T1#10 scFv and AFA1 V_{H}H were purified by IMAC as described [21].

### 2.3. Hybridoma cell lines.

The J774.A1 cell line (ATCC No. TIB-67) is an adherent MΦ-like cell line derived from BALB/c mice with reticulum cell sarcoma, and is active in Ab-dependent phagocytosis [23]. The 2.4G2 cell line (ATCC No. HB-197) is a rat hybridoma cell line that expresses an anti-FcγRIIB/III mAb [24]. The 1-9E10.2 (9E10) murine hybridoma cell line (ATCC no. CRL-1729) expresses an anti-c-Myc epitope tag IgG1 mAb (anti-c-Myc) [25]. All cell lines were maintained in Dulbecco Minimum Essential Medium (DMEM) containing 4 mM L-glutamine and 1.5 g sodium bicarbonate/L supplemented with 10% fetal bovine serum (FBS), and were grown at 37°C in 5% CO₂. Monoclonal IgGs were purified from the supernatant of the 2.4G2 and 9E10 cell lines (1-L) by protein G affinity chromatography. The J774.1 macrophage-like cells were resuspended, enumerated and used directly in the phagocytosis assays.

### 2.4. B5-1 scFv.

The scFv clone B5-1 (B5-1) binds to S. *enterica* serotype *Paratyphi* B LPS and has both c-Myc and penta-histidine c-terminal epitope tags [26]. The modified pUC18 plasmid containing this scFv construct was transformed into chemically competent *E. coli* HB2151 for soluble Ab expression. Briefly, a 1 L culture was grown at 37°C in LB broth containing 75 µg/ml carbenicillin and 0.01% glucose to an OD₆₀₀ = 0.9. The culture was induced with 1mM IPTG and grown for 16 h at 30°C. Culture supernatant was filtered (0.45 µm) and loaded onto a HisTrap™ column. B5-1 was purified by IMAC as previously described [21] to approximately 95% purity as analyzed by SDS-PAGE stained with Coomassie brilliant blue.

### 2.5. ELISAs.

All coating antigens, e.g. heat-killed S. *typhimurium* and heat-killed S. *enteriditis* (10⁷ cfu/well), S. *typhimurium* LPS (10 µg/mL), and the three anti-tag IgGs (i.e. anti-c-Myc, anti-Penta-His and anti-QCRL-1; 100 µg/mL), were dissolved in PBS and used to coat plates at 4°C for 16 h. All ELISA plates were blocked with 3% skim milk in PBS (3% MPBS) for 1-2 h at room temperature (RT) and all subsequent steps were done at RT and in 3% MPBS. For the monovalent scFv ELISAs, scFv was added at various concentration for 1 h, the wells were washed (3 x PBS-0.1% Tween (PBST)), and anti-Penta-His mAb was added at approximately 80 ng/mL for 1 h. Wells were washed (3 x PBST) and GAM-HRP (1:2500) was added for 1 h. In the bivalent ELISA, i.e. anti-epitope tag ELISA, B5-1 and the respective anti-epitope tag IgG (i.e. anti-c-Myc or anti-Penta-His) were premixed at various concentrations for 15 min at RT and added to the coated wells for 1 h. Wells were washed (3 x PBST) and GAM-HRP (1:2500) added for 1h as described above. In the C1q binding ELISAs, human C1q (2 µg/mL) was added for 1 h. Wells were washed (3 x PBST) and goat anti-C1q was added (1:2000) for 1 h followed by another wash (3 x PBST) and the addition of swine anti-goat-HRP (1:2000) for 1 h. All wells were washed (3 x PBST) prior to the addition of TMB substrate. All wells were stopped with 1.5 M H₂SO4 and read at 450 nm. A₄₅₀ nm of background binding to non-coated wells was subtracted from the A₄₅₀ nm values of sample wells.

### 2.6. Phagocytosis assays.

Approximately 2 x 10⁵ J774 macrophage cells/well were seeded into each well of a sterile 48-well microtiter plate containing DMEM (1 mL/well) amended with 10% FBS and 100 U/well IFN-γ, and incubated for 16 h at 37°C in 5% CO₂. Media was replaced with DMEM (1 mL/well) containing 100 U/well IFN-γ and 1 µg/mL *E. coli* LPS and incubated for 1.5-2 h. IFN-γ and IFN-γ + *E. coli* LPS were used to 'prime' and 'trigger' the MO cells, respectively, thus up regulating FcR expression [27]. Wells were washed (1 x PBS) and the premixed Ab and bacterial treatments were added to the wells (400 µl/well). Treatments were pre-mixed in bulk in the following manner: 1) B5-1 and the anti-tag IgG1 mAbs were combined in DMEM and rocked for 30 min at RT to allow the formation of B5-1-IgG1 complex; 2) The scFv-IgG1 complex was mixed with bacterial cells (10⁶ cfu/well) and incubated for 30 min at RT to allow opsonization to occur. The Ab and bacterial treatments were added to the MΦ coated wells (400 µl/well) and incubated at 37°C in 5% CO₂ for 25 min to allow phagocytosis. Wells were washed (3 x sterile PBS) and MΦ cells lysed for 30 min at RT with sterile ddH₂0 containing 0.05% Triton-X-100 (500 µl/well). MΦ cell lysis was confirmed by microscopic analysis. For assays in which bacterial viability was examined after phagocytosis (i.e. 30 and 90 min after 25 min of phagocytosis), the wells were washed (3 x PBS) to remove extracellular bacteria, as described above, prior to the addition of 1 mL DMEM/well and incubated at 37°C in 5% CO₂. After incubation (30 and 90 min) the wells were washed (3 x PBS) and lysed as described above. Cell lysates from each well were collected and placed on ice. Dilutions of the cell lysates from each well were prepared in BHI media and 3 x 10 µl spots from the respective well were plated on either Shigella-Salmonella agar or BHI agar plates. Plates were incubated at 37°C for 16 h and bacterial colonies were counted. All treatments were replicated 3 to 6 times and all experiments were repeated 1-3 times. Bacteria used in all assays were grown the same day, seeded from an overnight culture, to mid-log phase, pelleted, and washed (2 x PBS). In all assays the bacterial cell to MΦ cell multiplicity of infection was 10:1.

In the FcR blocking assay, 2.4G2 mAb was added to the 'triggered' MΦ cells for 1 h at 4°C, followed by washing (1x PBS) and the addition of the B5-1-anti-epitope tag IgG complex and bacterial treatments for 25 min as described above. In the assay with murine complement sera, 1.25% complement sera/well was added to the B5-1 and anti-tag IgG complex bulk premix (step 1, as above). The heat inactivated murine complement serum (HI-complement) treatment was heated at 56°C for 45 min prior to its addition in the Ab bulk premix. All assays were conducted with an anti-tag IgG to scFv molar ratio of 1:2.

### 2.7. In vivo scFv clearance assays.

In the first *in vivo* scFv clearance experiment, a comparison of the *in vivo* persistence of the T1#10 scFv and the T1#10-Penta-His IgG complex were examined after 15 min, 1 hr and 4 hrs. In the second *in vivo* experiment the B5-1 scFv was used so that comparisons could be made to the *in vitro* phagocytosis data. Here the B5-1-Penta-His IgG complex, the B5-1-non-specific anti-tag IgG (i.e. anti-QCRL-1), and B5-1 treatments were compared over longer time periods (i.e from 15 min to 6 h). In the third *in vivo* experiment, both the effects of anti-epitope tag IgG affinity on epitope-tagged rAb persistence was assessed, and a different epitope-tagged rAb format (V_{H}H) was used. The anti-epitope tag IgG1 Abs were administered at different concentrations (i.e. anti-c-Myc at 1mg/mouse and anti-Penta-His at 30 µg/mouse), which were based on the anti-epitope-tag IgG1 dissociation constants, while the quantity of administered rAb was held constant (i.e. 100 µg/mouse). All rAbs, i.e. T1#10 scFv, B5-1 scFv and V_{H}H, were labeled with fluorescein isothiocyante (FITC) using the EZ-Label Kit (Pierce Biotechnology, Rockford, IL, USA) as per the manufactured instructions at a FITC to rAb ratio range of approximately 2:1 and 10:1. The FITC-conjugated scFv was analyzed by SDS-PAGE and/or MALDI-TOF following conjugation. Female CD1 mice (25-30 g; Charles River Laboratories, Wilmington, MA, USA) were bleed one day prior to the day of the treatment injections. On the day of treatment, the mice were injected (i.v.) with 50 µg anti-tag IgG (*in vivo* experiments #1 and #2) that was premixed with the FITC-labeled scFv at either a 20:1 (*in vivo* experiment #1) or a 2:1 molar ratio of scFv to anti-epitope tag IgG (*in vivo* experiments #2 and #3). In the first and third *in vivo* experiments, the mice were grouped according to treatment with 5 replicate mice per treatment, and each mouse was bled at 3 times (i.e. 15, 60 and 240 min after injection) by saphenous vein bleeds (approximately 100-200 µl blood collected per time). Therefore, the experimental design consisted of 2 treatments x 5 replicate mice bled 2 or 3 times for a total of 10 mice. In the second *in vivo* assay, mice were grouped by treatment and by time point with 4 replicate mice per each treatment at each time point (i.e. 15 min, 1, 2, or 6 h after injection) and blood collected by terminal bleeds (approximately 2 mL blood collected per mouse). Therefore, the experimental design for the second *in vivo* experiment consisted of 3 treatments x 4 replicate mice x 4 time points for a total of 64 mice.

Serum was collected and analyzed in black 96-well cliniplates (Thermo Fisher Scientific, Vantaa, Finland; cat. no. 9502867) for total fluorescence on the 2100 EnVision™ Multi-Label Plate Reader (Perkin-Elmer, Boston, MA, USA). Briefly, the mouse serum (5 µl) and PBS (200 µl) was added to wells of a black 96-well plate and serially diluted. Serum from each mouse was analyzed and replicate data was pooled for each treatment. The total fluorescence of the pre-immune serum was measured and was subtracted from the treatment data (i.e. background fluorescence). Data is presented as a percentage of the injected dose, or maximum fluorescence. Total fluorescence measurements of the original injected dose were determined by diluting a volume of the original treatment preparation that was equivalent to the injection volume (100-150 µl) into a volume of PBS equivalent to the total blood volume of a mouse (approximately 2 ml), and then 5 µl of this diluted mixture was measured for total fluorescence as described above. Background fluorescence of the pre-immune serum, described above, was also subtracted from fluorescent values of treatments. Furthermore, ELISA was performed to compare scFv persistence and efficacy in the control and treatment groups in experiment #2, where B5-1 *in vivo* persistence and functional binding to S. *typhimurium* LPS (10 µg/mL) was determined. Equal volumes of mouse sera were pooled from each of the treatment groups per time point, and a 1/3 dilution of the pooled serum was prepared in 3% MPBS. The diluted serum was added to coated ELISA wells, serially diluted to 1/24, and incubated for 2 h at RT. B5-1, B5-1-anti-Penta-His IgG and B5-1-anti-QCRL-1 IgG binding were detected for 6xHis tagged B5-1 binding using anti-Penta-His and GAM-HRP as previously described. All experimental protocols in which animals were used was reviewed and approved by the University of Guelph Animal Care Committee (Guelph, ON, Canada).

### 2.8. Statistical analysis.

Three to six replicates were performed for each experiment and each experiment was repeated either one or three times. Summary results are represented as means ± SEM. A one-way analysis of variance was conducted and when data were found to be significantly different means were separated using either a Tukey's Honestly Significant Difference (HSD) test, for analysis of difference between categories, or Dunnett's multiple comparisons (two-sided) for analysis of the difference between treatments and the control. P values < 0.05 were considered significant. For all analysis Prism 4.0 (GraphPad Software, San Diego, CA) and XLSTAT software (Addinsoft, New York, NY, USA) were used.

### 3.0 Results

### 3.1. Antigen binding of the B5-1 scFv alone or in complex with an anti-epitope tag IgG

As determined by ELISA, the B5-1 scFv (monovalent format) bound to both heat-killed S. *typhimurium* and S. *typhimurium* LPS in a dose dependent manner, whereas it did not bind to heat-killed S. *enteriditis* (Figure 3a). When present in the bivalent format, i.e. premixing B5-1 with either the anti-c-Myc or anti-Penta-His mAbs, the apparent binding affinity of the B5-1 to S. *typhimurium* LPS was higher than when presented in the monovalent format (Figure 3b). Regardless of the anti-epitope tag mAb used, reducing the anti-epitope tag mAb concentration, while maintaining the B5-1 concentration constant, resulted in a dose-dependent reduction of detectable B5-1 binding to the S. *typhimurium* LPS. Based on the rate of change in absorbance the B5-1 anti-Penta-His complex bound better to S. *typhimurium* than the B5-1 anti-c-Myc complex (Figure 3b). These differences reflect the equilibrium dissociation constants (K_{d}) of the anti-c-Myc and anti-Penta-His anti-tag Abs for their epitope tag targets, i.e. anti-c-Myc has low K_{d} (refer to section 3.5).

### 3.2. Ability of the anti-tag IgG1 Abs to bind C1q

Each of the three anti-epitope tag IgG1 Abs resulted in a significant deposition of human C1q compared to the no Ab control (Figure 3c), indicating that all three anti-epitope tag IgG1 Abs have functional Fc regions for C1q recruitment. Binding of C1q to the B5-1-anti-epitope tag IgG1 complex which was also bound to immobilized target antigen, i.e. S. *typhimurium* LPS, was measured. When compared to the negative control, i.e. anti-QCRL-1, there was significantly more deposition of C1q on the ELISA plate when the specific anti-epitope tag IgG1, i.e. anti-c-Myc and anti-Penta-His, were bound to B5-1. Since the QCRL-1 epitope tag is not present on the B5-1 scFv, binding of the anti-QCRL-1 mAb to the B5-1 did not occur and thus little C1q deposition was detected (Figure 3d). In both experiments, the anti-Penta-His mAb bound less C1q than the anti-c-Myc mAb. It has been shown that IgG1 variants exist that have different capacities to bind C1q [28]; this may explain the difference in C1q binding to the anti-c-Myc and the anti-Penta-His mAbs.

### 3.3 J774 phagocytosis of S. typhimurium mediated by the scFv-anti-epitope tag IgG1 complexes

Phagocytosis of S. *typhimurium* by J774 cells was significantly greater in the presence of the B5-1-anti-epitope tag IgG1 complexes than B5-1 alone (Fig. 4a and 4b). Furthermore, the extent of phagocytosis mediated by both the B5-1-anti-c-Myc and B5-1-anti-Penta-His complexes were similar. The extent of phagocytosis was B5-1-anti-Penta-His complex = B5-1-anti-c-Myc complex > B5-1 = B5-1-anti-QCRL-1 (i.e. no complex) (Fig. 4a and 4b). These data indicate that specific binding of the B5-1-anti-epitope tag IgG1 complex to the target organism allows for increase in phagocytosis via FcR-mediated phagocytosis. It is not clear why B5-1 alone was the second best treatment. Perhaps the B5-1 scFvs formed dimeric/multimeric scFv-bacterial complexes resulting in non-FcR-mediated opsonization, e.g. scavenger receptors [29]. Controls including a non-specific scFv (i.e. T1#10, that binds to an unrelated peptide) in a T1#10-anti-c-Myc complex, and a B5-1-heat-inactivated anti-tag IgG1 (i.e anti-c-Myc) complex were examined for phagocytosis of S. *typhimurium.* Furthermore, B5-1-anti-c-Myc and B5-1-anti-Penta-His complexes were examined for their ability to phagocytose the non-specific bacterium S. *enteriditis.* These controls resulted in significantly less phagocytosis indicating that both an antigen specific rAb fragment and an intact, i.e. non denatured, anti-epitope tag IgG are required to achieve the best bacterial phagocytosis via the scFv-anti-epitope tag complex (Figure 4c and 4d).

### 3.4 Blocking FcR-mediated phagocytosis with the 2.4G2 anti-FcR mAb

Further support for the importance of the FcR-mediated interactions in bacterial phagocytosis with the B5-1-anti-c-Myc or B5-1anti-Pent-His complexes, were provided using an anti-FcR mAb blocking assay. Incubation of the J774 MΦ cells with the 2.4G2 anti-FcR mAb, in concentrations that exceeded that of the respective anti-epitope tag mAb concentration (i.e. used in the B5-1-anti-epitope tag complex) inhibited bacterial phagocytosis in a dose-dependent manner (50x data is presented; Figure 5a), indicating that 2.4G2 mAb blocked binding of both complexes to the Fc region, thus restricting recruitment of phagocytosis.

### 3.5. Effects of anti-epitope tag IgG1 affinity on bacterial phagocytosis

The equilibrium dissociation constant (K_{d}) that the anti-epitope tag Ab has for its epitope tag, and thus the epitope tagged scFv, has a significant effect on J774-mediated phagocytosis of S. *typhimurium.* Therefore, the B5-1-anti-Penta-His (K_{d} for anti-Penta-His is approximately 10 nM) [30] complex resulted in significantly more J774-mediated phagocytosis than did the B5-1-anti-c-Myc complex treatments (K_{d} for anti-c-Myc is approximately 560 nM; Figure 5b) [31]. Furthermore, significantly more binding to S. *typhimurium* LPS occurred with the B5-1-anti-Penta-His complex as determined by ELISA (Figure 3b). Thus, the higher K_{d} value of anti-c-Myc (and thus lower binding affinity for its target epitope) compared to anti-Penta-His, means that higher Ab concentrations are required to form the B5-1-anti-c-Myc complex compared to the concentrations required to form the B5-1-anti-Penta-His complex. Therefore, the ability to form a scFv-anti-tag IgG complex at lower anti-tag Ab concentrations has a significant effect on the number of antigenic targets that are bound, and thus phagocytosed by the murine J774 MΦ cells.

### 3.6. Phagocytosis in the presence of complement

J774-mediated phagocytosis was evaluated with the B5-1-anti-epitope tag mAb treatments in the absence and presence of 1.25% whole murine complement and 1.25% HI-complement. Bacterial phagocytosis was significantly greater in the presence of murine complement and the B5-1-anti-c-Myc treatment, compared to the no complement and HI-complement controls. Phagocytosis also increased with the B5-1-anti-Penta-His complex plus murine complement, compared to B5-1-anti-Penta-His complex without complement; however, this difference was significant P value = 0.06 (Figure 5c).

### 3.7. In vivo rAb clearance

The first *in vivo* rAb clearance experiment compared differences in persistence times of T1#10 (tagged with both c-Myc and 6xHis) and T1#10-Penta-His complex treatments. Regardless of the sampling time, the T1#10-anti-Penta-His complex resulted in significantly greater persistence of T1#10
when compared to the T1#10 treatment (i.e. control) (Figure 6a)

The second *in vivo* rAb clearance experiment compared three treatments, the B5-1-anti-Penta-His complex, B5-1-non-specific anti-tag IgG1 (i.e. anti-QCRL-1 mAb; no complex forms), and B5-1 at 15 min, 1, 2, and 6 h. The B5-1-anti-Penta-His treatment had significantly higher B5-1 persistence compared to the B5-1-anti-QCRL-1 and B5-1 control groups at the 15 min, 1, 2 and 6 h sampling times as measured by ELISA (Figure 6b). These results also indicated that a lower ratio of anti-tag mAb:rAb can be used, as the Penta-His mAb:B5-1 scFv ratio was 2:1, and that the rAb maintains its specific antigen-binding through *in vivo* passage as pooled sera were applied to specific S. Typhimurium LPS coated onto microtitre plate wells for ELISA.

In the third *in vivo* persistence assay, different quantities of the anti-c-Myc (1 mg) and anti-Penta-His (0.03 mg) Abs were examined for their ability to increase the circulation of a constant quantity of a c-Myc and 6xhis-tagged V_{H}H. At 15 min, both the V_{H}H-anti-Penta-His and V_{H}H-anti-cMyc complex were equally effective at maintaining the V_{H}H concentrations at significantly greater concentrations than the two controls (V_{H}H alone and V_{H}H+ anti-QCRL-1). Futhermore, approximately 33 times less anti-Penta-His than the anti-c-Myc was required. At 1 h, only the V_{H}H-anti-Penta-His complex had greater circulating V_{H}H concentration when compared to the other three treatments (Figure 6c). This data corroborates the affinity ELISA (Figure 3b) and phagocytosis (Figure 5b) data which indicate that an anti-epitope tag mAb with a higher affinity for its epitope tag (thus lower Kd, i.e. anti-Penta-His) results in formation of a more stable rAb-IgG complex thus mediating greater antigen binding, phagocytosis and longer *in vivo* persistence at lower anti-epitope tag concentrations.

Comparisons among the three *in vivo* clearance experiments show many similarities. For example, the two scFv to anti-epitope tag mAb ratios tested, i.e. 20:1 (*in vivo* experiment #1 with T1#10) and 2:1 (*in vivo* experiment #2 with B5-1), gave similar results (i.e. approximately 40%, 20% and 5% max fluorescence for the scFv-anti-Penta-His complex at 15 min and 1 h, and 4-6 h, respectively; Figures 6a and 6b) and suggest that a molar excess of scFv to anti-tag IgG beyond 2:1 has no additional effect on reducing *in vivo* rAb fragment clearance. Additionally, two different scFv fragments, i.e. T1#10 and B5-1, and a V_{H}H fragment were tested and each Ab fragment had similar and reduced clearance patterns when complexed with anti-Penta-His or anti-cMyc, thus illustrating the consistency and applicability of this technique. Furthermore, conjugation of the rAb fragments with FITC did not interfere with the formation of the scFv-anti-epitope tag IgG complex by as determined by ELISA (data not shown).

### 4.0 Discussion

The present inventors demonstrated that: 1) terminal epitope tags expressed on a rAb fragment can specifically recruit functional Fc regions, supplied by full-length anti-epitope tag IgGs, to antigens targeted by the epitope-tagged rAb fragments and, 2) an epitope-tagged rAb in complex with an anti-epitope tag IgG increases *in vivo* persistence of the rAb fragment. Proof that bivalent rAb-IgG recruited Fc-mediated effector functions was demonstrated *in vitro* by the binding of human complement C1q by ELISA and by greater phagocytosis of S. *typhimurium* by J774 MΦ cells following treatment with the B5-1-anti-tag IgG complexes. Proof of increased *in vivo* persistence of rAb when presented as a bivalent rAb-anti-epitope tag complex was demonstrated by increased persistence and greater quantities of epitope-tagged scFvs (i.e. B5-1 and T1#10) and V_{H}H at various times following i.v. administration to CD1 mice. Thus, use of a bivalent rAb-anti-epitope tag complex is a valid method by which to improve the therapeutic efficacy and utility of rAb fragments.

FcR-mediated effector functions, such as the ability to recruit complement C1q and increase MΦ phagocytosis, were demonstrated. Binding of murine complement C1q to the murine anti-epitope tag mAbs was not shown due to the limited availability of this reagent; however, this binding has been well established as reviewed by Kinshore and Reid (2000) [32]. Additionally, mouse and human C1q a, b and c proteins, that make up the C1q molecule, have approximately 70-80% homology and thus some degree of cross-reactive binding should be expected as was demonstrated here with the human C1q binding the to murine anti-epitope tag IgG1 Abs. Greater bacterial phagocytosis was seen with the B5-1-anti-Penta-His and B5-1-anti-c-Myc complexes in the presence of whole murine complement and is likely a contribution of both the classical and alternative complement pathways, i.e. the interaction of C1q with that anti-epitope tag IgG1, and C3-mediated bacterial opsonization and subsequent binding to the C3 receptor on the J774 MO cells, respectively. However, the relative contributions of these two factors are unknown. Regardless, the presence of murine complement acts to significantly increase J774 bacterial phagocytosis beyond that obtained with the B5-1-anti-epitope-tag IgG treatment without complement. Blocking the FcR with anti-FcR mAb (2.4G2) significantly reduced phagocytosis by the MΦ cells which would be otherwise mediated by either the B5-1-anti-Penta-His or B5-1-anti c-Myc complex, and thus provides further evidence for the importance of Fc-mediated function associated with the bivalent rAb-IgG complex treatments (Fig 5a). Other FcR-mediated effector functions, such as T-cell activation and cytokine release were not measured in this study but will be quantified in future experiment to determine the complete mechanisms of action of the bivalent rAb-IgG1 complexes.

ADCC and CDC were not demonstrated in the J774 phagocytosis assays and this is likely a result of the model microorganism used. *S. typhimurium* is a facultative intracellular pathogen that, by several complex processes, can survive, proliferate and survive in macrophage cells [33, 34]. To determine whether the B5-1-anti-c-Myc and B5-1-anti-Penta-His complexes could initiate ADCC with the J774 cells, the present inventors investigated intracellular *S. typhimurium* viability at 90 and 240 min following phagocytosis and found that intracellular bacterial viability did not change after phagocytosis following treatment with either complex, i.e. *S. typhimurium* cell death did not occur (data not shown). Additionally, phagocytosis of S. *typhimurium* in the presence of whole complement serum and the B5-1-anti-c-Myc or B5-1-anti-Penta-His complex, i.e. to determine the effects of CDC, increased bacterial phagocytosis approximately 2-fold; however, intracellular *S. typhimurium* proliferation and MΦ cell lysis approximately 30 min following phagocytosis was observed (data not shown). Thus, to fully appreciate the full range of potential FcR-mediated effector functions that can be initiated following treatment with bivalent rAb-IgG complex other antigenic targets such as non-intracellular pathogens or tumor cells need to be tested. Furthermore, it should be mentioned that the treatment of intracellular pathogens would likely not be suitable therapeutic target for bivalent rAb-IgG complexes as they could, in some cases, exacerbate disease symptoms via the complement-mediated lysis of cells containing viable pathogens.

The present inventors have demonstrated that the *in vivo* presence of a specific full-length anti-epitope tag Ab can increase the serum persistence of epitope-tagged rAb fragments. The increased serum persistence shown with the bivalent rAb-anti-eptiope tag IgG complexes is likely a result of increased apparent MW. Furthermore, valency will contribute to increase the serum persistence or half-life since K_{off} between the antigen and bivalent scFv-anti-epitope tag complex will be reduced thus reducing clearance based on low MW. Valency has been shown to have a dominant effect over MW in accounting for superior retention times of small rapidly cleared rAb molecules [35]. To determine whether valency will further increase the serum persistence or half-life the target antigen with multiple common epitopes (e.g. a tumor cell antigen) must be available for binding *in vivo.* Clearly, valency and MW have been shown to increase persistence or half-life since other technologies have been efficient at improving *in vivo* half-life or persistence of rAb fragments by increasing MW, such as by conjugation with polyethylene glycol (PEGylation) [36], and (in addition to increasing MW) by increasing avidity by mutimerization [36-38]. However, only a few of the multivalent design formats, e.g. scFv₂-Fc and scFv-C_{H}3, have the ability to recruit Fc-mediated effector functions [37, 38]. The advantage of using bivalent rAb-IgG complexes is that they can easily be non-covalently associated with any epitope-tagged monovalent rAb fragments to create a bivalent complex with a functional Fc region.

Results indicated that the strength of binding (i.e. affinity coupled with avidity) between the epitope tag and anti-epitope tag Ab is important in mediating longer *in vivo* persistence and Fc effector functions. For example, at a concentration 33 x lower than anti-c-Myc, anti-Penta-His (K_{d} approximately 10 nM) was as efficient as anti-c-Myc (K_{d} approximately 560 nM) at increasing the levels of circulating V_{HH} (Figure 6c). Additionally, the B5-1-anti-Penta-His complex resulted in both significantly greater antigen binding and phagocytosis, especially at lower anti-tag IgG concentrations, than did the B5-1-anti-c-Myc complex (Figures 3b and 5b). These data could be explained by the > 50-fold difference in affinity between the two anti-epitope tag Abs, suggesting that a high affinity interaction may be preferred between the anti-epitope tag IgG and epitope-tagged rAb fragment for potential therapeutic use.

Immunoglobulin isotypes have different affinities for different FcRs and thus Ab isotype is another factor that needs consideration in determining both the murine and human IgG that should be used *in vivo.* Murine isotypes IgG2a/c and IgG2b are the most potent in stimulating FcR-mediated effector functions (reviewed in [39]) and these may result in more significant FcR-mediated effector functions than was observed with the IgG1 isotype used in the present studies. For targeted human therapy, the superior effector function of IgG1 and IgG3 would make them primary isotype candidates as anti-epitope tag IgGs [6].

In addition to the importance of anti-epitope tag IgG isotype and affinity, the choice of the epitope tag and thus anti-epitope tag mAb used also requires consideration. Several different epitope tags are used in biological research today (reviewed in [4]). However, both the c-Myc and 6xHis epitopes have the potential disadvantages of targeting endogenous host proteins. Thus, an epitope tag sequence that is completely foreign to that of host proteins and one that could offer potential beneficial side effects may be desirable. For example, the HA tag (YPYDVPDYA (SEQ ID NO: 1)) from the human influenza virus hemaglutinin protein [40] may be one possibility such that non-specific cross-reactions may occur during an *in vivo* influenza infection and, in theory, would be less likely to result in negative side effects compared to the non-specific targeting of host tissues associated with c-Myc and 6xHis. Several other virus-derived epitopes have been used to label proteins including the V5 epitope from the simian virus 5 [41], polyoma virus T antigen epitopes [42], and the KT3 viral epitope [43]. Furthermore, rAb purification requires that the epitope tag used for affinity chromatography will ensure high quality and large quantities for therapeutic application. Kappa light chain scFv and Fab can be purified by protein L affinity chromatography, and thus may not require additional epitope tags beyond that targeted by the anti-epitope tag mAb. Conversely, V_{H}H purification relies solely on epitope tags. Thus, the type of rAb fragment(s) that is best suited for the therapeutic application of this technology may be determined by the tags required for purification because fewer tags may reduce possible *in vivo* cross-reactivity. In summary, selection of an epitope tag may be accomplished by determining tags that have efficient purification qualities, high affinity for the anti-epitope tag IgG, and are void of non-specific *in vivo* cross-reactivity.

The non-covalent formation of bivalent rAb-IgG complexes provides an easy format from which oligoclonal or polyclonal Ab (pAb) therapeutics can be created. Most research to date has focused on human(ized) mAb therapy directed at single antigenic epitopes. However, increasing therapeutic Ab potency should include the development and administration of pAb repertoires that mimic the natural pAb response and target multiple antigens [44]. The *in vivo* benefits of pAb therapies over mAb therapies have been demonstrated. For example, a pAb mixture consisting of three mAbs had a protective index of 20,000-fold greater than the LD₅₀ of BoNT/A, whereas individually, each mAb had a protective index of only approximately 20 times that of the LD₅₀ doses [45]. Other combinations of three or more mAbs have been shown to increase the potency of tetanus toxin and HIV virus neutralisation by 200 and 10-fold, respectively [46, 47]. The development of pAb therapeutics has been examined via recombinant methods [48-52], and via the development of transgenic animals with human Ig loci [53]. The Xenomouse transgenic system [54] has generated human Abs; however, these are focused towards single epitopes (i.e. mAbs) [55]. Homozygous heavy chain knockout transgenic cows has been described [56] and together with light-chain and prion protein knockouts [57], could form new platform for human pAbs production in the future. However, pAb production via the non-covalent formation of bivalent rAb-IgG complexes, would require only one (or few) human(ized) anti-epitope tag IgG molecules, while multi-antigen specificities are supplied by different epitope-tagged rAb fragments. A facility producing the anti-epitope tag IgG(s), in combination with the rapid and inexpensive production of polyclonal rAb repertoires, could provide speed and flexibility in pAb development. Therefore, only one anti-epitope tag mAb may be required to deliver several therapeutic rAb fragments. Thus a few anti-epitope tag IgG molecules could be tailored for specific and multiple types of therapy therapeutic outcomes (e.g. for enhanced immune engagement with FcγRs and complement (reviewed in [6, 39]). The use of bivalent rAb-IgG complexes in pAb therapeutic development is promising.

Therapeutic use of bivalent rAb-IgG complexes improves the therapeutic utility of monovalent rAbs. The present inventors have successfully shown that bivalent rAb-IgG complexes increased the *in vivo* persistence of rAb fragments and recruited Fc-mediated effector functions to target the antigens specified by the rAb.

### Example 2

### 1.0 Summary

Recombinant antibody fragments (rAb) are being increasingly exploited as diagnostic reagents and therapeutic drugs. However, their therapeutic applications are often compromised by their short serum half-lives and inability to mediate Fc-dependent effector functions. Here, the efficacy to improve the therapeutic potency of rAbs via the formation of a bivalent rAb-mAb complex through non-covalent binding of an epitope-tagged rAb with an anti-epitope tag mAb was demonstrated. The epitope-tagged rAb provided target specificity, while the anti-epitope tag mAb prolonged rAb serum persistence or half-life and also triggered immune effector functions via its Fc region. This was shown using c-myc- and 6xHis-tagged Fab and scFv both directed against *Pseudomonas aeruginosa* O6ad in combination with two different murine anti-epitope tag IgGs, anti-5xHis IgG (Penta-His) and anti-c-myc IgG (9E10), at a molar ratio of 2 to 1 (rAb to mAb). The data showed that complexes with the anti-tag IgGs significantly improved the antigen binding avidity of both the Fab and scFv by up to 260-fold, extended the serum persistence of the Fab in mice approximately by 2-fold, and effectively mediated complement deposition as well as opsonic phagocytosis of the target bacteria by murine J774.1A macrophages *in vitro.* These results demonstrated that the combination of an epitope-tagged rAb with an anti-epitope tag IgG is a simple and effective strategy for enhancing the therapeutic potency of rAbs by simultaneously improving their antigen binding ability and pharmacokinetic properties as well as conferring on the rAbs the ability to recruit Fc-dependent effector functions.

### 1.1 Introduction

Since the successful expression of functional antigen-binding antibody fragments in *Escherichia coli* [58], a number of rAbs with a variety of formats and unique properties have been generated. These include classic antibody fragments, i.e., F(ab)'₂, Fab, scFv, Fv, and V_{HH}, and their derivatives, i.e., multivalent and multispecific antibody fragments, and rAb-based fusion proteins [1, 59]. These rAbs retain the target specificity of their parent mAbs, and can be produced rapidly and economically in large quantities using prokaryotic or lower eukaryotic expression systems and engineered with desired properties for applications in diagnosis and therapeutics.

For some applications, such as viral and toxin neutralization, receptor blockade, cytokine inactivation, drug delivery, and tumor imaging, Fc-mediated functions are undesirable and thus, rAbs with antigen binding specificity are adequate and even preferred. Interest in use of rAbs in these situations is partially due to the lack of the Fc region, as its presence can lead to undesirable side-effects through activation of immune effectors, and more importantly due to their small size (15-90 kDa *vs.* 150 kDa for mAbs). These two features offer the advantages of lower immunogenicity when derived from heterologous antibodies, efficient target-specific accumulation that avoids non-specific exposure to normal tissues, and rapid clearance from the circulation that is beneficial for fast clearance of toxins from the body [60, 61] and for tumor imaging with reduced background signals [1, 62, 63]. In several studies on tumor imaging, rAbs have been shown to perform as well and, in some cases, even better than mAbs [64, 65]. In addition, small rAbs are best-suited for intracellular applications [66].

For some applications, it is desirable for rAbs to have a prolonged serum half-life and the ability to mediate Fc-dependent effector functions, i.e., antibody-dependent opsonization of target organisms and immune cell- and complement-dependent cytotoxicities. In this respect, the therapeutic applications of rAbs are compromised by following limitations: 1) low antigen binding avidity due to the monovalent binding, 2) a short circulating half-life (hours vs. 2-3 weeks for mAbs) due to rapid renal clearance because of their small size (15-90 kDa vs. glomerular cutoff < 70 kDa) and the lack of recycling mechanism mediated by a neonatal Fc receptor (FcRn), and 3) inability to mediate effector functions due to the lack of a Fc region.

In this example, rAbs were complexed with an intact mAb to improve their therapeutic potential by increasing their serum persistence or half-lives and by conferring on them the ability to recruit Fc-mediated effector functions. This was achieved by addition of a short antigenic epitope tag (i.e., c-myc and 6xHis) to C-terminus of a rAb, thus facilitating the non-covalent coupling of the epitope-tagged rAb with an intact anti-epitope tag mAb, resulting in the formation of a bivalent rAb-mAb complex. The epitope-tagged rAb conferred antigen-binding specificity and affinity, while the anti-tag mAb prolonged rAb serum persistence or half-life by increasing its size as well as by providing the FcRn-dependent recycling mechanism, and also by providing a functional Fc region to trigger immune effector functions against targets, thereby improving the therapeutic potency of the rAbs. By using this technique, enhancement of antigen binding activity has already been described for scFvs [5] (Example 1). However, the potential applications of this technique in improvement of therapeutic potency of Fab and its *in vivo* applications against infections have not yet been addressed.

To test the efficacy of this strategy for improving the therapeutic potential of rAbs, both c-myc- and 6xHis-tagged Fab and scFv fragments were produced, both of which were specific for P. *aeruginosa* O6ad lipopolysaccharide (LPS); then, the Fab and scFv were tested in combination with murine anti-tag mAbs, i.e., Penta-His or 9E10, for their *in vitro* antigen binding ability, *in vivo* serum persistence, and ability to mediate effector functions including complement fixation, complement-dependent cytotoxicity (CDC), as well as bacterial opsonization for phagocytosis by murine J774.1A macrophages. The results showed that regardless of the rAb formats used, complexes with either Penta-His or 9E10 significantly enhanced their target-binding avidity, extended their *in vivo* serum persistence in mice, and effectively recruited Fc-dependent effector functions including complement deposition and opsonization of the target bacteria by macrophages *in vitro.* These results demonstrated that complex formation with an anti-tag mAb is an efficient strategy for improvement of the therapeutic potency of epitope-tagged rAbs.

### 2.0 Materials and Methods

### 2.1 Materials

All chemicals were purchased from Sigma-Aldrich Canada Ltd. (Oakville, ON, Canada) unless otherwise stated. LPSs of *P. aeruginosa* serotype O10 and *Escherichia coli* serotype 0111:B4 were obtained from Sigma-Aldrich, while those from *P. aeruginosa* serotypes O6ad and PAO1 were isolated using the Tri-Reagent method as previously described [67]. A murine mAb QCRL-1 (IgG₁) was a kind gift from Dr. Susan Cole (Queen's University, Kingston, ON, Canada) and a murine mAb Penta-His (anti-5xHis IgG₁) [30] was purchased from QIAgen Inc. (Mississauga, ON, Canada).

### 2.2 Bacterial strains

*P. aeruginosa* serotype O6ad was kindly provided by Dr. John R. Schreiber (Tufts University, Boston, MA, USA), while P. *aeruginosa* serotypes PAO1 (ATCC BAA-47™) and O10 (ATCC 33357) were purchased from American Type Culture Collection (ATCC) (Manassas, Virginia, USA). All *P. aeruginosa* bacteria were cultured in tryptic soy broth (TSB) (Fisher Scientific, Mississauga, ON, Canada) at 37°C with shaking (230 rpm), harvested during log-phase growth by centrifugation (5000 rpm, 5 min, RT), and resuspended in Dulbecco's Modified Eagle's Medium (DMEM, containing 4 mM L-glutamine and 1.5 g sodium bicarbonate/L) (HyClone, Logan, Utah, USA) at the required concentrations. Bacteria for the opsonophagocytic experiments were prepared as described above, while those for Enzyme-Linked Immunosorbent Assay (ELISA) were prepared in PBS (pH 7.4), heat-killed at 60°C for 1 h, and stored at -20°C until later use.

### 2.3 Cell lines

Mammalian cell lines, including the murine macrophage J774.1A (ATCC TIB-67), rat/mouse hybridoma 2.4G2 (ATCC HB-197), and murine hybridoma 1-9E10.2 (9E10) (ATCC CRL-1729), were obtained from the ATCC. The 2.4G2 cells express a rat anti-mouse mAb 2.4G2 (IgG_{2b}) specific for FcγRIIB/III and possibly FcγRI [24, 68], while the 9E10 cells express a murine mAb 9E10 (anti-c-myc IgG₁) specific for a peptide immunogen derived from the human c-myc proto-oncoprotein [25]. All cell lines were stored in liquid nitrogen in DMEM supplemented with 10% dimethylsulfoxide (DMSO). All cells were cultured in DMEM supplemented with 10% fetal bovine serum (FBS) at 37°C in a humidity chamber with 5% CO₂. All experiments were performed in a humidity chamber with 5% CO₂ unless stated otherwise.

### 2.4 Antibody cloning, production, and purification

The human anti-*P*. *aeruginosa* O6ad LPS te-hS20 (IgG₁) was purified from transgenic tobacco according to McLean et al. [69]. The 2.4G2 and 9E10 mAbs were purified from mammalian cell culture supernatants using protein G affinity chromatography with an Akta FPLC system (EY Laboratory Inc. CA, USA) as previously described in Example 1.

To produce the Fab of the human anti-P. *aeruginosa* O6ad LPS te-hS20 [69], the kappa light (L) chain and Fd fragment of the gamma H chain were amplified by polymerase chain reaction (PCR) from pMM7 and pMM3, respectively, using forward primers (5'-GTATCTCTCGAGAAAAGAGAGGCTGAAGCTGACGTGGTTATGACACAAA CT-3' (SEQ ID NO: 2) and 5'-TATCTCTCGAGAAAAGAGAGGCTGAAGCTCAAGTTCAACTTGTTGAAAGT G-3' (SEQ ID NO: 3), respectively) and reverse primers (5'-TCCTGTTCTAGATTATCAACACTCTCCTCTATTGAAACTCTT -3' (SEQ ID NO: 4) and 5'-TCCTGTTCTAGATGTGTTTTGTCGCATGACTT-3' (SEQ ID NO: 5), respectively). These PCR products were digested with *Xho*I and X*ba*I; the L product was subcloned into pPICZαA (Invitrogen, Carlsbad, CA); the H product, into pPICZαA-Δ*Pme*I, generated by site-directed mutagenesis to destroy the *Pme*I site using the QuickChange Site-Directed Mutagenesis Kit (Stratagene), resulting in pPICZαA-L and pPICZαA-Δ*Pme*I-Fd, respectively. The L chain expression cassette was PCR-amplified from pPICZαA-L with forward primer 5'-CATGAGATCGGATCCAACAT-3' (SEQ ID NO: 6) and reverse primer 5'-AAAAAGAAACGAGGCGGTCT-3' (SEQ ID NO: 7), digested with BamHI and subsequently cloned into *Bam*HI-digested pPICZαA-Δ*Pme*I-Fd to generate the pPICZαA-Fab expression plasmid.

The anti-*P*. *aeruginosa* O6ad scFv coding sequence was synthesized at the PBI/NRC DNA/Peptide Synthesis Laboratory, National Research Council of Canada, (Saskatoon, SK). The DNA coding sequences of the V_{H} and the V_{L} of the IgG₁ were joined by a (Gly₄Ser)₃ linker. This coding sequence was amplified by PCR using a forward primer (5'-GTATCTCTCGAGAAAAGAGAGGCTGAAGCTGACGTGGTTATGACACAAA CT-3' (SEQ ID NO: 8)) and a reverse primer (5'-TCCTGTTCTAGAGAAACAGTAA CCAATGTTCC-3' (SEQ ID NO: 9)), digested with *Xho*I and *Xba*I and then subcloned into *Xho*I and *Xba*I double-digested pPICZαA, resulting in the pPICZαA-scFv expression plasmid.

The pPICZαA-Fab and pPICZαA-scFv plasmids were linearized with *Pme*I prior to introduction into *Pichia pastoris* X-33 (Invitrogen) by chemical transformation and screening with Zeocin (100 µg/ml, Invitrogen). Note that the gamma sequences of both the Fab and the scFv have 6 X His and c-myc motifs at their carboxyl termini.

Both the anti-*P*. *aeruginosa* O6ad Fab and scFv were expressed [70] and purified by immobilized metal affinity chromatography (IMAC) [21]. Since the anti-O6ad Fab fraction contained approximately 40% of glycosylated yeast α-factor signal sequence-linked Fab, it was further treated with concanavalin (Con) A-agarose to remove α-factor-linked Fab [71].

The purity of all antibodies and fragments was analyzed by sodium dodecyl sulfate (SDS)-polyacrylamide gel electrophoresis (PAGE) and Western immunoblotting [69], using Penta-His IgG (Qiagen, Mississauga, ON) and goat anti-mouse IgG conjugated with alkaline phosphatase (AP) (Pierce, Rockford, IL), both at 1:2000 dilutions in PBST as primary and secondary antibodies, respectively.

### 2.5 Antigenic binding assays

The binding abilities of anti-tag IgGs, including Penta-His and 9E10, to the epitope-tagged anti-O6ad scFv and Fab was tested by ELISA. The QCRL-1, an irrelevant anti-tag mAb, was used as a negative control. In brief, microtiter polystyrene plates (Costar, Corning, NY) were coated with either the anti-O6ad scFv or Fab (120 nM, 100 µl/well) and treated with serial dilutions of anti-tag IgG with an initial concentration of 60 nM, which were detected with a goat anti-mouse IgG conjugated with horseradish peroxidase (HRP) (Pierce) at 1:2000 dilutions. Plates were developed with 1-Step™ Turbo 3, 3', 5, 5' tetramethylbenzidine (TMB) substrate (Pierce), followed by termination with 1.5 M H₂SO₄ and optical density (OD) measurement at 450 nm. Wells coated with PBS were used as controls for background readings. All samples were run in triplicate and ODs were corrected by subtracting background readings.

The binding abilities of the anti-O6ad scFv and Fab to heat-killed *P. aeruginosa* O6ad bacteria (1 x 10⁸ CFU/ml) or its corresponding LPS (LPS_{O6ad}) (1 µg/ml) with or without forming immunocomplexes with the anti-tag IgG was determined by ELISAs, as described previously (Example 1). The ELISAs were also performed using other heat-killed *P. aeruginosa* strains PAO1 and O10 and their corresponding LPSs to determine the binding specificity.

### 2.6 C1q deposition assays

The capacity of the anti-O6ad scFv/Fab-anti-tag IgG complexes to recruit the first complement protein C1q was tested by ELISAs in the same manner as described (Example 1), except heat-killed *P. aeruginosa* O6ad (10⁸ CFU/ml) or LPS_{O6ad} (10 µg/ml) was used as coating antigens.

### 2.7 Opsonic phagocytosis assays

The ability of the anti-O6ad scFv/Fab-anti-tag IgG complexes to opsonize *P. aeruginosa* O6ad for uptake by murine macrophages J774.1A was assessed according to the method in Example 1. In this assay, the QCRL-1 was used as a negative control and te-hS20 (tobacco-derived human anti-O6ad IgG₁) [69] as a positive control. The opsonic phagocytosis assay was also carried out using other *P. aeruginosa* strains PAO1 and O10 to test the target specificity. Further, to determine whether the rAb-IgG complex-mediated opsonic phagocytosis was complement-dependent, the assay was also done in the presence of normal or heat-inactivated murine complement at a final concentration of 1.25%. For the dose response study, the assay was carried out in the same manner as above using the anti-O6ad scFv and Fab at the concentrations ranging from 13.09 nM to 837.5 nM with a molar ratio of rAb to anti-tag IgG at 2 to 1. All experiments were performed in at least duplicate and all treatments were repeated at least three times. The uptaken population of bacteria was calculated according to the formulae: % uptaken bacteria = (uptaken bacterial number at the end of 30 min incubation/initial bacterial number at the beginning of 30 min incubation) x 100%.

To assess whether the rAb-anti-tag IgG complexes mediate the bacterial phagocytosis through interaction with FcγRs present on macrophages, the opsonic phagocytosis assay was also carried out by pre-treatment of the macrophages with the anti-FcγRIIB/III mAb 2.4G2 at the concentrations 50 and 100 times greater than that of the anti-tag IgG applied, as stated previously (Example 1). Incubation of 2.4G2 untreated-macrophages with bacteria alone or antibody-opsonized bacteria, and incubation of 2.4G2-treated macrophages with bacteria alone were used as controls. Experiment was performed in at least duplicate and each treatment was replicated at least three times. The blocked population of bacteria was calculated according to the formulae: % of blocked phagocytosis = [(uptaken bacterial number without 2.4G2 - uptaken bacterial number with 2.4G2)/uptaken bacterial number without 2.4G2] x 100%.

### 2.8 In vivo serum persistence experiment

The *in vivo* serum persistence experiment was performed using CD1 female mice (Charles River Laboratories, Saint-Constant, Quebec, Canada) at 9-10 weeks of age, which were housed under conventional, open-top cage husbandry (Central Animal Facility, Ontario Veterinary College, University of Guelph). Since the *in vivo* serum persistence of a scFv was investigated previously in combination with the anti-tag IgGs (Example 1), in the current example only the anti-O6ad Fab was tested for *in vivo* persistence following complex formation with anti-tag IgGs. The Penta-His was chosen because of its high affinity to epitope-tagged rAbs. In brief, 50 microliters of pre-immune blood was collected via saphenous vein bleed on the day before antibody administration for use as background controls for Fab quantification. The anti-O6ad Fab was labeled with fluorescein isothiocyanate (FITC) using the EZ-Label Kit (Pierce) at FITC:Fab ratios of 8:1. The FITC-labeled Fab was mixed with Penta-His at final concentrations of 0.3 and 0.5 mg/ml, respectively, and then incubated for 1 h at RT before administrated into mice. At time point 0, groups of five CD1 female mice were injected intravenously (*i.v.*) with 100 µl of the antibody cocktail comprised of FITC-labeled Fab (30 µg/mouse) and Penta-His (50 µg/mouse). Control mice received the same volume of FITC-labeled Fab, or the mixture of FITC-labeled Fab and QCRL-1 at the same protein concentrations. Following antibody administration, blood samples were collected by terminal bleeds at 0.25, 0.5, 1, 2, 4, and 8 h time-points, and the sera were obtained by centrifugation (6000 x *g*, 15 min, 4°C) and used for Fab quantification. All animal work was done in accordance with the Guidelines for the Care and Use of Laboratory Animals (CCAC, Ottawa) and protocols approved by the Animal Care Committee of the University of Guelph.

The FITC-labeled Fabs in the collected sera were quantified by ELISA using heat-killed *P. aeruginosa* O6ad cells (10⁸ cells/ml) as a coating antigen and the HRP-conjugated goat anti-human IgG (Pierce) as a detection antibody, as well as by measuring fluorescence intensity using an EnVision™ Multi-Label Plate Reader (Perkin-Elmer, Boston, MA, USA), as described (Example 1).

### 2.9 In vivo protection experiment

The anti-O6ad scFv was chosen as the rAb in combination with anti-tag IgG for the *in vivo* protection study. The *in vivo* protection efficacy of the anti-O6ad scFv following the complex formation with Penta-His to prevent *P. aeruginosa* O6ad infections was investigated using a leukopenic mouse [72, 73] with the following modifications. Following establishment of leucopenia, the antibody cocktails consisting of scFv alone, scFv plus Penta-His, scFv plus QCRL-1, or te-hS20 alone in 80 µl sterile PBS (pH 7.4) was injected intravenously (*i.v.*) via tail vein at doses of 32 µg/mouse for scFv and 80 µg/mouse for mAb; 15 min later after antibody administration, *P. aeruginosa* O6ad cells at LD₈₀₋₉₀ (10³ bacteria/mouse in 50 µl sterile PBS) were administered *i.v.* via tail vein. Mice infected with bacteria and injected with the same volume of PBS were used as negative controls. At least ten mice were used for each treatment and mouse survival was monitored daily for 7 days. The experiment was done blinded. All animal work was undertaken in accordance with Guidelines for the Care and Use of Laboratory Animals (CCAC, Ottawa) and with protocols approved by the Animal Care Committee of the University of Guelph.

### 2.10 Statistical analyses

Statistical analyses were performed using SigmaStat for Windows software (SAS 8.2; SAS Institute, Cary, NC). All data are displayed as mean ± SD/SE (standard deviation or standard error) and graphs were generated using Excel software (MS Office). Comparisons between groups were performed using a One-way Analysis of Variance (ANOVA) multiple comparison test (SAS 8.2); probability values of less than 0.05 were considered significant.

### 3.0 Results

### 3.1 Complex with anti-tag IgGs enhanced antigen binding ability of rAbs

Both Penta-His and 9E10 bound to the epitope-tagged anti-O6ad scFv and Fab in a concentration-dependent manner (Fig. 7). Regardless of the rAb format used, the binding of Penta-His to the epitope-tagged rAbs was much greater than that of 9E10, especially at concentrations less than 7.5 nM (P<0.0001). At concentrations greater than 0.47 nM and less than 1.88 nM, Penta-His still bound the scFv (Fig. 7A) and Fab (Fig. 7B), whereas 9E10 had no binding to either fragment (Fig. 7). No significant difference was obtained in binding between Penta-His and 9E10 when the concentrations applied were higher than 30 nM, indicating that maximum binding was reached. As expected, QCRL-1, an irrelevant anti-tag mAb, had no binding to either the scFv or Fab at all tested antibody concentrations since the epitope recognized by QCRL-1 is not present on these fragments (Fig. 7). These data demonstrate that both Penta-His and 9E10 have high affinity for both epitope-tagged anti-O6ad scFv and Fab, with the Penta-His having the highest affinity. This result is consistent with the fact that Penta-His has a lower equilibrium dissociation constant (Kd) than 9E10 (10 nM for Penta-His vs. 560 nM for 9E10) [30, 31].

To determine if the formation of bivalent rAb-IgG complexes would enhance the antigen binding ability of rAbs, their binding to specific antigens was tested by ELISA. In this experiment, each rAb-IgG complex treatment (e.g., scFv + Penta-His; see Fig. 8) is prepared by mixing the anti-O6ad scFv or Fab with Penta-His, 9E10, or QCRL-1 prior to conducting the ELISA. Each sequential treatment (e.g., scFv, Penta-His; see Fig. 8) was comprised of the scFv or Fab added to the ELISA plate to interact with the attached antigen followed by washing of the plates before addition of one of the three anti-tag IgGs. The scFv or Fab applied together or sequentially with QCRL-1 were used as non-specific controls (i.e., no rAb-IgG complex should form since rAb has no tag recognized by QCRL-1). ELISA results showed that antigen binding ability of both the anti-O6ad scFv (Fig. 8A and 8B) and Fab (Fig. 8C and 8D) to either heat-killed *P. aeruginosa* O6ad (1 x 10⁸ CFU/ml) or LPS_{O6ad} (1 µg/ml) was significantly improved following complex formation with either Penta-His or 9E10 at a molar ratio of 2 to 1 (rAb to anti-tag IgG). At rAb concentrations of 0.94, 3.75, 15, and 60 nM, the binding of the scFv to heat-killed bacteria was 9.8-, 20.8-, 15.6-, and 3.4-fold higher (Fig. 8A) and 239.67-, 263.8-, 29.9-, and 14-fold higher to LPS_{O6ad} (Fig. 8B), respectively, in the rAb-IgG complex format with Penta-His than in the monomeric rAb format. Similar results were obtained when the scFv was complexed with 9E10; the binding to heat-killed bacteria was improved by 9.3-, 49-, 17.2-, and 7.1-fold (Fig. 8A), and to LPS_{O6ad} by 20.2-, 110.3-, 42.6-, and 17.6-fold (Fig. 8B), respectively, at the same rAb concentrations as above. Similarly, at rAb concentrations of 0.94, 3.75, 15, and 60 nM, the binding of the Fab to heat-killed bacteria was increased by 14.9-, 9.9-, 3.7-, and 1.3-fold (Fig. 8C) and by 38.9-, 15.8-, 6.3-, and 2.8-fold to LPS_{O6ad} in the rAb-IgG complex format with Penta-His than in the monomeric rAb format (Fig. 8C). The antigen binding ability of the Fab was also enhanced when complexed with 9E10, as shown by increased binding to heat-killed bacteria by 182.4-, 56.9-, 5.8-, and 1.6-fold (Fig. 8C) and to LPS_{O6ad} by 32.7-, 22.9-, 16.6-, and 4-fold (Fig. 8D), respectively, at the same rAb concentrations as above. No enhancement in antigen binding was observed when the scFv or Fab was mixed with QCRL-1, regardless of antigens used and antibody concentrations applied (Fig. 8). Further, complexes with anti-tag IgGs did not result in an increase in non-specific reactivity of the scFv or Fab with either *P. aeruginosa* PAO1 or O10 or with their corresponding LPSs (data not shown). These data demonstrated that the complex formation of epitope-tagged rAbs with anti-tag IgGs is an effective strategy to improve the binding capacity of rAbs to their targets.

The scFv-Penta-His complex displayed higher binding ability to heat-killed *P. aeruginosa* O6ad than did the scFv-9E10 complex at scFv concentrations greater than 3.75 nM (P<0.0001, Fig. 8A) and to LPS_{O6ad} at scFv concentrations greater than 15 nM (P<0.0001, Fig. 8B). Similarly, the Fab-Penta-His complex was a better binder than the Fab-9E10 complex to heat-killed bacteria (P<0.0001, Fig. 8C) and to LPS_{O6ad} (P<0.0001, Fig. 8D) at Fab concentrations greater than 0.23 nM and 0.94 nM, respectively. Taken together, these data indicate that Penta-His has a higher affinity than 9E10 for the epitope-tagged rAbs, which agrees with the previous description [30, 31].

### 3.2 rAb-IgG Complexes were able to deposit C1q

The ability of the anti-tag IgGs to recruit C1q, the first component of the complement cascade, was evaluated by ELISA. All three anti-tag IgGs, namely Penta-His, 9E10, and QCRL-1, were able to deposit human C1q via their Fc region at the concentration of 66.67 nM (Fig. 9A). 9E10 and QCRL-1 had similar ability to deposit C1q and their ability was 1.4-fold greater than that of Penta-His (P<0.0001, Fig. 9A). The differences in ability to deposit C1q might be related to the gamma chain variations among these murine IgG₁s, which is supported by the fact that IgG₁ variants have different capacities to bind C1q [28].

C1q deposition by the anti-tag IgGs (66.67 nM) was also examined following complex formation with the epitope-tagged anti-O6ad scFv (133.34 nM) or Fab (133.34 nM) at a molar ratio of 2 to 1 (rAb to anti-tag IgG) by ELISA using heat-killed P. *aeruginosa* O6ad (1 x 10⁸ CFU/ml) or LPS_{O6ad} (10 µg/ml) as coating antigens. Regardless of the coating antigens used, both scFv-Penta-His and scFv-9E10 complexes showed greater ability to deposit C1q as compared with the negative control scFv plus QCRL-1 (Fig. 9B). Similar results were obtained when the anti-tag IgGs were complexed with the Fab. The levels of C1q deposited by both Fab-Penta-His and Fab-9E10 complexes were much higher than that by the negative control Fab plus QCRL-1 (Fig. 9C). Regardless of the rAbs used, rAb-9E10 complex showed a greater ability to deposit C1q than rAb-Penta-His IgG complex (P<0.01, Fig. 9B and 9C), especially when LPS_{O6ad} was used as a coating antigen. This further confirms the different capacity of the Fc components of Penta-His and 9E10 to bind C1q. These data demonstrated that the combination of epitope-tagged rAbs with anti-tag IgGs is effective at recruiting complement protein C1q to target antigens through a Fc region provide by the anti-tag IgGs; this interaction does not interfere with rAb antigen-binding specificity.

### 3.3 rAb-IgG Complexes mediated bacterial phagocytosis

All four rAb-IgG complexes, including scFv-Penta-His, scFv-9E10, Fab-Penta-His and Fab-9E10, were able to mediate opsonic phagocytosis of target bacteria P. *aeruginosa* O6ad (1 x 10⁶ CFU) by murine macrophage J774.1A (1 x 10⁵ cells) at protein concentrations of 335 nM for rAbs and 167.5 nM for anti-tag IgGs. As shown in Figure 10, 42% and 24.8% of the total bacterial cells were phagocytosed by J774.1A in the presence of scFv-Penta-His and scFv-9E10 complexes, respectively (Fig. 10A), while 27% and 18.8% were phagocytosed in the presence of Fab-Penta-His and Fab-9E10 complexes, respectively (Fig. 10B). As expected, the presence of the positive control te-hS20 resulted in ca. 50% phagocytosis. By contrast, less than 0.6% of bacteria were phagocytosed by J774.1A without antibodies, while the presence of anti-O6ad scFv (Fig. 10A) and Fab (Fig. 10B) alone resulted in 12.64% and 4.45% phagocytosis, respectively, which were however significantly lower than the percentages when the scFv or Fab was complexed with the anti-tag IgGs (P<0.0001). Therefore, the enhanced bacterial uptake in the presence of rAb-IgG complexes most likely results from the interaction between Fc region and Fcγ receptor (FcyR). As expected, in the absence of the anti-O6ad scFv and Fab, none of the anti-tag IgGs including Penta-His, 9E10, and QCRL-1 showed significant opsonic phagocytosis activity (P>0.05), as compared to the control without antibodies. Moreover, when compared with the percentage of bacteria that underwent phagocytosis in the presence of the anti-O6ad scFv or Fab, the addition of QCRL-1 did not result in significant bacterial uptake (P>0.05). Furthermore, regardless of the rAb-IgG complexes used, the presence of 1.25% complement significantly enhanced bacterial uptake in all cases by approximately 1.5-fold, as compared with treatments in the absence of active complement (Fig. 11A and 11B) These data demonstrated that complex formation with anti-tag IgGs are able to effectively recruit Fc-mediated bacterial phagocytic function to epitope-tagged rAbs. As noted, rAb-Penta-His complexes had a better overall opsonic activity than rAb-9E10 complexes (Fig. 10, Fig. 11), indicating the importance of the affinity of anti-tag IgG to the epitope-tagged rAb in mediating Fc-dependent effector functions. Target specificity studies showed that none of the tested rAb-IgG complexes mediated the phagocytosis against non-specific *P. aeruginosa* strains PAO1 or O10 (Figure 16), strongly suggesting that rAb-IgG-mediated phagocytosis was *P. aeruginosa* O6ad-specific.

### 3.4 2.4G2 partially blocked rAb-IgG-mediated phagocytosis

To confirm that rAb-IgG complexes stimulated the bacterial phagocytic mechanism of macrophages through interaction with FcγRs present on their surface, the phagocytosis assay was carried out by pre-treatment of the IFNy-primed J774.1A macrophages with mAb 2.4G2 raised against mouse FcγRIIB/III [24, 68] to occupy FcyRs prior to addition of rAb-IgG-opsonized *P. aeruginosa* O6ad cells. Regardless of rAbs and anti-tag IgGs used, the opsonic activity of all four rAb-IgG complexes, including scFv-Penta-His, scFv-9E10, Fab-Penta-His, and Fab-9E10, was significantly attenuated by 1 h of pre-incubation of J774.1A cells with 2.4G2 in a concentration-dependent manner (P<0.0001, Fig. 12A); however, their activity was only partially inhibited by 50-65 % and 75-83% using 2.4G2 at concentrations of 50 and 100 times greater than that of the anti-tag IgG (167.5 nM), respectively (Fig. 12A). Longer (2 h) pre-incubation of macrophages with 2.4G2 did not enhance the inhibitory effects on rAb-IgG complex-mediated phagocytosis (data not shown). The incomplete inhibition of phagocytic activity by 2.4G2 may indicate the involvement of FcyRs other than FcγRIIB/III in Fc-mediated bacterial phagocytosis since three separate FcyRs have been found on mouse macrophages [74]. These data demonstrated that rAb-IgG complexes mediated phagocytosis of the target bacteria through interaction with FcyRs expressed on macrophages and showed that more than one type of FcyRs may be involved in this process.

### 3.5 rAb-IgG mediated phagocytosis in a dose-dependent manner

The ability of rAb-IgG complexes to mediate bacterial phagocytosis was also evaluated at various antibody concentrations at a constant 2:1 molar ratio of rAb to anti-tag IgG. All of the tested rAb-IgG complexes, including scFv-Penta-His, scFv-9E10, Fab-Penta-His, and Fab-9E10, mediated phagocytosis of *P. aeruginosa* O6ad by J774.1A in a dose-dependent manner, as indicated by enhanced bacterial uptake with increasing antibody concentrations (Fig. 13). Even at the lowest anti-tag IgG concentration (13.09 nM), the rAb-IgG complexes showed significant activity to mediate bacterial phagocytosis. For example, when compared to the control without antibodies, the presence of scFv-Penta-His and scFv-9E10 significantly increased bacterial uptake by 10.6-fold and 4.6-fold (P<0.05, Fig. 13A), respectively, while both Fab-Penta-His and Fab-9E10 significantly enhanced the bacterial uptake by 3.5-fold (P<0.001, Fig. 13B). In addition, the ability of scFv-Penta-His to mediate bacterial phagocytosis was ca. 2-fold higher than that of scFv-9E10 at anti-tag IgG concentrations greater than 13.09 nM and less than 209.38 nM (P<0.05, Fig. 13A). Similarly, Fab-Penta-His showed at least 1.2-fold higher activity to mediate phagocytosis than Fab-9E10 at all of anti-tag IgG concentrations tested (P<0.05, Fig. 13B). Overall, regardless of the antibody concentrations used, rAb-Penta-His displayed better opsonic ability than did rAb-9E10, which further reflects the high affinity of Penta-His for the epitope-tagged rAbs.

### 3.6 Complex formation with anti-tag IgG prolonged Fab serum persistence

In this example, only the Fab was studied for serum persistence in combination with Penta-His, since the *in vivo* serum persistence of a scFv, specific for *Salmonella enterica* serovar Typhimurium, was already shown to be improved following complex formation with Penta-His in Example 1. Furthermore, Penta-His but not 9E10 was chosen because of its high affinity to the epitope-tagged rAbs. As was measured by ELISA and shown in Figure 14, significantly more 6xHis-tagged Fab remained in serum throughout the course of this experiment when treated with the anti-Penta-His mAb. Both the Fab-alone and Fab-anti-QCRL-1 mAb (negative control) treatments had very similar Fab serum persistences. Furthermore, a rapid drop at 30 min and a slow rise at 1 h in the amounts of the administrated Fab occurred in all cases. This is probably a result of the rapid redistribution of the Fab into tissues within 30 min and then followed by the recirculation into the blood at 1 h after administration.

When compared with QCRL-1, the complex formation with Penta-His prolonged the serum persistence of the Fab by 4.3 fold, as determined by ELISA (Figure 14). Furthermore, the area under the plasma concentration time curve (AUC) was 4.1- fold greater, as determined by ELISA when the Fab was coadministered with Penta-His than with QCRL-, suggesting that the complex formation with Penta-His improves the bioavailability of the administered Fab. Results from these measurements clearly showed the same trends and demonstrated a significant improvement in the serum persistence of the Fab when it forms an immmunocomplex with an anti-tag IgG. The *in vivo* serum persistence experiments indicate that the complex formation with anti-tag IgGs is an effective strategy to extend the serum persistence of epitope-tagged rAbs by increasing their MW and possibly also by providing access to the FcRn-mediated recycling mechanism.

### 3.7 Complex formation with anti-tag IgG enhanced protection efficacy of scFv against P. aeruginosa infection

Pre-treatment of the *P. aeruginosa*-infected mice with a single *i.v.* dose of the anti-O6ad scFv or its parent IgG te-hS20 (32 µg/mouse for scFv and 80 µg/mouse for mAb) prolonged mouse survival as more mice remained alive at the end of the experiment, as compared to the controls that were treated with PBS (Fig. 15). Seventy-two hours post-infection with P. *aeruginosa* O6ad bacteria at LD₈₀₋₉₀ (10³ CFU/mouse), most of the control mice (i.e., 9 of 10 for PBS) were dead, whereas significantly less mice had died when treated with a specific antibody or antibody cocktail (i.e., 1 of 10 for te-hS20, 5 of 11 for scFv, 5 of 10 for scFv plus QCRL-1, and 0 of 10 for scFv-Penta-His). After seven days, 8 of 10, 4 of 11, 3 of 10, and 5 of 10 mice that received single *i.v.* doses of te-hS20, scFv, scFv plus QCRL-1, and scFv-Penta-His, respectively, were still alive. As noted, more mice survived when treated with scFv-Penta-His complex than did those received scFv alone or scFv plus QCRL-1, suggesting the complex formation with Penta-His enhanced the *in vivo* protection efficacy of the scFv, probably as a result of prolonged scFv serum persistence as well as Fc-mediated bacterial elimination mechanism following complex formation with Penta-His. However, the protection capacity of scFv-Penta-His complexes was much less as compared with that of whole IgG te-hS20, with 50% mice alive when treated with scFv-Penta-His and 80% with te-hS20 at the end of the experiment. The inefficient protection might result from inefficiency of murine IgG₁ in mediating Fc-associated effector functions [75-77].

### 4.0 Discussion

Recombinant antibody fragments are gaining favour as a new class of therapeutics; however, their potential application in clinic is often compromised by their rapid clearance through the kidneys and their inability to recruit Fc-mediated effector functions. In the current example, it was demonstrated that by forming complexes with anti-tag IgGs, the therapeutic potency of the epitope-tagged rAbs was markedly improved in antigen binding, *in vivo* serum persistence, and ability to mediate Fc-dependent effector functions. This strategy is based on the epitope tagging technique, in which an anti-epitope tag IgG non-covalently binds a rAb tagged with the specific epitope, thereby forming a trimeric rAb-mAb complex. Therefore, this strategy confers on the complex the bivalency for increased antigen binding avidity, larger MW for longer *in vivo* half-life, and an intact Fc region for recruitment of immune effector functions.

Following complex formation with anti-tag IgGs, the antigen binding of both anti-*P*. *aeruginosa* O6ad LPS scFv and Fab, which were tagged with both 6xHis and c-myc epitopes, was increased by up to 260-fold depending on the antigens, rAbs, anti-tag IgGs, and antibody concentrations tested (Fig. 8). The enhanced antigen binding ability was likely the consequence of increased binding valency, which improves binding avidity following the non-covalent link of two rAbs to one single anti-tag mAb. This approach has been used for screening rAbs with low antigen binding affinity from a phage displaying library, which may be missed by conventional selection methods, and also for increasing the sensitivity of immunoassays to detect the reactivity of rAbs with target antigens [5]. Additionally, the increased binding valency is crucial for effective tumor targeting and therapy by increasing the retention time on targets, thereby subsequently improving the tissue-specific accumulation of the rAbs [35]. Therefore, the formation of a bivalent rAb-mAb complex is beneficial for circulating tumor therapy and pathogen elimination by enhancing the target-binding avidity of the rAb but not suitable for the solid tumor targeting or therapy because of slow tissue penetration of the complex [78].

The Fab displayed significantly prolonged serum residence in mice when coadministered with Penta-His (Fig. 14). This strategy also promoted the serum persistence of a V_{H}H and a scFv (Example 1). The slow clearance and improved bioavailability of the Fab is believed to be a result of the increase in apparent MW [79] following complex formation with the anti-tag IgG. Many pharmacokinetic studies have successfully prolonged *in vivo* half-lives of antibody molecules by increasing the apparent MW via PEGylation [80, 81], linkage to albumin [82, 83], or polysialylation [84]. The increase in apparent MW can reduce renal clearance [79, 85], thereby effectively extending rAb-IgG complex persistence as a monomeric immunocomplex in the circulation, which has been proven to effectively avoid immune clearance mechanisms [86, 87]. Prolonged serum residence may also result from an FcRn-mediated recycling effect, which in part provides IgG with longer serum persistence [88, 89]. In summary, the increased persistence in the systemic circulation would greatly contribute to the therapeutic efficacy of target-specific rAbs.

By complex formation with anti-tag IgG, Fc-mediated effector functions such as complement fixation and phagocyte-dependent bacterial phagocytosis were effectively recruited to targets specified by both scFv and Fab. The *in vitro* binding data showed that all tested rAb-IgG complexes were able to deposit complement C1q to the bacterial surface (Fig. 9B and 9C) via the Fc region provided by the anti-tag IgGs. The enhancement (ca. 1.5-fold) of the rAb-IgG complex-mediated bacterial phagocytosis in the presence of 1.25% whole murine complement (Fig. 11) further confirms the recruitment of complement to the target bacteria. Since complement alone in the absence of antibodies yielded minor bacterial uptake, the enhanced phagocytic activity in the presence of complement likely resulted from the recruitment of complement by rAb-IgG-antigen immune complex and also from C3-mediated bacterial opsonization without assistance of antibodies [90].

All tested rAb-IgG complexes exhibited high opsonic activity for uptake of *P. aeruginosa* O6ad bacteria by J774.1A macrophages (Fig. 10). This opsonic activity was not complement dependent; however, the presence of 1.25% complement greatly enhanced the bacterial uptake (Fig. 11), which may result from simultaneous stimulation of macrophages by both FcγRs and complement receptors [90]. Furthermore, a significant reduction in rAb-IgG-mediated bacterial uptake following FcR blocking with anti-Fc_{γ}RIIB/III mAb 2.4G2 (Fig. 12) confirms the involvement of the Fc region provided by the anti-tag IgGs in the phagocytic process.

The *in vivo* experiment showed that coadministration with Penta-His enhanced the protection efficacy of the anti-O6ad scFv against infection with LD₈₀₋₉₀ (10³ CFU/mouse) *P. aeruginosa* O6ad in a leukopenic mouse model, as indicated by prolonged animal survival and more animals alive at the end of the experiment as compared with the controls treated with scFv alone or scFv plus QCRL-1 (Fig. 15). The fact that this experiment was performed blinded (*i.e*., the identity of each antibody treatment was not known by the experimenter while performing the experiment) strengthens the data. However, scFv-Penta-His was significantly less protective than was the intact mAb te-hS20. The difference in the protective efficacies may result from differences in antigen binding avidity, pharmacokinetic properties, and Fc-mediated effector functions. Coadministration of the scFv with Penta-His does not warrant every single Penta-His binds two scFvs and instead, all of the following components should exist in the circulation: bivalent scFv-Penta-His, monovalent scFv-Penta-His, scFv, and Penta-His. Therefore, the actual amount of bivalent scFv-Penta-His administered was less than that of te-hS20. In addition, the scFv might dissociate from scFv-Penta-His complex in circulation once injected to reach the equilibrium because of the clearance of free scFv through kidneys. More importantly, the less protective ability of the scFv-Penta-His complex might be also related to differences in isotype: anti-Penta-His is murine IgG₁ and te-hS20 is a human IgG1. Despite this, the *in vivo* experiment demonstrated that the immunocomplexing strategy enhanced the protection efficacy of the rAbs.

Regardless of the rAb format used, Penta-His was more efficient than 9E10 at increasing antigen binding of rAbs (greater than 1.2-fold) (Fig. 8), prolonging their serum half-lives (Fig. 14) (Example 1), and recruiting phagocytic activity (1.2-fold greater in the case of Fab and 2-fold in the case of scFv) (Fig. 10). The superior ability of Penta-His to improve the therapeutic potential of rAbs is assumed to be related to its high binding affinity to the target epitope tag (6xHis) added to rAbs (Kd ca. 10 nM for Penta-His vs. Kd ca. 560 nM for 9E10) [30, 31]. This fact is supported by *in vitro* binding experiments which showed that the binding of Penta-His to rAbs was up to 10-fold greater than that of 9E10, especially at lower antibody concentrations (Fig. 7). This result is consistent with a previous pharmacokinetic investigation showing that a fluoresecin-ethanolamine (FL-EA) conjugate, which had high binding to endogenous anti-FL antibodies, persisted longer in circulation than did its lower binding counterpart, eosin Y-EA (EY-EA) [85]. Based on these observations, it is concluded that the binding affinity of an anti-tag mAb to its target epitope tag is a critical factor in determining the efficiency of the epitope tagging technique in therapeutic applications. In addition, the difference in the target-binding ability between Penta-His and 9E10 also may depend on the spatial availability of the 6xHis and c-myc epitope tags of the rAbs to the anti-tag IgGs. In fact, on both of the anti-O6ad scFv and Fab, the 6xHis tag is located at the very end of the C-terminus and thus, is more readily bound by the anti-tag IgG when compared to the c-myc tag, which is located between the gamma heavy chain C-terminus and 6xHis tag. In this respect, to increase the binding capacity, the multiple epitope tags could be used since several groups have shown this to be an effective strategy to increase signal strength in detection applications [91-93]. However, for therapeutic applications, the addition of multiple epitope tags may trigger the formation of polymeric immune complexes that can be rapidly cleared from the systemic circulation [86, 94-96].

In addition to the coadministration of the epitope-tagged rAbs with the anti-tag mAbs, recipients can be actively immunized with the immunogenic epitope tag conjugated to a suitable carrier protein (e.g. KLH) to raise their own serum titers of anti-tag antibodies prior application of epitope-tagged rAbs. This immunization would be an economic way to apply the epitope tag technique to therapeutic applications, since only the rAb would have to be injected and the anti-tag IgG would already be in circulation. Several studies have demonstrated the efficiency of endogenous IgGs as drug carriers for the improvement of pharmacokinetics of small drugs and for systemic drug delivery to target tissues [85, 94, 97, 98]. For example, by binding to endogenous IgG in mice, the half-lives of both CpG oligodeoxynucleotides (CpG-ODNs) [94] and a bispecific diabody [97] were increased by 100-300 fold and 6-fold, respectively. More importantly, when immunocomplexed with endogenous IgG, CpG-ODNs displayed enhanced antitumor activity [94] and the diabody was able to recruit complement, induce mononuclear phagocyte respiratory burst and phagocytosis, as well as promote synergistic cytotoxicity towards colon carcinoma cells in conjunction with CD8⁺ T cells [97]. Therefore, by either coadministration or active immunization, the rAb-anti-tag IgG platform technology described herein is useful for improving therapeutic potential of rAbs of virtually any antigenic target. To be therapeutically useful, epitope tags added to rAbs must be carefully chosen. Both the 6xHis and c-myc epitope tags are very effective tags; however, they have the potential to target endogenous host proteins and induce autoimmune side effects. Therefore, to avoid potential non-specific cross-reaction with endogenous proteins, epitope tags that are not homologous to host proteins and are highly antigenic may also be used.

Regarding the capability to enhance therapeutic potency of rAbs, the strategy disclosed herein has several advantages over other currently used approaches. The strategy disclosed herein not only prolonged the serum persistence of rAbs by increasing the apparent MW but also enhanced the antigen binding avidity by increasing their binding valency via the non-covalent link of two rAbs to one single anti-tag mAb, while the antigen binding affinity of individual rAb is not altered. In comparison, the commonly used approaches for improving the pharmacokinetic profiles of antibody molecules, such as PEGylation [81, 99], polysialylation [84, 100], and coupling to albumin [83, 101, 102] often cause a substantial reduction in the antigen binding activity even at a low modification ratio [36, 80, 103]. Moreover, in the case of whole antibodies, the effector functions of complement fixation and FcR binding are also substantially impaired as a result of modification [79]. Most importantly, the immunocomplexing technique recruited the desired effector functions to rAbs via providing a functional Fc region by the anti-tag IgG, leading to the elimination of target cells via ADCP, ADCC, or CDC [97] and further provided protection against bacterial infection (Fig. 15). Other strategies, such as fusion with the Fc region [104-106] and engineering into bispecific fragments with a second binding site capable of retargeting effector cells [102, 107], could recruit certain effector functions and subsequently lead to target cell-killing [59, 108, 109]. However, these techniques usually involve multiple cloning steps or chemical coupling, require mammalian cells for production, and are also time-consuming. Furthermore, unwanted mispairing of the heavy and light chains associated with bispecific antibody production has been problemic and often compromises large scale production [110-112], thereby greatly impeding their clinical application. By contrast, the technology described herein has the advantage of simplicity. It is achieved by genetic fusion of a short tag motif of choice to rAbs with desired target specificity and by mixing the rAbs with an anti-tag mAb of choice prior to its desired applications. It avoids reliance on mammalian cell culture required for rAb-Fc fusions, and the need for chemical conjugation and additional purification required for PEGylated or polysialylated proteins [103, 113]. Furthermore, epitope-tagged rAbs are produced economically at high yields in bacteria or yeast. Finally, the addition of a tag provides the additional advantage of high quality purification of the tagged rAb in large quantities via affinity chromatography [114].

Another attractive application of the immunocomplexing technique disclosed herein is the generation of polyclonal antibodies (pAb), which may be prepared by mixing one anti-tag mAb with several different rAbs with specificities to different antigenic epitopes on the same antigen. Alternatively, a few anti-tag mAbs of different isotypes may be used in a single preparation of pAb to achieve optimal therapeutic efficacy by triggering all possible Fc-mediated effector functions [19]. Several preclinical studies have shown that the combination of a few mAbs that target different epitopes on the same antigen is substantially more efficient than using one mAb to neutralize a toxin [45] or virus [115, 116], increase clearance of inflammatory cytokines [86], or treat cancer [117-119]. In particular, a mixture of mAbs specific to nine different epitopes on the extracellular domain of HER-2 had more effective anti-tumor activity than each individual mAb both *in vitro* and *in vivo* in a mouse model [118]. They induced different mechanisms of growth inhibition, leading to synergistic cell death when used together [118]. Simultaneous binding to several antigenic epitopes on the same antigen makes mAbs readily trigger complement activation and FcyR engagement [6].

Taken together, the data demonstrated that overall activity of rAbs is promoted by non-covalent binding to the anti-tag IgGs. This immunocomplexing provides a new way to extend the activity of rAbs by combining the affinity and specificity of the rAbs with the bivalency, pharmacokinetics, and effector functions of an intact mAb. This strategy is broadly applicable to improve the therapeutic potential of many other rAbs with different specificities.

While the present disclosure has been described with reference to what are presently considered to be the preferred examples, it is to be understood that the disclosure is not limited to the disclosed examples.

**Table 1: Non-exhaustive list of epitopes and known full-length Abs that bind to the epitopes**

| Epitope Name | Amino Acid Sequence | Antibody that binds to the epitope | Source | Reference |
|---|---|---|---|---|
| c-MYC1 | EQKLISEEDL (SEQ ID NO: 10) (amino acids 410-419 of human c-myc protein) | MAb 9E10 | | Evan *et al.,* 1985 [12] |
| POLY HIS | H_{≥5} | Anti-His MAb | QIAgen | |
| FLAG® | DYKDDDDK (SEQ ID NO: 11) | Anti-FLAG M1, M2 and M5 MAbs | Sigma/Kodak | Thomas et *al*., 1988 [121] |
| V5 | GKPIPNPLLGLDST (SEQ ID NO: 12) | Anti-V5 Antibody | | |
| QCRL-1³ | SSYSGDI (SEQ ID NO: 13) (amino acids 918-924 of human MRP) | Anti-QCRL-1 MAb | | Hipfner *et al*., 1997 [120] |

### FULL CITATIONS FOR REFERENCES REFERRED TO IN THE SPECIFICATION

1. Holliger, P. and P.J. Hudson; Engineered antibody fragments and the rise of single domains. Nat Biotechnol, 2005. 23(9): p. 1126-36.
2. Wu, A.M. and P.D. Senter, Arming antibodies: prospects and challenges for immunoconjugates. Nat Biotech, 2005. 23(9): p. 1137-1146.
3. Liu, X., L.M. Pop, and E.S. Vitetta, Engineering therapeutic monoclonal antibodies. Immunol Rev, 2008. 222(1): p. 9-27.
4. Maue, R.A., Understanding ion channel biology using epitope tags: Progress, pitfalls, and promise. J Cell Physiol, 2007. 213(3): p. 618-625.
5. Wang, X., et al., Enhancement of scFv fragment reactivity with target antigens in binding assays following mixing with anti-tag monoclonal antibodies. J Immunol Methods, 2004. 294(1-2): p. 23-35.
6. Desjarlais, J.R., et al., Optimizing engagement of the immune system by anti-tumor antibodies: an engineer's perspective. Drug Discov Today, 2007. 12(21-22): p. 898-910.
7. Muyldermans, S., Single domain camel antibodies: current status. J Biotechnol, 2001. 74(4): p. 277-302.
8. Coomber, D.W., Panning of antibody phage-display libraries. Standard protocols. Methods Mol Biol, 2002. 178: p. 133-45.
9. Amstutz, P., et al., In vitro display technologies: novel developments and applications. Curr Opin Biotechnol, 2001. 12(4): p. 400-5.
10. Hanes, J., L. Jermutus, and A. Pluckthun, Selecting and evolving functional proteins in vitro by ribosome display. Methods Enzymol, 2000. 328: p. 404-30.
11. Pluckthun, A., Escherichia coli producing recombinant antibodies. Bioprocess Technol, 1994. 19: p. 233-52.
12. Joosten, V., et al., The production of antibody fragments and antibody fusion proteins by yeasts and filamentous fungi. Microb Cell Fact, 2003. 2(1): p. 1.
13. Kruger, C., et al., In situ delivery of passive immunity by lactobacilli producing single-chain antibodies. Nat Biotechnol, 2002. 20(7): p. 702-6.
14. Harrison, J.S. and E. Keshavarz-Moore, Production of antibody fragments in Escherichia coli. Ann N Y Acad Sci, 1996. 782: p. 143-58.
15. Enders, J.F., T.H. Weller, and F.C. Robbins, Cultivation of the Lansing Strain of Poliomyelitis Virus in Cultures of Various Human Embryonic Tissues. Science, 1949. 109(2822): p. 85-87.
16. Chu, L. and D. K. Robinson, Industrial choices for protein production by large-scale cell culture. Curr Opin Biotechnol, 2001. 12(2): p. 180-7.
17. Cacciuttolo and etal, Large scale production of a monoclonal IgM in a hybridoma suspension culture. Pharm Technol, 1998. 22: p. 44-58.
18. Kalyanpur, M., Downstream processing in the biotechnology industry. Mol Biotechnol, 2002. 22(1): p. 87-98.
19. Logtenberg, T., Antibody cocktails: next-generation biopharmaceuticals with improved potency. Trends Biotechnol, 2007. 25(9): p. 390-4.
20. Hipfner, D.R., et al., Location of a protease-hypersensitive region in the multidrug resistance protein (MRP) by mapping of the epitope of MRP-specific monoclonal antibody QCRL-1. Cancer Res, 1996. 56(14): p. 3307-3314.
21. Weisser, N.E., K.C. Almquist, and J.C. Hall, A rAb screening method for improving the probability of identifying peptide mimotopes of carbohydrate antigens. Vaccine, 2007. 25(23): p. 4611-22.
22. Zhang, J., et al., A pentavalent single-domain antibody approach to tumor antigen discovery and the development of novel proteomics reagents. J Mol Biol, 2004. 341(1): p. 161-169.
23. Ralph, P. and I. Nakoinz, Phagocytosis and cytolysis by a macrophage tumour and its cloned cell line. Nature, 1975. 257(5525): p. 393-394.
24. Unkeless, J.C., Characterization of a monoclonal antibody directed against mouse macrophage and lymphocyte Fc receptors. J Exp Med, 1979. 150(3): p. 580-96.
25. Evan, G.I., et al., Isolation of monoclonal antibodies specific for human c-myc proto-oncogene product. Mol Cell Biol, 1985. 5(12): p. 3610-6.
26. Deng, S., et al., Basis for selection of improved carbohydrate-binding single-chain antibodies from synthetic gene libraries. Proc Natl Acad Sci USA, 1995. 92(11): p. 4992-4996.
27. Coligan, J.E., Current protocols in immunology. Vol. 14.2. 2001, New York: Greene Pub. Associates and Wiley-Interscience.
28. Idusogie, E.E., et al., Mapping of the C1q binding site on rituxan, a chimeric antibody with a human IgG1 Fc. J Immunol, 2000. 164(8): p. 4178-84.
29. Underhill, D.M. and A. Ozinsky, Phagocytosis of microbes: Complexity in Action. Ann Rev Immunol, 2002. 20(1): p. 825-852.
30. Muller, K.M., et al., Tandem immobilized metal-ion affinity chromatography/immunoaffinity purification of His-tagged proteinsevaluation of two anti-His-tag monoclonal antibodies. Anal Biochem, 1998. 259(1): p. 54-61.
31. Hilpert, K., et al., Anti-c-myc antibody 9E10: epitope key positions and variability characterized using peptide spot synthesis on cellulose. Protein Eng, 2001. 14(10): p. 803-6.
32. Kishore, U. and K.B.M. Reid, C1q: Structure, function, and receptors. Immunopharmacology, 2000. 49(1-2): p. 159-170.
33. Haraga, A., M.B. Ohlson, and S.I. Miller, Salmonellae interplay with host cells. Nat Rev Micro, 2008. 6(1): p. 53-66.
34. Prost, L.R., S. Sanowar, and S.I. Miller, Salmonella sensing of anti-microbial mechanisms to promote survival within macrophages. Immunol Rev, 2007. 219(1): p. 55-65.
35. Adams, G.P., et al., Avidity-mediated enhancement of in vivo tumor targeting by single-chain Fv dimers. Clin Cancer Res, 2006. 12(5): p. 1599-605.
36. Kubetzko, S., et al., PEGylation and multimerization of the anti-p185HER-2 single chain Fv fragment 4D5: effects on tumor targeting. J Biol Chem, 2006. 281(46): p. 35186-201.
37. Natsume, A., et al., Fucose removal from complex-type oligosaccharide enhances the antibody-dependent cellular cytotoxicity of single-gene-encoded bispecific antibody comprising of two single-chain antibodies linked to the antibody constant region. J Biochem, 2006. 140(3): p. 359-368.
38. Shahied, L.S., et al., Bispecific minibodies targeting HER2/neu and CD16 exhibit improved tumor lysis when placed in a divalent tumor antigen binding format. J Biol Chem, 2004. 279(52): p. 53907-53914.
39. Nimmerjahn, F. and J.V. Ravetch, Antibodies, Fc receptors and cancer. Curr Opin Immunol, 2007. 19(2): p. 239-245.
40. Wilson, I.A., et al., The structure of an antigenic determinant in a protein. Cell, 1984. 37(3): p. 767-778.
41. Southern, J.A., et al., Identification of an epitope on the P and V proteins of simian virus 5 that distinguishes between two isolates with different biological characteristics. J Gen Virol, 1991. 72(7): p. 1551-1557.
42. Grussenmeyer, T., et al., Complexes of polyoma virus medium T antigen and cellular proteins. Proc Natl Acad Sci USA, 1985. 82(23): p. 7952-7954.
43. MacArthur, H. and G. Walter, Monoclonal antibodies specific for the carboxy terminus of simian virus 40 large T antigen. J Virol, 1984. 52(2): p. 483-491.
44. Glassy, M.C. and M.E. McKnight, Requirements for human antibody cocktails for oncology. Expert Opin Biol Ther, 2005. 5(10): p. 1333-1338.
45. Nowakowski, A., et al., Potent neutralization of botulinum neurotoxin by recombinant oligoclonal antibody. Proc Natl Acad Sci USA, 2002. 99(17): p. 11346-50.
46. Volk, W.A., et al., Neutralization of tetanus toxin by distinct monoclonal antibodies binding to multiple epitopes on the toxin molecule. Infect Immun, 1984. 45(3): p. 604-609.
47. Zwick, M.B., et al., Neutralization synergy of human immunodeficiency virus type 1 primary isolates by cocktails of broadly neutralizing antibodies. J Virol, 2001. 75(24): p. 12198-12208.
48. Den, W., et al., A bidirectional phage display vector for the selection and mass transfer of polyclonal antibody libraries. J Immunol Methods, 1999. 222(1-2): p. 45-57.
49. Santora, K.E., et al., Generation of a polyclonal Fab phage display library to the human breast carcinoma cell line BT-20. Comb Chem High Throughput Screen, 2000. 3(1): p. 51-57.
50. Sharon, J., et al., Recombinant polyclonal antibody libraries. Comb Chem High Throughput Screen, 2000. 3(3): p. 185-96.
51. Chen, L., et al., Polyclonal Fab phage display libraries with a high percentage of diverse clones to Cryptosporidium parvum glycoproteins. Int J Parasitol, 2003. 33(3): p. 281-291.
52. Williams, B.R. and J. Sharon, Polyclonal anti-colorectal cancer Fab phage display library selected in one round using density gradient centrifugation to separate antigen-bound and free phage. Immunology Lett, 2002. 81(2): p. 141-148.
53. Lonberg, N., Human antibodies from transgenic animals. Nat Biotech, 2005. 23(9): p. 1117-1125.
54. Green, L.L., et al., Antigen-specific human monoclonal antibodies from mice engineered with human Ig heavy and light chain YACs. Nat Genet, 1994. 7(1): p. 13-21.
55. Jakobovits, A., et al., From XenoMouse technology to panitumumab, the first fully human antibody product from transgenic mice. Nat Biotech, 2007. 25(10): p. 1134-1143.
56. Kuroiwa, Y., et al., Sequential targeting of the genes encoding immunoglobulin-mu and prion protein in cattle. Nat Genet, 2004. 36(7): p. 775-780.
57. Richt, J.A., et al., Production of cattle lacking prion protein. Nat Biotech, 2007. 25(1): p. 132-138.
58. Skerra, A. and A. Pluckthun, Assembly of a functional immunoglobulin Fv fragment in Escherichia coli. Science, 1988. 240(4855): p. 1038-41.
59. Muller, D. and R.E. Kontermann, Recombinant bispecific antibodies for cellular cancer immunotherapy. Curr Opin Mol Ther, 2007. 9(4): p. 319-26.
60. Maynard, J.A., et al., Protection against anthrax toxin by recombinant antibody fragments correlates with antigen affinity. Nat Biotechnol, 2002. 20(6): p. 597-601.
61. Mozdzanowska, K., J. Feng, and W. Gerhard, Virus-neutralizing activity mediated by the Fab fragment of a hemagglutinin-specific antibody is sufficient for the resolution of influenza virus infection in SCID mice. J Virol, 2003. 77(15): p. 8322-8.
62. Behr, T.M., D.M. Goldenberg, and W. Becker, Reducing the renal uptake of radiolabeled antibody fragments and peptides for diagnosis and therapy: present status, future prospects and limitations. Eur J Nucl Med, 1998. 25(2): p. 201-12.
63. Kuss-Reichel, K., Grauer, L., Karavodin, L.M., Knott, C., Krusemeier, M., and Kay, N.E., Will immunogenicity limit the use, efficacy, and future development of therapeutic monoclonal antibodies. Clin Diagn Lab Immunol, 1994. 1: p. 365-372.
64. Kenanova, V., et al., Radioiodinated versus radiometal-labeled anti-carcinoembryonic antigen single-chain Fv-Fc antibody fragments: optimal pharmacokinetics for therapy. Cancer Res, 2007. 67(2): p. 718-26.
65. Wu, A.M., et al., Anti-carcinoembryonic antigen (CEA) diabody for rapid tumor targeting and imaging. Tumor Targeting, 1999. 4: p. 47-58.
66. Lo, A.S., Q. Zhu, and W.A. Marasco, Intracellular antibodies (intrabodies) and their therapeutic potential. Handb Exp Pharmacol, 2008(181): p. 343-73.
67. Yi, E.C. and M. Hackett, Rapid isolation method for lipopolysaccharide and lipid A from gram-negative bacteria. Analyst, 2000. 125(4): p. 651-6.
68. Balogh, P., J.G. Tew, and A.K. Szakal, Simultaneous blockade of Fc gamma receptors and indirect labeling of mouse lymphocytes by the selective detection of allotype-restricted epitopes on the kappa chain of rat monoclonal antibodies. Cytometry, 2002. 47(2): p. 107-10.
69. McLean, M.D., et al., A human anti-Pseudomonas aeruginosa serotype O6ad immunoglobulin G1 expressed in transgenic tobacco is capable of recruiting immune system effector function in vitro. Antimicrob Agents Chemother, 2007. 51(9): p. 3322-8.
70. Palczewska, M., P. Groves, and J. Kuznicki, Use of Pichia pastoris for the expression, purification, and characterization of rat calretinin "EF-hand" domains. Protein Expr Purif, 1999. 17(3): p. 465-76.
71. Palczewska, M., G. Batta, and P. Groves, Concanavalin A-agarose removes mannan impurities from an extracellularly expressed Pichia pastoris recombinant protein. Cell Mol Biol Lett, 2003. 8(3): p. 783-92.
72. Pier, G.B., et al., In vitro and in vivo activity of polyclonal and monoclonal human immunoglobulins G, M, and A against Pseudomonas aeruginosa lipopolysaccharide. Infect Immun, 1989. 57(1): p. 174-9.
73. Schreiber, J.R., et al., Anti-idiotype-induced, lipopolysaccharide-specific antibody response to Pseudomonas aeruginosa. II. Isotype and functional activity of the anti-idiotype-induced antibodies. J Immunol, 1991. 146(1): p. 188-93.
74. Vincendeau, P., M. Daeron, and S. Daulouede, Identification of antibody classes and Fc receptors responsible for phagocytosis of Trypanosoma musculi by mouse macrophages. Infect Immun, 1986. 53(3): p. 600-5.
75. Azeredo da Silveira, S., et al., Complement activation selectively potentiates the pathogenicity of the IgG2b and IgG3 isotypes of a high affinity anti-erythrocyte autoantibody. J Exp Med, 2002. 195(6): p. 665-72.
76. Michaelsen, T.E., et al., The four mouse IgG isotypes differ extensively in bactericidal and opsonophagocytic activity when reacting with the P1.16 epitope on the outer membrane PorA protein of Neisseria meningitidis. Scand J Immunol, 2004. 59(1): p. 34-9.
77. Ralph, P., et al., All classes of murine IgG antibody mediate macrophage phagocytosis and lysis of erythrocytes. J Immunol, 1980. 125(5): p. 1885-8.
78. Jain, R.K. and L.T. Baxter, Mechanisms of heterogeneous distribution of monoclonal antibodies and other macromolecules in tumors: significance of elevated interstitial pressure. Cancer Res, 1988. 48(24 Pt 1): p. 7022-32.
79. Caliceti, P. and F.M. Veronese, Pharmacokinetic and biodistribution properties of poly(ethylene glycol)-protein conjugates. Adv Drug Deliv Rev, 2003. 55(10): p. 1261-77.
80. Chapman, A.P., PEGylated antibodies and antibody fragments for improved therapy: a review. Adv Drug Deliv Rev 2002. 54: p. 531-545.
81. Fishburn, C.S., The Pharmacology of PEGylation: Balancing PD with PK to Generate Novel Therapeutics. Journal of Pharmaceutical Sciences, 2007. (www.interscience.wilev.com): p. 1-17.
82. Chaudhury, C., et al., Albumin binding to FcRn: distinct from the FcRn-IgG interaction. Biochemistry, 2006. 45(15): p. 4983-90.
83. Muller, D., et al., Improved pharmacokinetics of recombinant bispecific antibody molecules by fusion to human serum albumin. J Biol Chem, 2007. 282(17): p. 12650-60.
84. Gregoriadis, G., et al., Improving the therapeutic efficacy of peptides and proteins: a role for polysialic acids. Int J Pharm, 2005. 300(1-2): p. 125-30.
85. Rehlaender, B.N. and M.J. Cho, Anti-drug antibodies as drug carriers. I. For small molecules. Pharm Res, 2001. 18(6): p. 745-52.
86. Montero-Julian, F.A., et al., Pharmacokinetic study of anti-interleukin-6 (IL-6) therapy with monoclonal antibodies: enhancement of IL-6 clearance by cocktails of anti-IL-6 antibodies. Blood, 1995. 85(4): p. 917-24.
87. Skogh, T., et al., Physicochemical properties and blood clearance of human serum albumin conjugated to different extents with dinitrophenyl groups. Int Arch Allergy Appl Immunol, 1983. 70(3): p. 238-44.
88. Junghans, R.P. and C.L. Anderson, The protection receptor for IgG catabolism is the beta2-microglobulin-containing neonatal intestinal transport receptor. Proc Natl Acad Sci U S A, 1996. 93(11): p. 5512-6.
89. Lencer, W.I. and R.S. Blumberg, A passionate kiss, then run: exocytosis and recycling of IgG by FcRn. Trends Cell Biol, 2005. 15(1): p. 5-9.
90. Vasta, G.R., et al., C-type lectins and galectins mediate innate and adaptive immune functions: their roles in the complement activation pathway. Dev Comp Immunol, 1999. 23(4-5): p. 401-20.
91. Hernan, R., K. Heuermann, and B. Brizzard, Multiple epitope tagging of expressed proteins for enhanced detection. Biotechniques, 2000. 28(4): p. 789-93.
92. Nakajima, K. and Y. Yaoita, Construction of multiple-epitope tag sequence by PCR for sensitive Western blot analysis. Nucleic Acids Res, 1997. 25(11): p. 2231-2.
93. Zhang, L., R. Hernan, and B. Brizzard, Multiple tandem epitope tagging for enhanced detection of protein expressed in mammalian cells. Mol Biotechnol, 2001. 19(3): p. 313-21.
94. Palma, E. and M.J. Cho, Improved systemic pharmacokinetics, biodistribution, and antitumor activity of CpG oligodeoxynucleotides complexed to endogenous antibodies in vivo. J Control Release, 2007. 120(1-2): p. 95-103.
95. Schumaker, V.N., G. Green, and R.L. Wilder, A theory of bivalent antibody-bivalent hapten interactions. Immunochemistry, 1973. 10(8): p. 521-8.
96. Wilder, R.L., G. Green, and V.N. Schumaker, Bivalent hapten-antibody interactions--III. Formation of an unusual polymer by IGA (MOPC 315) upon binding a bivalent hapten. Immunochemistry, 1975. 12(1): p. 54-60.
97. Holliger, P., et al., Retargeting serum immunoglobulin with bispecific diabodies. Nat Biotechnol, 1997. 15(7): p. 632-6.
98. Rehlaender, B.N. and M.J. Cho, Antibodies as drug carriers. II. For proteins. Pharm Res, 2001. 18(6): p. 753-60.
99. Yang, K., et al., Tailoring structure-function and pharmacokinetic properties of single-chain Fv proteins by site-specific PEGylation. Protein Eng, 2003. 16(10): p. 761-70.
100. Constantinou, A., et al., Modulation of antibody pharmacokinetics by chemical polysialylation. Bioconjug Chem, 2008. 19(3): p. 643-50.
101. Holt, L.J., et al., Anti-serum albumin domain antibodies for extending the half-lives of short lived drugs. Protein Eng Des Sel, 2008. 21(5): p. 283-8.
102. Muller, D., et al., Improved pharmacokinetics of recombinant bispecific antibody molecules by fusion to human serum albumin. J Bio Chem, 2007. 282(17): p. 12650-12660.
103. Kubetzko, S., C.A. Sarkar, and A. Pluckthun, Protein PEGylation decreases observed target association rates via a dual blocking mechanism. Mol Pharmacol, 2005. 68(5): p. 1439-54.
104. Lu, D., et al., Simultaneous blockade of both the epidermal growth factor receptor and the insulin-like growth factor receptor signaling pathways in cancer cells with a fully human recombinant bispecific antibody. J Biol Chem, 2004. 279(4): p. 2856-65.
105. Powers, D.B., et al., Expression of single-chain Fv-Fc fusions in Pichia pastoris. J Immunol Methods, 2001. 251(1-2): p. 123-35.
106. Rossi, E.A., et al., Novel designs of multivalent anti-CD20 humanized antibodies as improved lymphoma therapeutics. Cancer Res, 2008. 68(20): p. 8384-92.
107. Germain, C., et al., Redirecting NK cells mediated tumor cell lysis by a new recombinant bifunctional protein. Protein Eng Des Sel, 2008. 21(11): p. 665-72.
108. Marvin, J.S. and Z. Zhu, Bispecific antibodies for dual-modality cancer therapy: killing two signaling cascades with one stone. Curr Opin Drug Discov Devel, 2006. 9(2): p. 184-93.
109. Nieri, P., et al., Antibodies for therapeutic uses and the evolution of biotechniques. Curr Med Chem, 2009. 16(6): p. 753-79.
110. Suresh, M.R., A.C. Cuello, and C. Milstein, Bispecific monoclonal antibodies from hybrid hybridomas. Methods Enzymol, 1986. 121: p. 210-28.
111. Schoonooghe, S., et al., Efficient production of human bivalent and trivalent anti-MUC1 Fab-scFv antibodies in Pichia pastoris. BMC Biotechnol, 2009. 9: p. 70.
112. Schoonjans, R., et al., Fab chains as an efficient heterodimerization scaffold for the production of recombinant bispecific and trispecific antibody derivatives. J Immunol, 2000. 165(12): p. 7050-7.
113. Bailon, P., et al., Rational design of a potent, long-lasting form of interferon: a 40 kDa branched polyethylene glycol-conjugated interferon alpha-2a for the treatment of hepatitis C. Bioconjug Chem, 2001. 12(2): p. 195-202.
114. Brizzard, B., Epitope tagging. Biotechniques, 2008. 44(5): p. 693-5.
115. lorio, R.M. and M.A. Bratt, Neutralization of Newcastle disease virus by monoclonal antibodies to the hemagglutinin-neuraminidase glycoprotein: requirement for antibodies to four sites for complete neutralization. J Virol, 1984. 51(2): p. 445-51.
116. Martinez, I. and J.A. Melero, Enhanced neutralization of human respiratory syncytial virus by mixtures of monoclonal antibodies to the attachment (G) glycoprotein. J Gen Virol, 1998. 79 (Pt 9): p. 2215-20.
117. Nahta, R., M.C. Hung, and F.J. Esteva, The HER-2-targeting antibodies trastuzumab and pertuzumab synergistically inhibit the survival of breast cancer cells. Cancer Res, 2004. 64(7): p. 2343-6.
118. Spiridon, C.I., et al., Targeting multiple Her-2 epitopes with monoclonal antibodies results in improved antigrowth activity of a human breast cancer cell line in vitro and in vivo. Clin Cancer Res, 2002. 8(6): p. 1720-30.
119. Tonra, J.R., et al., Synergistic antitumor effects of combined epidermal growth factor receptor and vascular endothelial growth factor receptor-2 targeted therapy. Clin Cancer Res, 2006. 12(7 Pt 1): p. 2197-207.
120. Hipfner, D.R., et al., Membrane topology of the multidrug resistance protein (MRP). A study of glycosylation-site mutants reveals an extracytosolic NH2 terminus. J Biol Chem, 1997. 272(38): p. 23623-30.
121. Thomas P. Hopp1, Kathryn S. Prickett1, Virginia L. Price1, Randell T. Libby2, Carl J. March1, Douglas Pat Cerretti1, David L. Urdal1 & Paul J. Conlon1 A Short Polypeptide Marker Sequence Useful for Recombinant Protein Identification and Purification. Bio/Technology 6, 1204 - 1210 (1988).

## Claims

1. An antibody fragment linked to an epitope and an antibody that binds to the epitope for use in treating an infection from bacteria or viruses in an animal in need thereof, wherein:
(i) the antibody fragment linked to the epitope is to be administered to the animal; and
(ii) the antibody that binds to the epitope is to be administered to the animal prior to, at the same time or after said antibody fragment linked to the epitope;
wherein upon use a complex forms between the antibody fragment linked to the epitope and the antibody that binds to the epitope, thereby enhancing the efficacy of the antibody fragment compared to an equivalent uncomplexed antibody fragment, and wherein the antibody fragment binds to a target antigen selected from bacterial antigens or viral antigens.

2. The antibody fragment linked to an epitope and an antibody that binds to the epitope for use as claimed in claim 1 wherein the enhanced efficacy of the antibody fragment comprises (i) an increased therapeutic effect of the antibody fragment; (ii) an increased persistence and/or stability of the antibody fragment; or (iii) an increased immune response.

3. The antibody fragment linked to an epitope and an antibody that binds to the epitope for use as claimed in claim 2 wherein the increased therapeutic effect comprises enhanced protective efficacy of the antibody fragment against bacterial infection.

4. The antibody fragment linked to an epitope and an antibody that binds to the epitope for use as claimed in claim 1 wherein the enhanced efficacy of the antibody fragment comprises (i) activation of downstream immune system functions; (ii) increased recruitment of FcR-mediated effector functions; or (iii) recruitment of the complement system and/or increasing phagocytosis.

5. The antibody fragment linked to an epitope and an antibody that binds to the epitope for use as claimed in any one of claims 1 to 4 wherein the antibody fragment linked to the epitope is a fusion protein.

6. The antibody fragment linked to an epitope and an antibody that binds to the epitope for use as claimed in any one of claims 1 to 4 wherein the antibody fragment is linked to the epitope via a chemical crosslink.

7. The antibody fragment linked to an epitope and an antibody that binds to the epitope for use as claimed in any one of claims 1 to 6 wherein the antibody fragment is selected from: scFv antibodies, disulphide stabilized scFv fragments, VHH single domain antibodies and Fab antibodies.

8. The antibody fragment linked to an epitope and an antibody that binds to the epitope for use as claimed in any one of claims 1 to 7 wherein the epitope is selected from: cellular antigens, humoral antigens, pathogens, toxins, viruses, bacteria, tumour antigens or autoantigens, glutathione-S-transferase (GST) or portion thereof, c-Myc or portion thereof, poly-histidine (6X-His), penta-histidine (Penta-His), FLAG®, green fluorescent protein (GFP) or portion thereof, maltose binding protein (MBP) or portion thereof, influenza A virus haemaglutinin (HA) tag; YPYDVPDYA (SEQ ID NO:1)) or portion thereof, β-galactosidase (β-gal) or portion thereof, GAL4 or portion thereof, human MRP or portion thereof, V5 epitope from the simian virus, polyoma virus T antigen epitopes, QCRL-1 and the KT3 viral epitope or portions thereof.

9. The antibody fragment linked to an epitope and an antibody that binds to the epitope for use as claimed in any one of claims 1 to 8 wherein the antibody is selected from a polyclonal antibody, a monoclonal antibody, an IgG, an IgM, an IgA, an IgE and an IgD.

10. The antibody fragment linked to an epitope and an antibody that binds to the epitope for use as claimed in any one of claims 1 to 9 wherein the antibody fragment forms a complex with the antibody in a 20:1 ratio or a 2:1 ratio.

## Patentansprüche

1. Antikörperfragment, verbunden mit einem Epitop und einem Antikörper, der an das Epitop bindet, zur Verwendung beim Behandeln einer Infektion von Bakterien oder Viren in einem Lebewesen bei Bedarf dafür, wobei:
(i) das mit dem Epitop verbundene Antikörperfragment an das Lebewesen zu verabreichen ist; und
(ii) der Antikörper, der an das Epitop bindet, an das Lebewesen vor, gleichzeitig oder nachdem das Antikörperfragment mit dem Epitop verbunden worden ist, zu verabreichen ist;
wobei bei Verwendung sich ein Komplex zwischen dem mit dem Epitop verbundenen Antikörperfragment und dem Antikörper, der an das Epitop bindet, bildet, wodurch die Effizienz des Antikörperfragments, verglichen mit einem äquivalenten nicht komplexierten Antikörperfragment, erhöht wird und wodurch das Antikörperfragment an ein Zielantigen, ausgewählt aus bakteriellen Antigenen oder viralen Antigenen, bindet.

2. Antikörperfragment, verbunden mit einem Epitop und einem Antikörper, der an das Epitop bindet, zur Verwendung nach Anspruch 1, wobei die erhöhte Effizienz des Antikörperfragments (i) einen erhöhten therapeutischen Effekt des Antikörperfragments; (ii) eine erhöhte Persistenz und/oder Stabilität des Antikörperfragments; oder (iii) eine erhöhte Immunantwort umfasst.

3. Antikörperfragment, verbunden mit einem Epitop und einem Antikörper, der an das Epitop bindet, zur Verwendung nach Anspruch 2, wobei der erhöhte therapeutische Effekt erhöhte protektive Effizienz des Antikörperfragments gegen bakterielle Infektion umfasst.

4. Antikörperfragment, verbunden mit einem Epitop und einem Antikörper, der an das Epitop bindet, zur Verwendung nach Anspruch 1, wobei die erhöhte Effizienz des Antikörperfragments (i) Aktivierung von Stromabwärts-Immunsystem-Funktionen; (ii) erhöhte Beschaffung von FcR-vermittelten Effektorfunktionen; oder (iii) Beschaffung des Komplementsystems und/oder sich erhöhender Phagozytose umfasst.

5. Antikörperfragment, verbunden mit einem Epitop und einem Antikörper, der an das Epitop bindet, zur Verwendung nach einem der Ansprüche 1 bis 4, wobei das mit dem Epitop verbundene Antikörperfragment ein Fusionsprotein ist.

6. Antikörperfragment, verbunden mit einem Epitop und einem Antikörper, der an das Epitop bindet, zur Verwendung nach einem der Ansprüche 1 bis 4, wobei das Antikörperfragment mit dem Epitop über eine chemische Vernetzung verbunden ist.

7. Antikörperfragment, verbunden mit einem Epitop und einem Antikörper, der an das Epitop bindet, zur Verwendung nach einem der Ansprüche 1 bis 6, wobei das Antikörperfragment ausgewählt ist aus: scFv-Antikörpern, Disulfid stabilisierten scFv-Fragmenten, VHH-Einzeldomän-Antikörpern und Fab-Antikörpern.

8. Antikörperfragment, verbunden mit einem Epitop und einem Antikörper, der an das Epitop bindet, zur Verwendung nach einem der Ansprüche 1 bis 7, wobei das Epitop ausgewählt ist aus: zellulären Antigenen, humoralen Antigenen, Pathogenen, Toxinen, Viren, Bakterien, Tumor-Antigenen oder Autoantigenen, Glutathion-S-Transferase (GST) oder einem Abschnitt davon, c-Myc oder einem Abschnitt davon, Poly-histidin (6X-His), Penta-histidin (Penta-His), FLAG®, grün fluoreszierendem Protein (GFP) oder einem Abschnitt davon, Maltose bindendem Protein (MBP) oder einem Abschnitt davon, Influenza A-Virus-Hämaglutinin (HA) tag; YPYDVPDYA (SEQ ID NR.:1)) oder einem Abschnitt davon, β-Galactosidase (β-gal) oder einem Abschnitt davon, GAL4 oder einem Abschnitt davon, Human-MRP oder einem Abschnitt davon, V5-Epitop von dem Simianvirus, Polyomavirus T-Antigen-Epitopen, QCRL-1 und dem KT3 viralen Epitop oder Abschnitten davon.

9. Antikörperfragment, verbunden mit einem Epitop und einem Antikörper, der an das Epitop bindet, zur Verwendung nach einem der Ansprüche 1 bis 8, wobei der Antikörper ausgewählt ist aus einem polyklonalen Antikörper, einem monoklonalen Antikörper, einem IgG, einem IgM, einem IgA, einem IgE und einem IgD.

10. Antikörperfragment, verbunden mit einem Epitop und einem Antikörper, der an das Epitop bindet, zur Verwendung nach einem der Ansprüche 1 bis 9, wobei das Antikörperfragment einen Komplex mit dem Antikörper in einem Verhältnis von 20:1 oder einem Verhältnis von 2:1 bildet.

## Revendications

1. Fragment d'anticorps lié à un épitope et un anticorps qui se lie à l'épitope pour une utilisation dans le traitement d'une infection à partir de bactéries ou de virus chez un animal ayant besoin d'un tel traitement, dans lequel :
(i) le fragment d'anticorps lié à l'épitope doit être administré à l'animal ; et
(ii) l'anticorps qui se lie à l'épitope doit être administré à l'animal avant, en même temps ou après ledit fragment d'anticorps lié à l'épitope ;
dans laquelle après l'utilisation un complexe se forme entre le fragment d'anticorps lié à l'épitope et l'anticorps qui se lie à l'épitope, améliorant ainsi l'efficacité du fragment d'anticorps comparé à un fragment d'anticorps non complexé équivalent, et dans laquelle le fragment d'anticorps se lie à un antigène cible sélectionné parmi des antigènes bactériens ou des antigènes viraux.

2. Fragment d'anticorps lié à un épitope et un anticorps qui se lie à l'épitope pour une utilisation selon la revendication 1 dans laquelle l'efficacité améliorée du fragment d'anticorps comprend (i) un effet thérapeutique augmenté du fragment d'anticorps ; (ii) une persistance et/ou une stabilité augmentée du fragment d'anticorps ; ou (iii) une réponse immunitaire augmentée.

3. Fragment d'anticorps lié à un épitope et un anticorps qui se lie à l'épitope pour une utilisation selon la revendication 2 dans laquelle l'effet thérapeutique augmenté comprend l'efficacité protectrice améliorée du fragment d'anticorps contre l'infection bactérienne.

4. Fragment d'anticorps lié à un épitope et un anticorps qui se lie à l'épitope pour une utilisation selon la revendication 1 dans laquelle l'efficacité augmentée du fragment d'anticorps comprend (i) l'activation de fonctions du système immunitaire en aval ; (ii) le recrutement augmenté des fonctions effectrices médiées par Fc-R ; ou (iii) le recrutement du système et/ou l'augmentation de la phagocytose.

5. Fragment d'anticorps lié à un épitope et un anticorps qui se lie à l'épitope pour une utilisation selon l'une quelconque des revendications 1 à 4 dans laquelle le fragment d'anticorps lié à l'épitope est une protéine de fusion.

6. Fragment d'anticorps lié à un épitope et un anticorps qui se lie à l'épitope pour une utilisation selon l'une quelconque des revendications 1 à 4 dans laquelle le fragment d'anticorps est lié à l'épitope via une réticulation chimique.

7. Fragment d'anticorps lié à un épitope et un anticorps qui se lie à l'épitope pour une utilisation selon l'une quelconque des revendications 1 à 6 dans laquelle le fragment d'anticorps est sélectionné parmi : les anticorps scFv, les fragment scFv stabilisés au disulfide, les anticorps à domaine unique VHH et les anticorps Fab.

8. Fragment d'anticorps lié à un épitope et un anticorps qui se lie à l'épitope pour une utilisation selon l'une quelconque des revendications 1 à 7 dans laquelle l'épitope est sélectionné parmi : les antigènes cellulaires, les antigènes humoraux, les pathogènes, les toxines, les virus, les bactéries, les antigènes tumoraux ou autoantigènes, la glutathion-S-transférase (GST) ou une partie de celle-ci, c-Myc ou une partie de celle-ci, la poly-histidine (6X-His), la penta-histidine (Penta-His), FLAG®, la protéine fluorescente verte (GFP) ou une partie de celle-ci, la protéine de liaison au maltose (MBP) ou une partie de celle-ci, l'étiquette hémagglutinine du virus de la grippe A (HA) ; YPYDVPDYA (SEQ ID NO : 1)) ou une partie de celle-ci, la β-galactosidase (β-gal) ou une partie de celle-ci, GAL4 ou une partie de celle-ci, la MRP humaine ou une partie de celle-ci, l'épitope V5 du virus simien, les épitopes de l'antigène T du virus du polyome, QCRL-1 et l'épitope viral KT3 ou des parties de ceux-ci.

9. Fragment d'anticorps lié à un épitope et un anticorps qui se lie à l'épitope pour une utilisation selon l'une quelconque des revendications 1 à 8 dans laquelle l'anticorps est sélectionné parmi un anticorps polyclonal, un anticorps monoclonal, une IgG, une IgM, une IgA, une IgE et une IgD.

10. Fragment d'anticorps lié à un épitope et un anticorps qui se lie à l'épitope pour une utilisation selon l'une quelconque des revendications 1 à 9 dans laquelle le fragment d'anticorps forme un complexe avec l'anticorps dans un rapport 20 : 1 ou un rapport 2 : 1.


### SEQUENCE LISTING

<110> University of Guelph
   Hall, J.C.
   McLean, Michael D.
   Xie, Xuemei
   Weisser, Nina
   Almquist, Kurt C.
<120> Methods Of Improving The Therapeutic Efficacy And Utility Of Antibody Fragments
<130> 20436-5
<150> US 61/111,915
   <151> 2008-11-06
<160> 13
<170> PatentIn version 3.3
<210> 1
   <211> 9
   <212> PRT
   <213> Artificial Sequence
<220>
   <223> Human influenza virus hemagglutinin protein
<400> 1
   Tyr Pro Tyr Asp Val Pro Asp Tyr Ala
   1               5
<210> 2
   <211> 51
   <212> DNA
   <213> Artificial Sequence
<220>
   <223> Primer
<400> 2
   gtatctctcg agaaaagaga ggctgaagct gacgtggtta tgacacaaac t 51
<210> 3
   <211> 50
   <212> DNA
   <213> Artificial Sequence
<220>
   <223> Primer
<400> 3
   tatctctcga gaaaagagag gctgaagctc aagttcaact tgttgaaagt 50
<210> 4
   <211> 42
   <212> DNA
   <213> Artificial Sequence
<220>
   <223> Primer
<400> 4
   tcctgttcta gattatcaac actctcctct attgaaactc tt 42
<210> 5
   <211> 32
   <212> DNA
   <213> Artificial Sequence
<220>
   <223> Primer
<400> 5
   tcctgttcta gatgtgtttt gtcgcatgac tt 32
<210> 6
   <211> 20
   <212> DNA
   <213> Artificial Sequence
<220>
   <223> Primer
<400> 6
   catgagatcg gatccaacat 20
<210> 7
   <211> 20
   <212> DNA
   <213> Artificial Sequence
<220>
   <223> Primer
<400> 7
   aaaaagaaac gaggcggtct 20
<210> 8
   <211> 51
   <212> DNA
   <213> Artificial Sequence
<220>
   <223> Primer
<400> 8
   gtatctctcg agaaaagaga ggctgaagct gacgtggtta tgacacaaac t 51
<210> 9
   <211> 32
   <212> DNA
   <213> Artificial Sequence
<220>
   <223> Primer
<400> 9
   tcctgttcta gagaaacagt aaccaatgtt cc 32
<210> 10
   <211> 10
   <212> PRT
   <213> Artificial Sequence
<220>
   <223> Epitope Of Human c-MYC Oncogene
<400> 10
   Glu Gln Lys Leu Ile Ser Glu Glu Asp Leu
   1               5                   10
<210> 11
   <211> 8
   <212> PRT
   <213> Artificial Sequence
<220>
   <223> Novel amino acid sequence, aka FLAG tag
<400> 11
   Asp Tyr Lys Asp Asp Asp Asp Lys
   1               5
<210> 12
   <211> 14
   <212> PRT
   <213> Artificial Sequence
<220>
   <223> The V5 epitope tag is derived from a small epitope (Pk) present on the P and V proteins of the paramyxovirus of simian virus 5 (SV5).
<400> 12
   Gly Lys Pro Ile Pro Asn Pro Leu Leu Gly Leu Asp Ser Thr
   1               5                   10
<210> 13
   <211> 7
   <212> PRT
   <213> Artificial Sequence
<220>
   <223> QCRL-1 epitope from human MRP1 transporter protein
<400> 13
   Ser Ser Tyr Ser Gly Asp Ile
   1               5